(19) Europäisches Patentamt
European Patent Office
Office européen des brevets

(11) **EP 1 651 660 B1**

(12) **EUROPEAN PATENT SPECIFICATION**

(45) Date of publication and mention
of the grant of the patent:
**24.01.2018 Bulletin 2018/04**

(51) Int Cl.:
***C12N 9/22*** *(2006.01)*     ***C12N 15/62*** *(2006.01)*
***C12N 15/01*** *(2006.01)*

(21) Application number: **04780272.3**

(22) Date of filing: **06.08.2004**

(86) International application number:
**PCT/US2004/025407**

(87) International publication number:
**WO 2005/014791 (17.02.2005 Gazette 2005/07)**

(54) **METHODS AND COMPOSITIONS FOR TARGETED CLEAVAGE AND RECOMBINATION**

VERFAHREN UND ZUSAMMENSETZUNGEN ZUR GEZIELTEN SPALTUNG UND REKOMBINATION

METHODES ET COMPOSITIONS PERMETTANT UN CLIVAGE ET UNE RECOMBINAISON CIBLES

(84) Designated Contracting States:
**AT BE BG CH CY CZ DE DK EE ES FI FR GB GR HU IE IT LI LU MC NL PL PT RO SE SI SK TR**

(30) Priority: **08.08.2003 US 493931 P**
**07.11.2003 US 518253 P**
**18.12.2003 US 530541 P**
**05.02.2004 US 542780 P**
**26.03.2004 US 556831 P**
**01.06.2004 US 575919 P**

(43) Date of publication of application:
**03.05.2006 Bulletin 2006/18**

(60) Divisional application:
**15159259.9 / 2 927 318**
**17165881.8 / 3 222 715**

(73) Proprietor: **Sangamo Therapeutics, Inc.**
**Richmond, CA 94804 (US)**

(72) Inventors:
• **PABO, Carl, O.**
**Richmond, CA 94804 (US)**
• **URNOV, Fyodor**
**Richmond, CA 94804 (US)**
• **HOLMES, Michael, C.**
**Richmond, CA 94804 (US)**
• **MILLER, Jeffrey, C.**
**Richmond, CA 94804 (US)**
• **GUSCHIN, Dmitry**
**Richmond, CA 94804 (US)**

(74) Representative: **Carpmaels & Ransford LLP**
**One Southampton Row**
**London WC1B 5HA (GB)**

(56) References cited:
WO-A-03/087341       WO-A1-96/40882
WO-A2-03/080809      WO-A2-2004/037977
US-A1- 2002 022 021   US-A1- 2002 107 214
US-B1- 6 265 196

• **PORTEUS M H ET AL: "Chimeric nucleases stimulate gene targeting in human cells", SCIENCE, AMERICAN ASSOCIATION FOR THE ADVANCEMENT OF SCIENCE, WASHINGTON, DC; US, vol. 300, 2 May 2003 (2003-05-02), page 763, XP002974231, ISSN: 0036-8075, DOI: 10.1126/SCIENCE.1078395**
• **BIBIKOVA M ET AL: "Targeted chromosomal cleavage and mutagenesis in drosophila using zinc-finger nucleases", GENETICS, GENETICS SOCIETY OF AMERICA, AUSTIN, TX, US, vol. 161, no. 3, July 2002 (2002-07), pages 1169-1175, XP002261110, ISSN: 0016-6731**
• **BIBIKOVA M ET AL: "Enhancing gene targeting with designed zinc finger nucleases", SCIENCE, AMERICAN ASSOCIATION FOR THE ADVANCEMENT OF SCIENCE, WASHINGTON, DC; US, vol. 300, 2 May 2003 (2003-05-02), page 764, XP002974267, ISSN: 0036-8075, DOI: 10.1126/SCIENCE.1079512**

- REBAR ET AL: "Induction of angiogenesis in a mouse model using engineered transcription factors", NATURE MEDICINE, NATURE PUBLISHING GROUP, NEW YORK, NY, US, vol. 8, no. 12, 4 November 2002 (2002-11-04), pages 1427-1432, XP002993087, ISSN: 1078-8956, DOI: 10.1038/NM795
- HUANG B ET AL: "Splase: a new class IIS zinc-finger restriction endonuclease with specificity for Sp1 binding sites", JOURNAL OF PROTEIN CHEMISTRY, US, vol. 15, no. 5, 1 July 1996 (1996-07-01), pages 481-489, XP009158302, ISSN: 0277-8033
- AGGARWAL ET AL: 'Novel site-specific DNA endonucleases' CURR. OPIN. STRUCT. BIOL. vol. 8, May 1998, pages 19 - 25, XP002236854
- XU ET AL: 'Engineering a nicking endonuclease N.AlwI by domain swapping' PROC. NATL. ACAD. SCI. vol. 98, no. 23, 06 November 2001, pages 12990 - 12995, XP002993294
- DAVID A WAH ET AL: "Structure of FokI has implications for DNA cleavage", PROCEEDINGS OF THE NATIONAL ACADEMY OF SCIENCES, NATIONAL ACADEMY OF SCIENCES, US, vol. 95, no. 18, 1 September 1998 (1998-09-01), pages 10564-10569, XP008160883, ISSN: 0027-8424
- BIBIKOVA M ET AL: "Stimulation of homologous recombination through targeted cleavage by chimeric nucleases", MOLECULAR AND CELLULAR BIOLOGY, AMERICAN SOCIETY FOR MICROBIOLOGY, WASHINGTON, US, vol. 21, no. 1, 1 January 2001 (2001-01-01), pages 289-297, XP002974229, ISSN: 0270-7306, DOI: 10.1128/MCB.21.1.289-297.2001
- BITINAITE JURATE ET AL: "FokI dimerization is required for DNA cleavage", PROCEEDINGS OF THE NATIONAL ACADEMY OF SCIENCES, NATIONAL ACADEMY OF SCIENCES, US, vol. 95, no. 18, 1 September 1998 (1998-09-01), pages 10570-10575, XP002212149, ISSN: 0027-8424, DOI: 10.1073/PNAS.95.18.10570
- SMITH J ET AL: "Requirements for double-strand cleavage by chimeric restriction enzymes with zinc finger DNA-recognition domains", NUCLEIC ACIDS RESEARCH, OXFORD UNIVERSITY PRESS, GB, vol. 28, no. 17, 1 January 2000 (2000-01-01), pages 3361-3369, XP002974228, ISSN: 0305-1048, DOI: 10.1093/NAR/28.17.3361

**Description**

TECHNICAL FIELD

The present disclosure is in the field of genome engineering and homologous recombination.

BACKGROUND

A major area of interest in genome biology, especially in light of the determination of the complete nucleotide sequences of a number of genomes, is the targeted alteration of genome sequences. To provide but one example, sickle cell anemia is caused by mutation of a single nucleotide pair in the human β-globin gene. Thus, the ability to convert the endogenous genomic copy of this mutant nucleotide pair to the wild-type sequence in a stable fashion and produce normal β-globin would provide a cure for sickle cell anemia.

Attempts have been made to alter genomic sequences in cultured cells by taking advantage of the natural phenomenon of homologous recombination. *See,* for example, Capecchi (1989) Science 244:1288-1292; U.S. Patent Nos. 6,528,313 and 6,528,314. If a polynucleotide has sufficient homology to the genomic region containing the sequence to be altered, it is possible for part or all of the sequence of the polynucleotide to replace the genomic sequence by homologous recombination. However, the frequency of homologous recombination under these circumstances is extremely low. Moreover, the frequency of insertion of the exogenous polynucleotide at genomic locations that lack sequence homology exceeds the frequency of homologous recombination by several orders of magnitude.

The introduction of a double-stranded break into genomic DNA, in the region of the genome bearing homology to an exogenous polynucleotide, has been shown to stimulate homologous recombination at this site by several thousand-fold in cultured cells. Rouet et al. (1994) Mol. Cell. Biol. 14:8096-8106; Choulika et al. (1995) Mol. Cell. Biol. 15:1968-1973; Donoho et al. (1998) Mol. Cell. Biol. 18:4070-4078. *See* also Johnson et al. (2001) Biochem. Soc. Trans. 29:196-201; and Yanez et al. (1998) Gene Therapy 5:149-159. In these methods, DNA cleavage in the desired genomic region was accomplished by inserting a recognition site for a meganuclease *(i.e.,* an endonuclease whose recognition sequence is so large that it does not occur, or occurs only rarely, in the genome of interest) into the desired genomic region. Bibikova et al. (Science (2003) 300(5620): 764) describes a method for altering a gene in Drosophila using zinc finger nucleases, further details for which are provided in Bibikova et al. (Genetics (2002) 161(3): 1169-1175).However, meganuclease cleavage-stimulated homologous recombination relies on either the fortuitous presence of, or the directed insertion of, a suitable meganuclease recognition site in the vicinity of the genomic region to be altered. Since meganuclease recognition sites are rare (or nonexistent) in a typical mammalian genome, and insertion of a suitable meganuclease recognition site is plagued with the same difficulties as associated with other genomic alterations, these methods are not broadly applicable.

Thus, there remains a need for compositions and methods for targeted alteration of sequences in any genome.

SUMMARY

The invention provides a method for altering a first nucleotide sequence in a region of interest in cellular chromatin, the method comprising: (a) engineering a first zinc finger binding domain to bind to a second nucleotide sequence in the region of interest, wherein the second sequence comprises at least 9 nucleotides; (b) providing a second zinc finger binding domain to bind to a third nucleotide sequence, wherein the third sequence comprises at least 9 nucleotides; (c) expressing a first fusion protein in the cell, the first fusion protein comprising the first zinc finger binding domain and a first FokI cleavage half-domain; (d) expressing a second fusion protein in the cell, the second fusion protein comprising the second zinc finger binding domain and a second FokI cleavage half-domain, wherein at least one of the fusion proteins comprises an alteration in the amino acid residue at one or more of positions 446, 447, 479, 483, 484, 486, 487, 490, 491, 496, 498, 499, 500, 531, 534, 537 and 538 of the dimerization interface of the cleavage half-domain that inhibits homodimerization of the cleavage half-domain; and (e) contacting the cell with a polynucleotide comprising a fourth nucleotide sequence, wherein the fourth nucleotide sequence is homologous but non-identical with the first nucleotide sequence; wherein binding of the first fusion protein to the second sequence and binding of the second fusion protein to the third sequence positions the cleavage half-domains such that the cellular chromatin is cleaved in the region of interest, thereby facilitating homologous recombination between the first nucleotide sequence and the fourth nucleotide sequence, resulting in alteration of the first nucleotide sequence.

The invention also provides a cell comprising: (a) a first nucleotide sequence in a region of interest in the cellular chromatin of the cell; (b) a first fusion protein or a polynucleotide encoding the first fusion protein, wherein the first fusion protein comprises a first zinc finger binding domain and a first *FokI* cleavage half-domain, wherein the first zinc finger domain is engineered to bind to a second nucleotide sequence in the region of interest, wherein the second sequence comprises at least 9 nucleotides; (c) a second fusion protein or a polynucleotide encoding the second fusion protein, wherein the second fusion protein comprises a second zinc finger binding domain and a second *FokI* cleavage half-domain, wherein the second zinc finger domain is capable of binding to a third nucleotide sequence, wherein the third sequence comprises at least 9 nucleotides, wherein at least one of the fusion proteins comprises an alteration in the amino acid residue at one or more of positions 446, 447, 479, 483, 484, 486, 487, 490, 491, 496, 498, 499, 500, 531, 534, 537 and 538 of the dimerization interface of the cleavage half-domain that inhibits homodimerization of the cleavage half-domain; and (d) a polynucleotide comprising a fourth nucleotide sequence, wherein the fourth nucleotide sequence is homologous but non-identical with the first nucleotide sequence; wherein binding of the first fusion protein to the second sequence and binding of the second fusion protein to the third sequence positions the cleavage half-domains such that the cellular chromatin is cleaved in the region of interest, thereby facilitating homologous recombination between the first nucleotide sequence and the fourth nucleotide sequence, resulting in alteration of the first nucleotide sequence.

The invention further provides a pharmaceutical composition for altering a first nucleotide sequence in a region of interest in cellular chromatin comprising: (a) a first fusion protein or a polynucleotide encoding the first fusion protein, wherein the first fusion protein comprises a first zinc finger binding domain and a first *FokI* cleavage half-domain, wherein the first zinc finger domain is engineered to bind to a second nucleotide sequence in the region of interest, wherein the second sequence comprises at least 9 nucleotides; (b) a second fusion protein or a polynucleotide encoding the second fusion protein, wherein the second fusion protein comprises a second zinc finger binding domain and a second *FokI* cleavage half-domain; wherein the second zinc finger domain is capable of binding to a third nucleotide sequence, wherein the third sequence comprises at least 9 nucleotides, wherein at least one of the fusion proteins comprises an alteration in the amino acid residue at one or more of positions 446, 447, 479, 483, 484, 486, 487, 490, 491, 496, 498, 499, 500, 531, 534, 537 and 538 of the dimerization interface of the cleavage half-domain that inhibits homodimerization of the cleavage half-domain; and (c) a polynucleotide comprising a fourth nucleotide sequence, wherein the fourth nucleotide sequence is homologous but non-identical with the first nucleotide sequence, wherein, in use, binding of the first fusion protein to the second sequence and binding of the second fusion protein to the third sequence positions the cleavage half-domains such that the cellular chromatin is cleaved in the region of interest, thereby facilitating homologous recombination between the first nucleotide sequence and the fourth nucleotide sequence, resulting in alteration of the first nucleotide sequence.

The invention provides an in vitro method for inactivating a target gene by targeted deletion of a genomic sequence of the target gene, the method comprising cleaving the target gene at two cleavage sites such that the two sites are joined so as to delete the sequence between the two cleavage sites, thereby inactivating the target gene, wherein the method comprises expressing two fusion proteins in a cell, each fusion protein comprising a zinc finger binding domain and a *FokI* cleavage half-domain, wherein at least one of the fusion proteins comprises an alteration in the amino acid residue at one or more of positions 446, 447, 479, 483, 484, 486, 487, 490, 491, 496, 498, 499, 500, 531, 534, 537 and 538 of the dimerization interface of the cleavage half-domain that inhibits homodimerization of the cleavage half-domain, wherein binding of the fusion proteins to the target gene positions the cleavage half-domains such that a functional cleavage domain is reconstituted leading to cleavage of the target gene.

The present disclosure provides compositions and methods for targeted cleavage of cellular chromatin in a region of interest and/or homologous recombination at a predetermined region of interest in cells. Cells include cultured cells, cells in an organism and cells that have been removed from an organism for treatment in cases where the cells and/or their descendants will be returned to the organism after treatment. A region of interest in cellular chromatin can be, for example, a genomic sequence or portion thereof. Compositions include fusion polypeptides comprising an engineered zinc finger binding domain (e.g., a zinc finger

binding domain having a novel specificity) and a cleavage domain, and fusion polypeptides comprising an engineered zinc finger binding domain and a cleavage half-domain. Cleavage domains and cleavage half domains can be obtained, for example, from various restriction endonucleases and/or homing endonucleases.

Cellular chromatin can be present in any type of cell including, but not limited to, prokaryotic and eukaryotic cells, fungal cells, plant cells, animal cells, mammalian cells, primate cells and human cells.

A method for cleavage of cellular chromatin in a region of interest (*e.g.,* a method for targeted cleavage of genomic sequences) is described, the method comprising: (a) selecting a first sequence in the region of interest; (b) engineering a first zinc finger binding domain to bind to the first sequence; and (c) expressing a first fusion protein in the cell, the first fusion protein comprising the first engineered zinc finger binding domain and a cleavage domain; wherein the first fusion protein binds to the first sequence and the cellular chromatin is cleaved in the region of interest. The site of cleavage can be coincident with the sequence to which the fusion protein binds, or it can be adjacent (e.g., separated from the near edge of the binding site by 1, 2, 3, 4, 5, 6, 7, 8, 9, 10, 11, 12, 13, 14, 15 or more nucleotides). A fusion protein can be expressed in a cell, *e.g.,* by delivering the fusion protein to the cell or by delivering a polynucleotide encoding the fusion protein to a cell, wherein the polynucleotide, if DNA, is transcribed, and an RNA molecule delivered to the cell or a transcript of a DNA molecule delivered to the cell is translated, to generate the fusion protein. Methods for polynucleotide and polypeptide delivery to cells are presented elsewhere in this disclosure.

The cleavage domain may comprise two cleavage half-domains that are covalently linked in the same polypeptide. The two cleavage half-domains can be derived from the same endonuclease or from different endonucleases.

Targeted cleavage of cellular chromatin in a region of interest is achieved by expressing two fusion proteins in a cell, each fusion protein comprising a zinc finger binding domain and a cleavage half-domain. One or both of the zinc finger binding domains of the fusion proteins can be engineered to bind to a target sequence in the vicinity of the intended cleavage site. If expression of the fusion proteins is by polynucleotide delivery, each of the two fusion proteins can be encoded by a separate polynucleotide, or a single polynucleotide can encode both fusion proteins.

Accordingly, a method for cleaving cellular chromatin in a region of interest can comprise (a) selecting a first sequence in the region of interest; (b) engineering a first zinc finger binding domain to bind to the first sequence; (c) expressing a first fusion protein in the cell, the first fusion protein comprising the first zinc finger binding domain and a first cleavage half-domain; and (d) expressing a second fusion protein in the cell, the second fusion protein comprising a second zinc finger binding domain and a second cleavage half-domain, wherein the first fusion protein binds to the first sequence, and the second fusion protein binds to a second sequence located between 2 and 50 nucleotides from the first sequence, thereby positioning the cleavage half-domains such that the cellular chromatin is cleaved in the region of interest.

Binding of the first and second fusion proteins positions the cleavage half-domains such that a functional cleavage domain is reconstituted.

In certain embodiments, the second zinc finger binding domain is engineered to bind to the second sequence. The first and second cleavage half-domains are derived from the Type IIS restriction endonuclease *Fok* I.

Any of the methods described herein may comprise (a) selecting first and second sequences in a region of interest, wherein the first and second sequences are between 2 and 50 nucleotides apart; (b) engineering a first zinc finger binding domain to bind to the first sequence; (c) engineering a second zinc finger binding domain to bind to the second sequence; (d) expressing a first fusion protein in the cell, the first fusion protein comprising the first engineered zinc finger binding domain and a first cleavage half-domain; (e) expressing a second fusion protein in the cell, the second fusion protein comprising the second engineered zinc finger binding domain and a second cleavage half-domain; wherein the first fusion protein binds to the first sequence and the second fusion protein binds to the second sequence, thereby positioning the first and second cleavage half-domains such that the cellular chromatin is cleaved in the region of interest.

The first and second cleavage half-domains are derived from the Type IIS restriction endonuclease, *Fok* I. Cellular chromatin can be cleaved at one or more sites between the first and second sequences to which the fusion proteins bind.

In further embodiments of the disclosure, a method for cleavage of cellular chromatin in a region of interest comprises (a) selecting the region of interest; (b) engineering a first zinc finger binding domain to bind to a first sequence in the region of interest; (c) providing a second zinc finger binding domain which binds to a second sequence in the region of interest, wherein the second sequence is located between 2 and 50 nucleotides from the first sequence; (d) expressing a first fusion protein in the cell, the first fusion protein comprising the first zinc finger binding domain and a first cleavage half-domain; and (e) expressing a second fusion protein in the cell, the second fusion protein comprising the second zinc finger binding domain and a second cleavage half domain; wherein the first fusion protein binds to the first sequence, and the second fusion protein binds to the second sequence, thereby positioning the cleavage half-domains such that the cellular chromatin is cleaved in the region of interest.

In any of the methods described herein, the first and second cleavage half-domains may be derived from the same endonuclease or from different endonucleases. In additional embodiments, the second zinc finger binding domain is engineered to bind to the second sequence.

If one or more polynucleotides encoding the fusion proteins are introduced into the cell, an exemplary method for targeted cleavage of cellular chromatin in a region of interest comprises (a) selecting the region of interest; (b) engineering a first zinc finger binding domain to bind to a first sequence in the region of interest; (c) providing a second zinc finger binding domain which binds to a second sequence in the region of interest, wherein the second sequence is located between 2 and 50 nucleotides from the first sequence; and (d) contacting a cell with (i) a first polynucleotide encoding a first fusion protein, the fusion protein comprising the first zinc finger binding domain and a first cleavage half-domain, and (ii) a second polynucleotide encoding a second fusion protein, the fusion protein comprising the second zinc finger binding domain and a second cleavage half domain; wherein the first and second fusion proteins are expressed, the first fusion protein binds to the first sequence and the second fusion protein binds to the second sequence, thereby positioning the cleavage half-domains such that the cellular chromatin is cleaved in the region of interest. In a variation of this method, a cell is contacted with a single polynucleotide which encodes both fusion proteins.

For any of the aforementioned methods, the cellular chromatin can be in a chromosome, episome or organellar genome. In addition, in any of the methods described herein, at least one zinc finger binding domain is engineered, for example by design or selection methods.

Similarly, for any of the aforementioned methods, the cleavage half domain can be derived from, for example, a homing endonuclease or a restriction endonuclease, for example, a Type IIS restriction endonuclease. An exemplary Type IIS restriction endonuclease is *Fok* I.

For any of the methods of targeted cleavage, targeted mutagenesis and/or targeted recombination disclosed herein utilizing fusion proteins comprising a cleavage half-domain, the near edges of the binding sites of the fusion proteins can be separated by 5 or 6 base pairs. In these embodiments, the binding domain and the cleavage domain of the fusion proteins can be separated by a linker of 4 amino acid residues.

It is possible to obtain increased cleavage specificity by utilizing fusion proteins in which one or both cleavage half-domains contains an alteration in the amino acid sequence of the dimerization interface.

Targeted mutagenesis of a region of interest in cellular chromatin can occur when a targeted cleavage event, as describe above, is followed by non-homologous end joining (NHEJ). Accordingly, methods for alteration of a first nucleotide sequence in a region of interest in cellular chromatin are described, wherein the methods comprise the steps of (a) engineering a first zinc finger binding domain to bind to a second nucleotide sequence in the region of interest, wherein the second sequence comprises at least 9 nucleotides; (b) providing a second zinc finger binding domain to bind to a third nucleotide sequence, wherein the third sequence comprises at least 9 nucleotides and is located between 2 and 50 nucleotides from the second sequence; (c) expressing a first fusion protein in the cell, the first fusion protein comprising the first zinc finger binding domain and a first cleavage half-domain; and (d) expressing a second fusion protein in the cell, the second fusion protein comprising the second zinc finger binding domain and a second cleavage half domain; wherein the first fusion protein binds to the second sequence, and the second fusion protein binds to the third sequence, thereby positioning the cleavage half-domains such that the cellular chromatin is cleaved in the region of interest and the cleavage site is subjected to non-homologous end joining.

Targeted mutations resulting from the aforementioned method include, but are not limited to, point mutations (*i.e.,* conversion of a single base pair to a different base pair), substitutions (*i.e.*, conversion of a plurality of base pairs to a different sequence of identical length), insertions or one or more base pairs, deletions of one or more base pairs and any combination of the aforementioned sequence alterations.

Methods for targeted recombination (for, *e.g.*, alteration or replacement of a sequence in a chromosome or a region of interest in cellular chromatin) are also disclosed. For example, a mutant genomic sequence can be replaced by a wild-type sequence, *e.g.*, for treatment of genetic disease or inherited disorders. In addition, a wild-type genomic sequence can be replaced by a mutant sequence, *e.g.,* to prevent function of an oncogene product or a product of a gene involved in an inappropriate inflammatory response. Furthermore, one allele of a gene can be replaced by a different allele.

In such methods, one or more targeted nucleases create a double-stranded break in cellular chromatin at a predetermined site, and a donor polynucleotide, having homology to the nucleotide sequence of the cellular chromatin in the region of the break, is introduced into the cell. Cellular DNA repair processes are activated by the presence of the double-stranded break and the donor polynucleotide is used as a template for repair of the break, resulting in the introduction of all or part of the nucleotide sequence of the donor into the cellular chromatin. Thus a first sequence in cellular chromatin can be altered and, in certain embodiments, can be converted into a sequence present in a donor polynucleotide.

In this context, the use of the terms "replace" or "replacement" can be understood to represent replacement of one nucleotide sequence by another, (*i.e.,* replacement of a sequence in the informational sense), and does not necessarily require physical or chemical replacement of one polynucleotide by another.

Accordingly, a method for replacement of a region of interest in cellular chromatin (*e.g.*, a genomic sequence) with a first nucleotide sequence is disclosed, the method comprising: (a) engineering a zinc finger binding domain to bind to a second sequence in the region of interest; (b) expressing a fusion protein in a cell, the fusion protein comprising the zinc finger binding domain and a cleavage domain; and (c) contacting the cell with a polynucleotide comprising the first nucleotide sequence; wherein the fusion protein binds to the second sequence such that the cellular chromatin is cleaved in the region of interest and a nucleotide sequence in the region of interest is replaced with the first nucleotide sequence. Generally, cellular chromatin is cleaved in the region of interest at or adjacent to the second sequence. The cleavage domain can comprise two cleavage half-domains, which can be derived from the same or from different nucleases.

In addition, a method for replacement of a region of interest in cellular chromatin (*e.g.,* a genomic sequence) with a first nucleotide sequence is disclosed, the method comprising: (a) engineering a first zinc finger binding domain to bind to a second sequence in the region of interest; (b) providing a second zinc finger binding domain to bind to a third sequence in the region of interest; (c) expressing a first fusion protein in a cell, the first fusion protein comprising the first zinc finger binding domain and a first cleavage half-domain; (d) expressing a second fusion protein in the cell, the second fusion protein comprising the second zinc finger binding domain and a second cleavage half-domain; and (e) contacting the cell with a polynucleotide comprising the first nucleotide sequence; wherein the first fusion protein binds to the second sequence and the second fusion protein binds to the third sequence, thereby positioning the cleavage half-domains such that the cellular chromatin is cleaved in the region of interest and a nucleotide sequence in the region of interest is replaced with the first nucleotide sequence. Generally, cellular chromatin is cleaved in the region of interest at a site between the second and third sequences.

Additional methods for replacement of a region of interest in cellular chromatin (*e.g.,* a genomic sequence) with a first nucleotide sequence comprise: (a) selecting a second sequence, wherein the second sequence is in the region of interest and has a length of at least 9 nucleotides; (b) engineering a first zinc finger binding domain to bind to the second sequence; (c) selecting a third sequence, wherein the third sequence has a length of at least 9 nucleotides and is located between 2 and 50 nucleotides from the second sequence; (d) providing a second zinc finger binding domain to bind to the third sequence; (e) expressing a first fusion protein in a cell, the first fusion protein comprising the first zinc finger binding domain and a first cleavage half-domain; (f) expressing a second fusion protein in the cell, the second fusion protein comprising the second zinc finger binding domain and a second cleavage half-domain; and (g) contacting the cell with a polynucleotide comprising the first nucleotide sequence; wherein the first fusion protein binds to the second sequence and the second fusion protein binds to the third sequence, thereby positioning the cleavage half-domains such that the cellular chromatin is cleaved in the region of interest and a nucleotide sequence in the region of interest is replaced with the first nucleotide sequence. Generally, cellular chromatin is cleaved in the region of interest at a site between the second and third sequences.

In another aspect, methods for targeted recombination are disclosed in which, a first nucleotide sequence, located in a region of interest in cellular chromatin, is replaced with a second nucleotide sequence. The methods comprise (a) engineering a first zinc finger binding domain to bind to a third sequence in the region of interest; (b) providing a second zinc finger binding domain to bind to a fourth sequence; (c) expressing a first fusion protein in a cell, the fusion protein comprising the first zinc finger binding domain and a first cleavage half-domain; (d) expressing a second fusion protein in the cell, the second fusion protein comprising the second zinc finger binding domain and a second cleavage half-domain; and (e) contacting a cell with a polynucleotide comprising the second nucleotide sequence; wherein the first fusion protein binds to the third sequence and the second fusion protein binds to the fourth sequence, thereby positioning the cleavage half-domains such that the cellular chromatin is cleaved in the region of interest and the first nucleotide sequence is replaced with the second nucleotide sequence.

A method for alteration of a first nucleotide sequence in a region of interest in cellular chromatin is disclosed, the method comprising the steps of (a) engineering a first zinc finger binding domain to bind to a second nucleotide sequence in the region of interest, wherein the second sequence comprises at least 9 nucleotides; (b) providing a second zinc finger binding domain to bind to a third nucleotide sequence, wherein the third sequence comprises at least 9 nucleotides and is located between 2 and 50 nucleotides from the second sequence; (c) expressing a first fusion protein in the cell, the first fusion protein comprising the first zinc finger binding domain and a first cleavage half-domain; (d) expressing a second fusion protein in the cell, the second fusion protein comprising the second zinc finger binding domain and a second cleavage half domain; and (e) contacting the cell with a polynucleotide comprising a fourth nucleotide sequence, wherein the fourth nucleotide sequence is homologous but non-identical with the first nucleotide sequence; wherein the first fusion protein binds to the second sequence, and the second fusion protein binds to the third sequence, thereby positioning the cleavage half-domains such that the cellular chromatin is cleaved in the region of interest and the first nucleotide sequence is altered. In certain embodiments, the first nucleotide sequence is converted to the fourth nucleotide sequence. In additional embodiments, the second and third nucleotide sequences (*i.e.*, the binding sites for the fusion proteins) are present in the polynucleotide comprising the fourth nucleotide sequence (*i.e.*, the donor polynucleotide) and the polynucleotide comprising the fourth nucleotide sequence is cleaved.

In the aforementioned methods for targeted recombination, the binding sites for the fusion proteins (*i.e.*, the third and fourth sequences) can comprise any number of nucleotides. Preferably, they are at least nine nucleotides in length, but they can also be larger (*e.g.*, 10, 11, 12, 13, 14, 15, 16, 17, 18 and up to 100 nucleotides, including any integral value between 9 and 100 nucleotides); moreover the third and fourth sequences need not be the same length. The distance between the binding sites (*i.e.*, the length of nucleotide sequence between the third and fourth sequences) can be any integral number of nucleotide pairs between 2 and 50, (*e.g.,* 5 or 6 base pairs) as measured from the near end of one binding site to the near end of the other binding site.

In the aforementioned methods for targeted recombination, cellular chromatin can be cleaved at a site located between the binding sites of the two fusion proteins. In certain embodiments, the binding sites are on opposite DNA strands. Moreover, expression of the fusion proteins in the cell can be accomplished either by introduction of the proteins into the cell or by introduction of one or more polynucleotides into the cell, which are optionally transcribed (if the polynucleotide is DNA), and the transcript(s) translated, to produce the fusion proteins. For example, two polynucleotides, each comprising sequences encoding one of the two fusion proteins, can be introduced into a cell. Alternatively, a single polynucleotide comprising sequences encoding both fusion proteins can be introduced into the cell.

Thus, a method for replacement of a region of interest in cellular chromatin (e.g., a genomic sequence) with a first nucleotide sequence comprises: (a) engineering a first zinc finger binding domain to bind to a second sequence in the region of interest; (b) providing a second zinc finger binding domain to bind to a third sequence; and (c) contacting a cell with:

(i) a first polynucleotide comprising the first nucleotide sequence;

(ii) a second polynucleotide encoding a first fusion protein, the first fusion protein comprising the first zinc finger binding domain and a first cleavage half-domain; and

(iii) a third polynucleotide encoding a second fusion protein, the second fusion protein comprising the second zinc finger binding domain and a second cleavage half-domain;

wherein the first and second fusion proteins are expressed, the first fusion protein binds to the second sequence and the second fusion protein binds to the third sequence, thereby positioning the cleavage half-domains such that the cellular chromatin is cleaved in the region of interest; and the region of interest is replaced with the first nucleotide sequence.

In preferred methods for targeted recombination and/or replacement and/or alteration of a sequence in a region of interest in cellular chromatin, a chromosomal sequence is altered by homologous recombination with an exogenous "donor" nucleotide sequence. Such homologous recombination is stimulated by the presence of a double-stranded break in cellular chromatin, if sequences homologous to the region of the break are present. Double-strand breaks in cellular chromatin can also stimulate cellular mechanisms of non-homologous end joining.

In any of the methods described herein, the first nucleotide sequence (the "donor sequence") can contain sequences that are homologous, but not identical, to genomic sequences in the region of interest, thereby stimulating homologous recombination to insert a non-identical sequence in the region of interest. Thus, in certain embodiments, portions of the donor sequence that are homologous to sequences in the region of interest exhibit between about 80 to 99% (or any integer therebetween) sequence identity to the genomic sequence that is replaced. In other embodiments, the homology between the donor and genomic sequence is higher than 99%, for example if only 1 nucleotide differs as between donor and genomic sequences of over 100 contiguous base pairs. In certain cases, a non-homologous portion of the donor sequence can contain sequences not present in the region of interest, such that new sequences are introduced into the region of interest. In these instances, the non-homologous sequence is generally flanked by sequences of 50-1,000 base pairs (or any integral value therebetween) or any number of base pairs greater than 1,000, that are homologous or identical to sequences in the region of interest. In other embodiments, the donor sequence is non-homologous to the first sequence, and is inserted into the genome by non-homologous recombination mechanisms.

Described herein are methods for targeted recombination and/or replacement and/or alteration of a sequence of interest in cellular chromatin, where the first and second cleavage half-domains can be derived from the same endonuclease or from different endonucleases. Endonucleases include, but are not limited to, homing endonucleases and restriction endonucleases. Exemplary restriction endonucleases are Type IIS restriction endonucleases; an exemplary Type IIS restriction endonuclease is *Fok* I.

The region of interest can be in a chromosome, episome or organellar genome. The region of interest can comprise a mutation, which can replaced by a wild type sequence (or by a different mutant sequence), or the region of interest can contain a wild-type sequence that is replaced by a mutant sequence or a different allele. Mutations include, but are not limited to, point mutations (transitions, transversions), insertions of one or more nucleotide pairs, deletions of one or more nucleotide pairs, rearrangements, inversions and translocations. Mutations can change the coding sequence, introduce premature stop codon(s) and/or modify the frequency of a repetitive sequence motif (*e.g.,* trinucleotide repeat) in a gene. For applications in which targeted recombination is used to replace a mutant sequence, cellular chromatin is generally cleaved at a site located within 100 nucleotides on either side of the mutation, although cleavage sites located up to 6-10 kb from the site of a mutation can also be used.

In any of the methods described herein, the second zinc finger binding domain can be engineered, for example designed and/or selected.

Further, the donor polynucleotide can be DNA or RNA, can be linear or circular, and can be single-stranded or double-stranded. It can be delivered to the cell as naked nucleic acid, as a complex with one or more delivery agents (*e.g.*, liposomes, poloxamers) or contained in a viral delivery vehicle, such as, for example, an adenovirus or an adeno-associated Virus (AAV). Donor sequences can range in length from 10 to 1,000 nucleotides (or any integral value of nucleotides therebetween) or longer.

Similarly, polynucleotides encoding fusions between a zinc finger binding domain and a cleavage domain or half-domain can be DNA or RNA, can be linear or circular, and can be single-stranded or double-stranded. They can be delivered to the cell as naked nucleic acid, as a complex with one or more delivery agents (*e.g.*, liposomes, poloxamers) or contained in a viral delivery vehicle, such as, for example, an adenovirus or an adeno-associated virus (AAV). A polynucleotide can encode one or more fusion proteins.

In the methods for targeted recombination disclosed herein, as with the methods for targeted cleavage, a cleavage domain or half-domain can derive from any nuclease, *e.g.,* a homing endonuclease or a restriction endonuclease, in particular, a Type IIS restriction endonuclease. Cleavage half-domains can derived from the same or from different endonucleases. An exemplary source, from which a cleavage half-domain can be derived, is the Type IIS restriction endonuclease *Fok* I.

In certain embodiments, the frequency of homologous recombination can be enhanced by arresting the cells in the G2 phase of the cell cycle and/or by activating the expression of one or more molecules (protein, RNA) involved in homologous recombination and/or by inhibiting the expression or activity of proteins involved in non-homologous end-joining.

BRIEF DESCRIPTION OF THE DRAWINGS

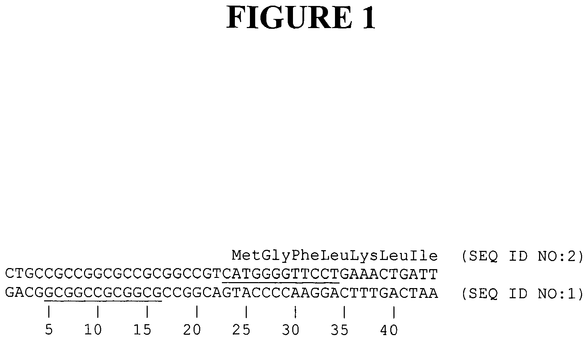

**Figure 1** shows the nucleotide sequence, in double-stranded form, of a portion of the human hSMC1L1 gene encoding the amino-terminal portion of the protein (SEQ ID NO:1) and the encoded amino acid sequence (SEQ ID NO:2). Target sequences for the hSMC1-specific ZFPs are underlined (one on each DNA strand).

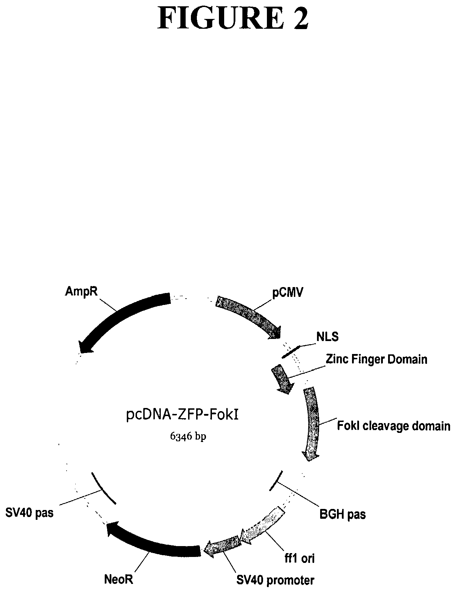

**Figure 2** shows a schematic diagram of a plasmid encoding a ZFP-*Fok*I fusion for targeted cleavage of the hSMC1 gene.

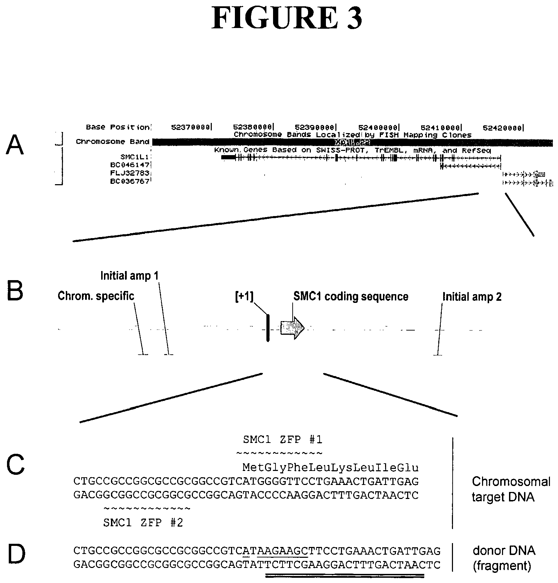

**Figure 3 A-D** show a schematic diagram of the hSMC1 gene. Figure 3A shows a schematic of a portion of the human X chromosome which includes the hSMC1 gene. Figure 3B shows a schematic of a portion of the hSMC1 gene including the upstream region (left of +1), the first exon (between +1 and the right end of the arrow labeled "SMC1 coding sequence") and a portion of the first intron. Locations of sequences homologous to the initial amplification primers and to the chromosome-specific primer (see Table 3) are also provided. Figure 3C shows the nucleotide sequence of the human X chromosome in the region of the SMC1 initiation codon (SEQ ID NO: 3), the encoded amino acid sequence (SEQ ID NO: 4), and the target sites for the SMC1-specific zinc finger proteins. Figure 3D shows the sequence of the corresponding region of the donor molecule (SEQ ID NO: 5), with differences between donor and chromosomal sequences underlined. Sequences contained in the donor-specific amplification primer (Table 3) are indicated by double underlining.

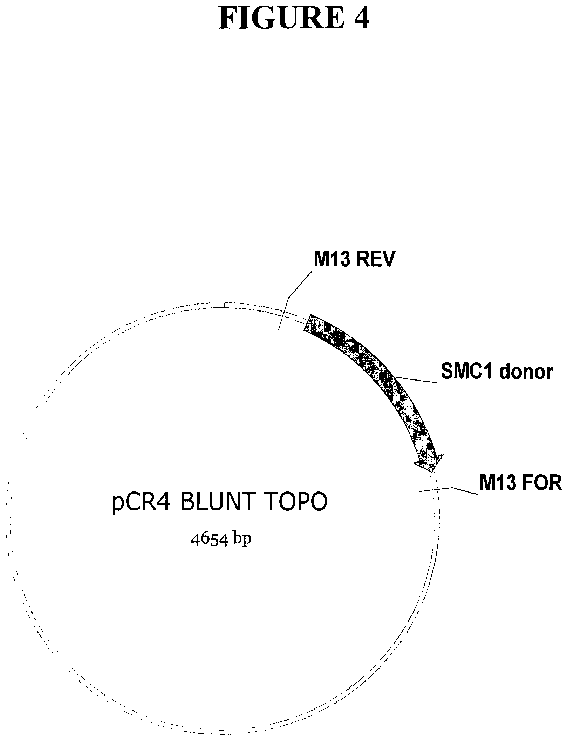

**Figure 4** shows a schematic diagram of the hSMC1 donor construct.

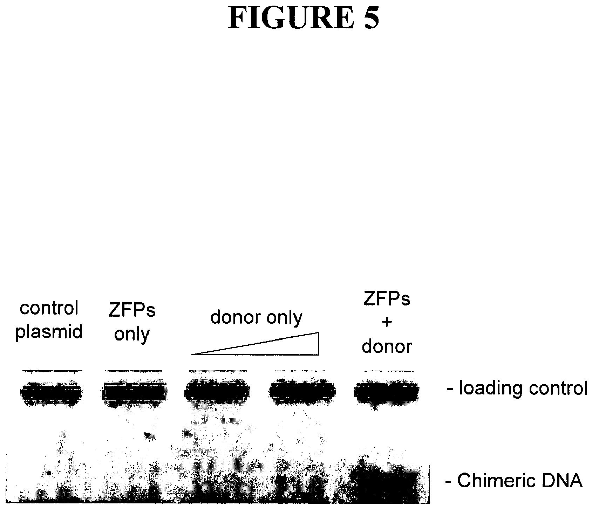

**Figure 5** shows PCR analysis of DNA from transfected HEK293 cells. From left, the lanes show results from cells transfected with a plasmid encoding GFP (control plasmid), cells transfected with two plasmids, each of which encodes one of the two hSMC1-specific ZFP-*Fok*I fusion proteins (ZFPs only), cells transfected with two concentrations of the hSMC1 donor plasmid (donor only), and cells transfected with the two ZFP-encoding plasmids and the donor plasmid (ZFPs + donor). See Example 1 for details.

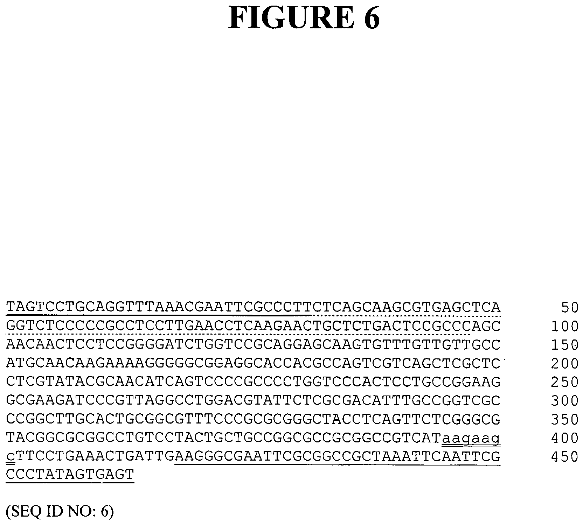

**Figure 6** shows the nucleotide sequence of an amplification product derived from a mutated hSMC1 gene (SEQ ID NO:6) generated by targeted homologous recombination. Sequences derived from the vector into which the amplification product was cloned are single-underlined, chromosomal sequences not present in the donor molecule are indicated by dashed underlining (nucleotides 32-97), sequences common to the donor and the chromosome are not underlined (nucleotides 98-394 and 402-417), and sequences unique to the donor are double-underlined (nucleotides 395-401). Lower-case letters represent sequences that differ between the chromosome and the donor.

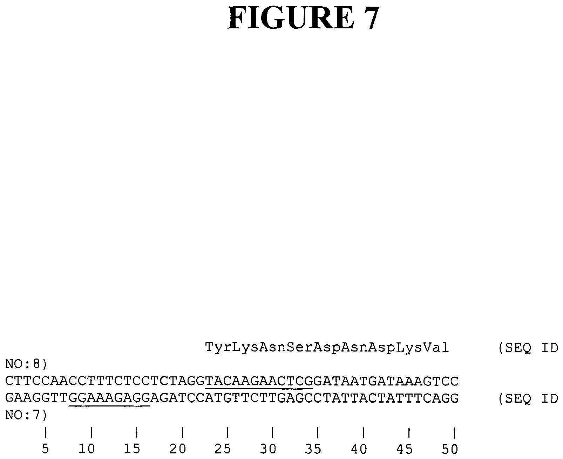

**Figure 7** shows the nucleotide sequence of a portion of the human IL2Rγ gene comprising the 3' end of the second intron and the 5' end of third exon (SEQ ID NO:7) and the amino acid sequence encoded by the displayed portion of the third exon (SEQ ID NO:8). Target sequences for the second pair of IL2Rγ-specific ZFPs are underlined. *See Example 2 for details.*

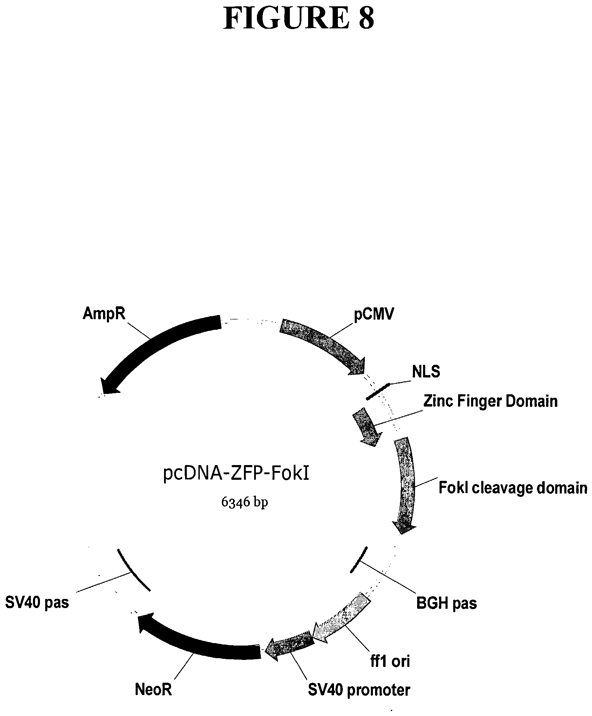

**Figure 8** shows a schematic diagram of a plasmid encoding a ZFP-*Fok*I fusion for targeted cleavage of IL2Rγ gene.

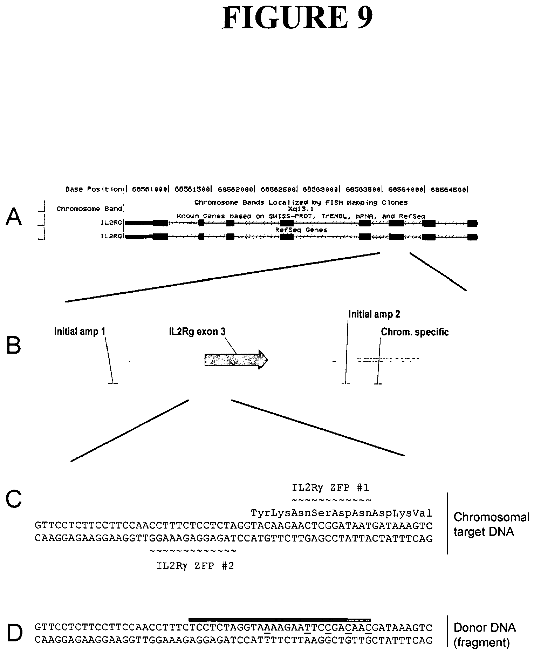

**Figure 9 A-D** show a schematic diagram of the IL2Rγ gene. Figure 9A shows a schematic of a portion of the human X chromosome which includes the IL2Rγ gene. Figure 9B shows a schematic of a portion of the IL2Rγ gene including a portion of the second intron, the third exon and a portion of the third intron. Locations of sequences homologous to the initial amplification primers and to the chromosome-specific primer (see Table 5) are also provided. Figure 9C shows the nucleotide sequence of the human X chromosome in the region of the third exon of the IL2Rγ gene (SEQ ID NO: 9), the encoded amino acid sequence (SEQ ID NO: 10), and the target sites for the first pair of IL2Rγ-specific zinc finger proteins. Figure 9D shows the sequence of the corresponding region of the donor molecule (SEQ ID NO: 11), with differences between donor and chromosomal sequences underlined. Sequences contained in the donor-specific amplification primer (Table 5) are indicated by double overlining.

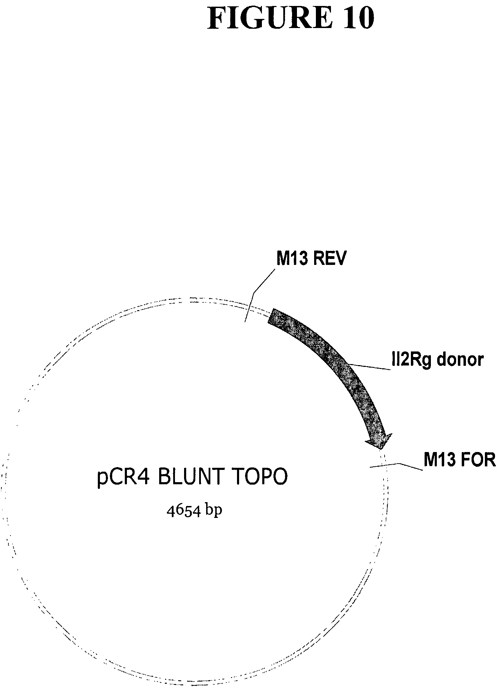

**Figure 10** shows a schematic diagram of the IL2Rγ donor construct.

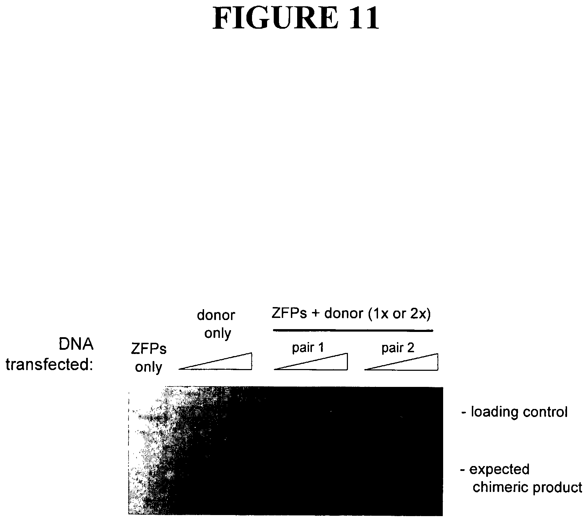

**Figure 11** shows PCR analysis of DNA from transfected K652 cells. From left, the lanes show results from cells transfected with two plasmids, each of which encodes one of a pair of IL2Rγ -specific ZFP-*Fok*I fusion proteins (ZFPs only, lane 1), cells transfected with two concentrations of the IL2Rγ donor plasmid (donor only, lanes 2 and 3), and cells transfected with the two ZFP-encoding plasmids and the donor plasmid (ZFPs + donor, lanes 4-7). Each of the two pairs of IL2Rγ-specific ZFP-*Fok*I fusions were used (identified as "pair 1" and "pair 2") and use of both pairs resulted in production of the diagnostic amplification product (labeled "expected chimeric product" in the Figure). See Example 2 for details.

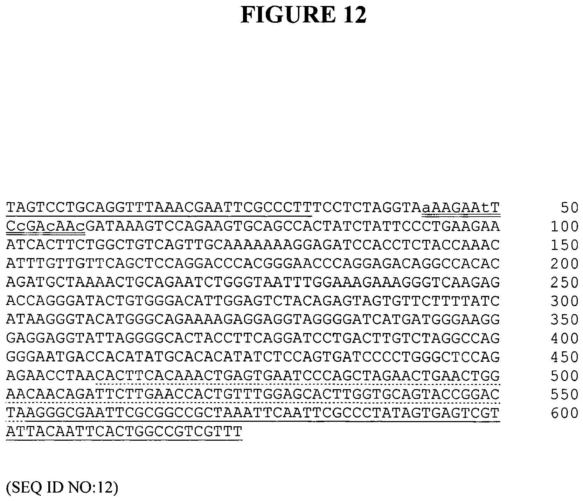

**Figure 12** shows the nucleotide sequence of an amplification product derived from a mutated IL2Rγ gene (SEQ ID NO:12) generated by targeted homologous recombination. Sequences derived from the vector into which the amplification product was cloned are single-underlined, chromosomal sequences not present in the donor molecule are indicated by dashed underlining (nucleotides 460-552), sequences common to the donor and the chromosome are not underlined (nucleotides 32-42 and 59-459), and a stretch of sequence containing nucleotides which distinguish donor sequences from chromosomal sequences is double-underlined (nucleotides 44-58). Lower-case letters represent nucleotides whose sequence differs between the chromosome and the donor.

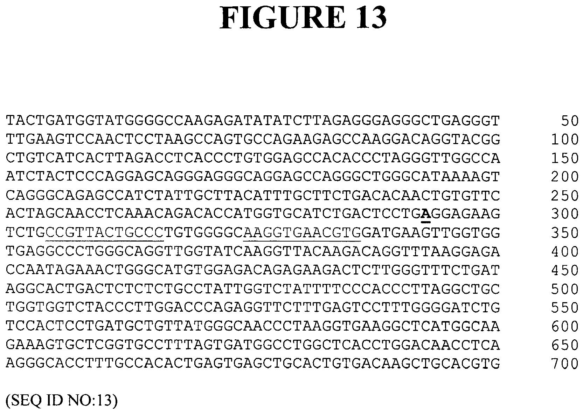

**Figure 13** shows the nucleotide sequence of a portion of the human beta-globin gene encoding segments of the core promoter, the first two exons and the first intron (SEQ ID NO:13). A missense mutation changing an A (in boldface and underlined) at position 5212541 on Chromosome 11 (BLAT, UCSC Genome Bioinformatics site) to a T results in sickle cell anemia. A first zinc finger/*Fok*I fusion protein was designed such that the primary contacts were with the underlined 12-nucleotide sequence AAGGTGAACGTG (nucleotides 305-316 of SEQ ID NO: 13), and a second zinc finger/*Fok*I fusion protein was designed such that the primary contacts were with the complement of the underlined 12-nucleotide sequence CCGTTACTGCCC (nucleotides 325-336 of SEQ ID NO:13).

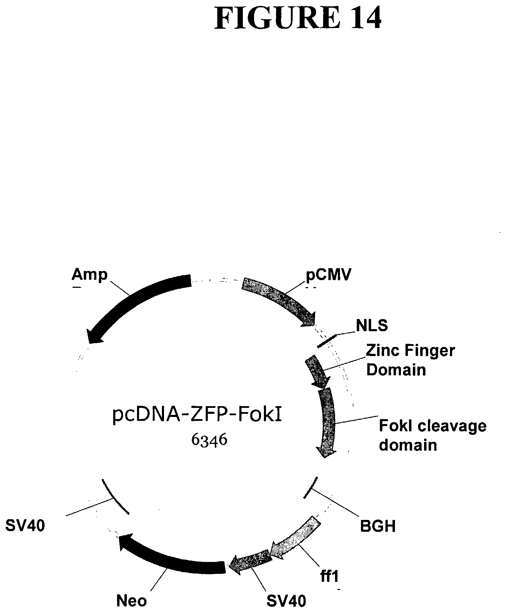

**Figure 14** is a schematic diagram of a plasmid encoding ZFP-*Fok*I fusion for targeted cleavage of the human beta globin gene.

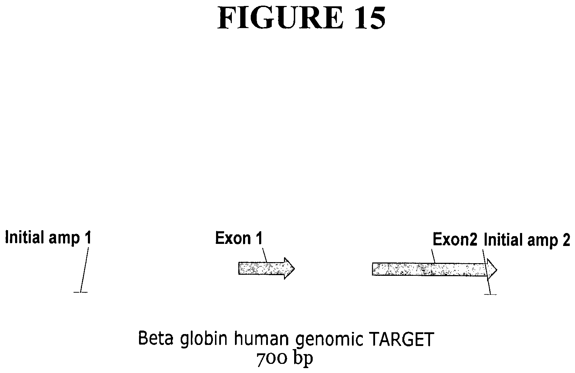

**Figure 15** is a schematic diagram of the cloned human beta globin gene showing the upstream region, first and second exons, first intron and primer binding sites.

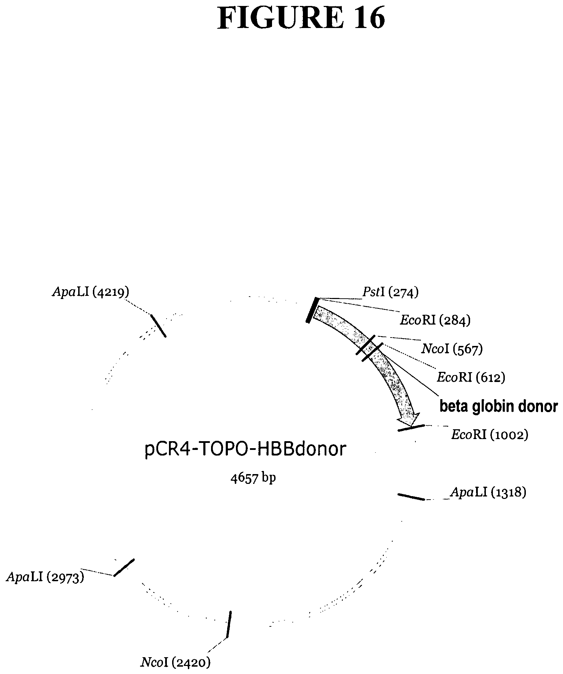

**Figure 16** is a schematic diagram of the beta globin donor construct, pCR4-TOPO-HBBdonor.

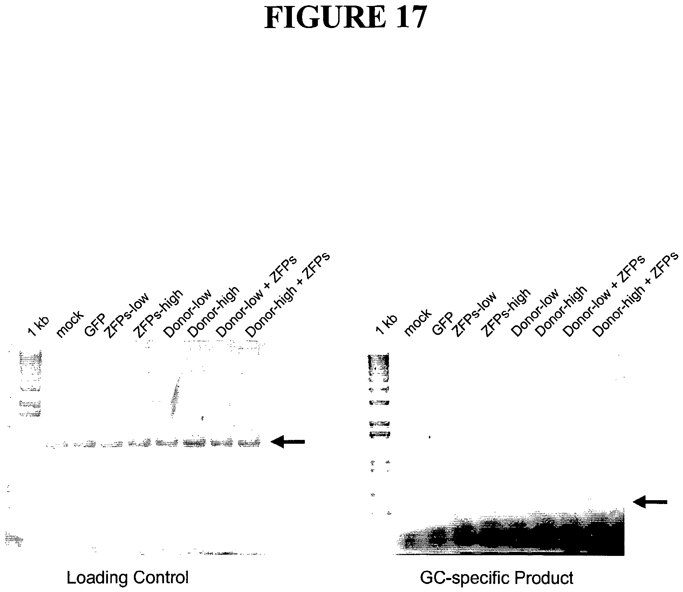

**Figure 17** shows PCR analysis of DNA from cells transfected with two pairs of β-globin-specific ZFP nucleases and a beta globin donor plasmid. The panel on the left is a loading control in which the initial amp 1 and initial amp 2 primers (Table 7) were used for amplification. In the experiment shown in the right panel, the "chromosome-specific and "donor-specific" primers (Table 7) were used for amplification. The leftmost lane in each panel contains molecular weight markers and the next lane shows amplification products obtained from mock-transfected cells. Remaining lanes, from left to right, show amplification product from cells transfected with: a GFP-encoding plasmid, 100ng of each ZFP/*Fok*I-encoding plasmid, 200ng of each ZFP/*Fok*I-encoding plasmid, 200 ng donor plasmid, 600 ng donor plasmid, 200 ng donor plasmid + 100 ng of each ZFP/*Fok*I-encoding plasmid, and 600 ng donor plasmid + 200 ng of each ZFP/*Fok*I-encoding plasmid.

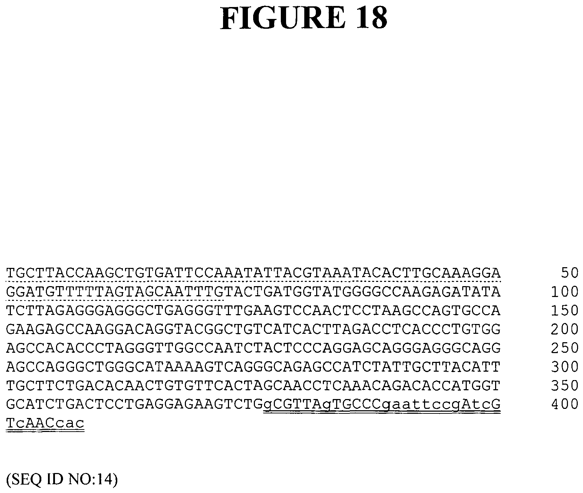

**Figure 18** shows the nucleotide sequence of an amplification product derived from a mutated beta-globin gene (SEQ ID NO:14) generated by targeted homologous recombination. Chromosomal sequences not present in the donor molecule are indicated by dashed underlining (nucleotides 1-72), sequences common to the donor and the chromosome are not underlined (nucleotides 73-376), and a stretch of sequence containing nucleotides which distinguish donor sequences from chromosomal sequences is double-underlined (nucleotides 377-408). Lower-case letters represent nucleotides whose sequence differs between the chromosome and the donor.

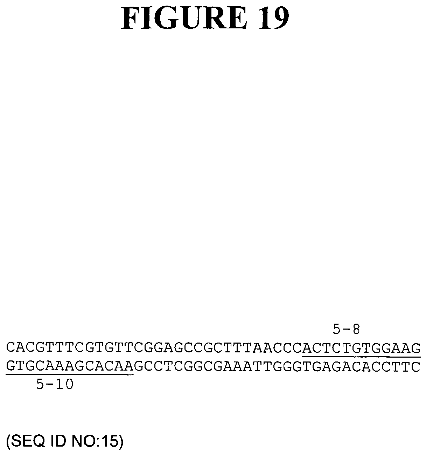

**Figure 19** shows the nucleotide sequence of a portion of the fifth exon of the Interleukin-2 receptor gamma chain (IL-2Rγ) gene (SEQ ID NO:15). Also shown (underlined) are the target sequences for the 5-8 and 5-10 ZFP/*Fok*I fusion proteins. See Example 5 for details.

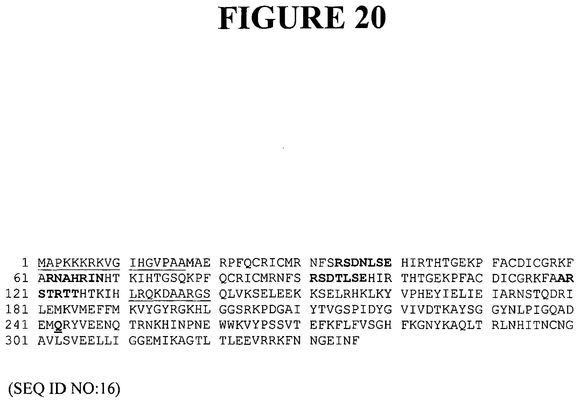

**Figure 20** shows the amino acid sequence of the 5-8 ZFP/*Fok*I fusion targeted to exon 5 of the human IL-2Rγ gene (SEQ ID NO:16). Amino acid residues 1-17 contain a nuclear localization sequence (NLS, underlined); residues 18-130 contain the ZFP portion, with the recognition regions of the component zinc fingers shown in boldface; the ZFP-*Fok*I linker (ZC linker, underlined) extends from residues 131 to 140 and the *Fok*I cleavage half-domain begins at residue 141 and extends to the end of the protein at residue 336. The residue that was altered to generate the Q486E mutation is shown underlined and in boldface.

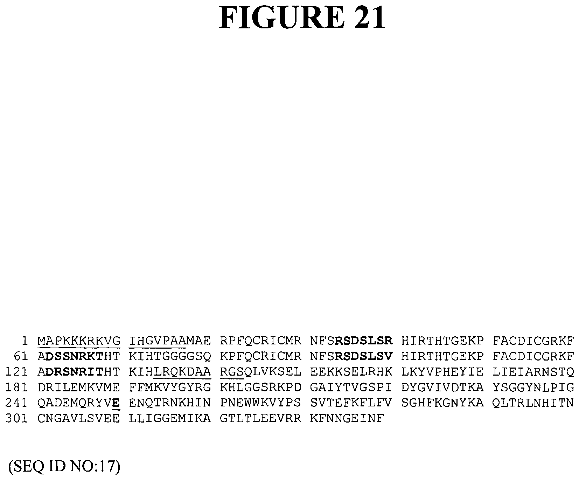

**Figure 21** shows the amino acid sequence of the 5-10 ZFP/*Fok*I fusion targeted to exon 5 of the human IL-2Rγ gene (SEQ ID NO:17). Amino acid residues 1-17 contain a nuclear localization sequence (NLS, underlined); residues 18-133 contain the ZFP portion, with the recognition regions of the component zinc fingers shown in boldface; the ZFP-*Fok*I linker (ZC linker, underlined) extends from residues 134 to 143 and the *Fok*I cleavage half-domain begins at residue 144 and extends to the end of the protein at residue 339. The residue that was altered to generate the E490K mutation is shown underlined and in boldface.

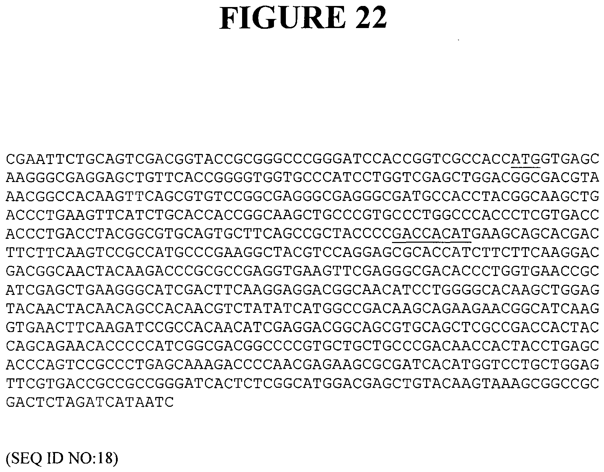

**Figure 22** shows the nucleotide sequence of the enhanced Green Fluorescent Protein gene (SEQ ID NO:18) derived from the *Aequorea victoria* GFP gene (Tsien (1998) Ann. Rev. Biochem. 67:509-544). The ATG initiation codon, as well as the region which was mutagenized, are underlined.

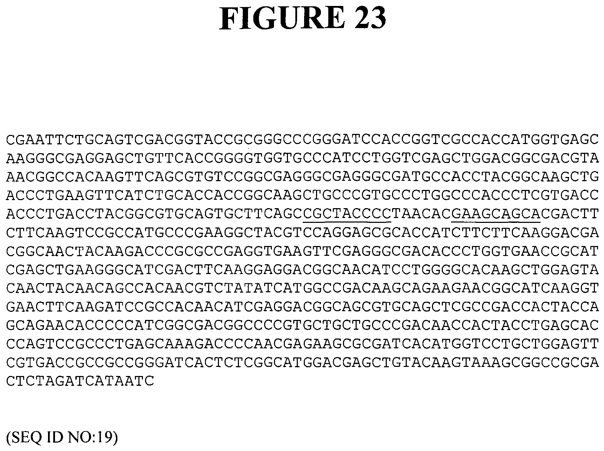

**Figure 23** shows the nucleotide sequence of a mutant defective eGFP gene (SEQ ID NO: 19). Binding sites for ZFP-nucleases are underlined and the region between the binding sites corresponds to the region that was modified.

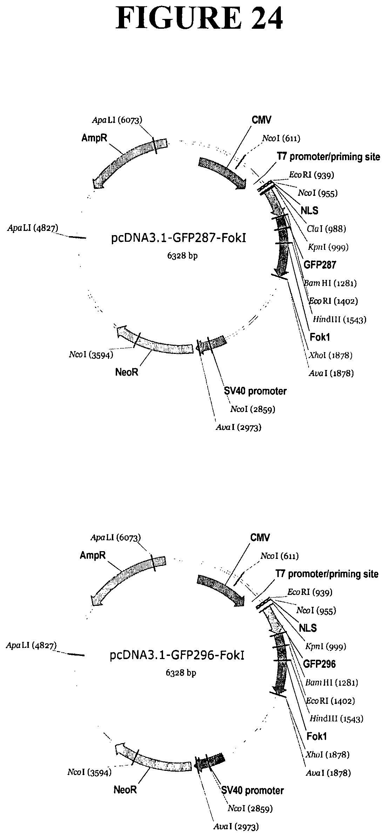

**Figure 24** shows the structures of plasmids encoding Zinc Finger Nucleases targeted to the eGFP gene.

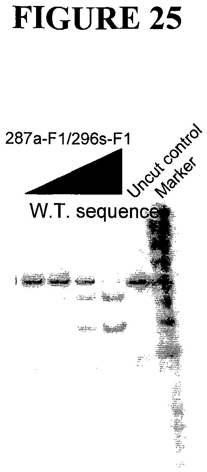

**Figure 25** shows an autoradiogram of a 10% acrylamide gel used to analyze targeted DNA cleavage of a mutant eGFP gene by zinc finger endonucleases. See Example 8 for details.

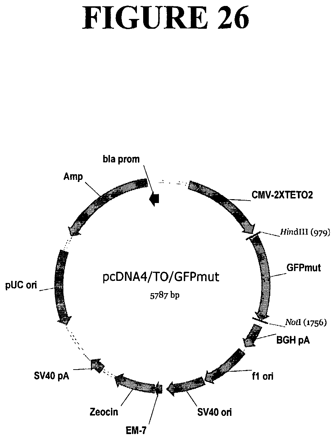

**Figure 26** shows the structure of plasmid pcDNA4/TO/GFPmut (see Example 9).

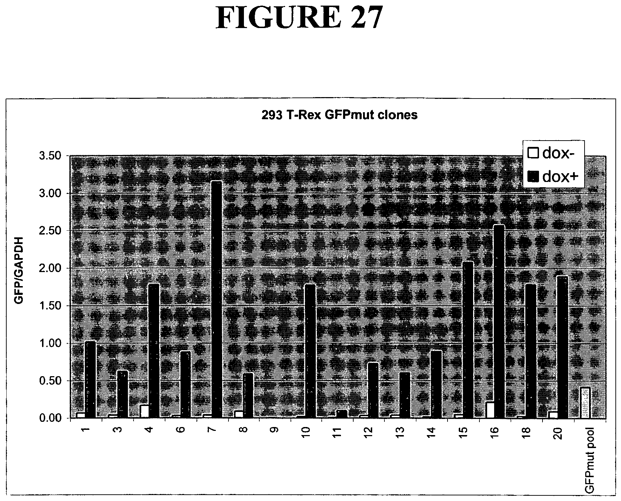

**Figure 27** shows levels of eGFPmut mRNA, normalized to GAPDH mRNA, in various cell lines obtained from transfection of human HEK293 cells. Light bars show levels in untreated cells; dark bars show levels in cell that had been treated with 2 ng/ml doxycycline. See Example 9 for details.

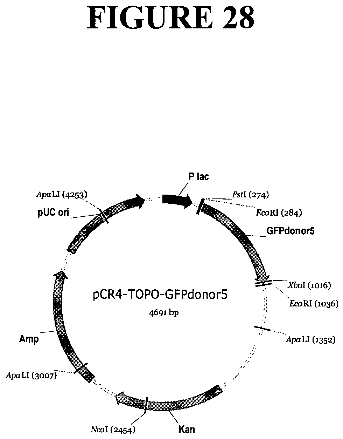

**Figure 28** shows the structure of plasmid pCR(R)4-TOPO-GFPdonor5. See Example 10 for details.

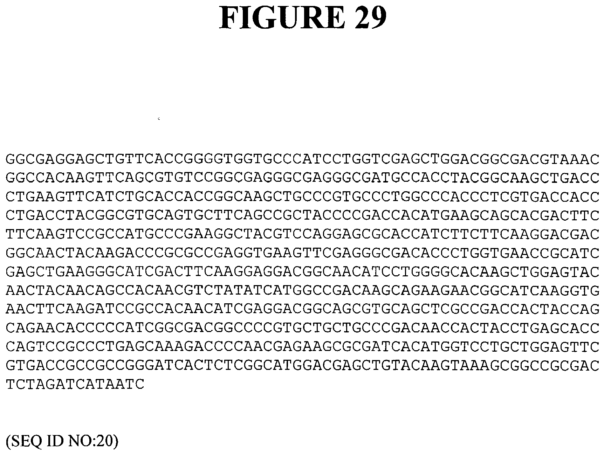

**Figure 29** shows the nucleotide sequence of the eGFP insert in pCR(R)4-TOPO-GFPdonor5 (SEQ ID NO:20). The insert contains sequences encoding a portion of a non-modified enhanced Green Fluorescent Protein, lacking an initiation codon. See Example 10 for details.

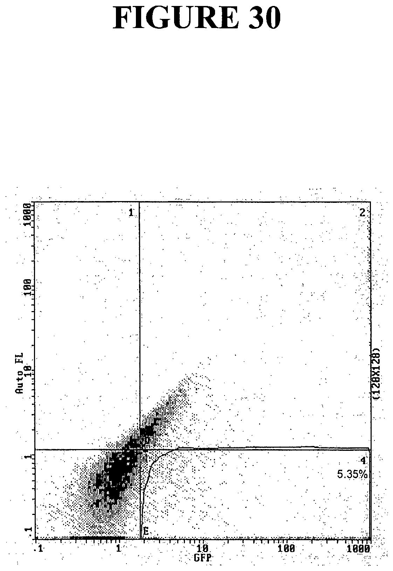

**Figure 30** shows a FACS trace of T18 cells transfected with plasmids encoding two ZFP nucleases and a plasmid encoding a donor sequence, that were arrested in the G2 phase of the cell cycle 24 hours post-transfection with 100 ng/ml nocodazole for 48 hours. The medium was replaced and the cells were allowed to recover for an additional 48 hours, and gene correction was measured by FACS analysis. *See* Example 11 for details.

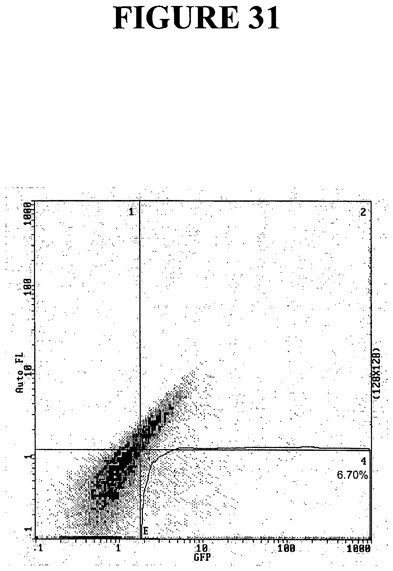

**Figure 31** shows a FACS trace of T18 cells transfected with plasmids encoding two ZFP nucleases and a plasmid encoding a donor sequence, that were arrested in the G2 phase of the cell cycle 24 hours post-transfection with 0.2 uM vinblastine for 48 hours. The medium was replaced and the cells were allowed to recover for an additional 48 hours, and gene correction was measured by FACS analysis. *See* Example 11 for details.

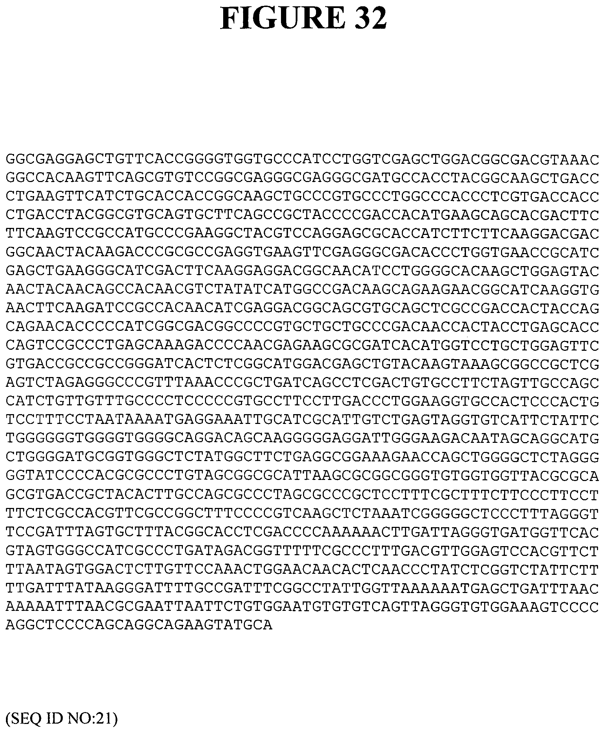

**Figure 32** shows the nucleotide sequence of a 1,527 nucleotide eGFP insert in pCR(R)4-TOPO (SEQ ID NO:21). The sequence encodes a non-modified enhanced Green Fluorescent Protein lacking an initiation codon. *See* Example 13 for details.

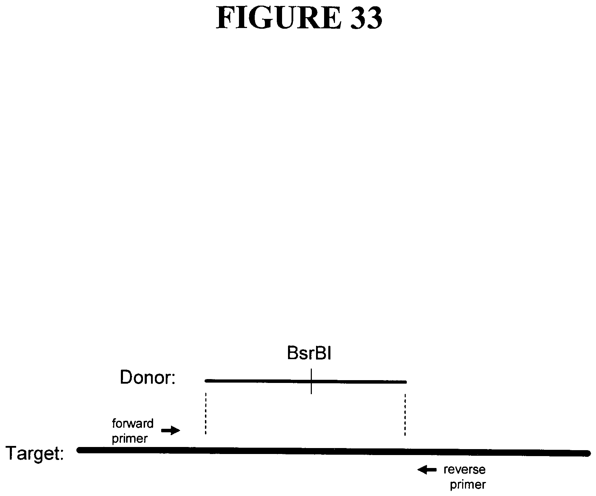

**Figure 33** shows a schematic diagram of an assay used to measure the frequency of editing of the endogenous human IL-2Rγ gene. See Example 14 for details.

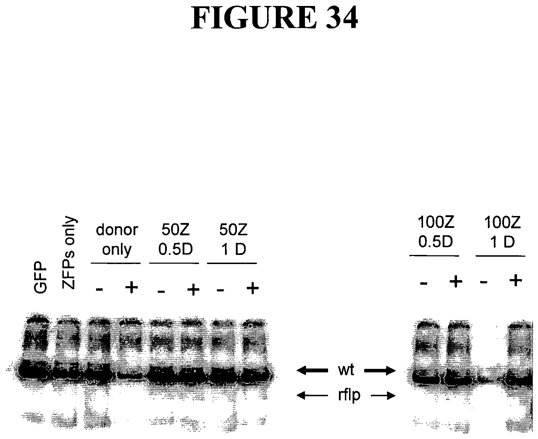

**Figure 34** shows autoradiograms of acrylamide gels used in an assay to measure the frequency of editing of an endogenous cellular gene by targeted cleavage and homologous recombination. The lane labeled "GFP" shows assay results from a control in which cells were transfected with an eGFP-encoding vector; the lane labeled "ZFPs only" shows results from another control experiment in which cells were transfected with the two ZFP/nuclease-encoding plasmids (50 ng of each) but not with a donor sequence. Lanes labeled "donor only" show results from a control experiment in which cells were transfected with 1 ug of donor plasmid but not with the ZFP/nuclease-encoding plasmids. In the experimental lanes, 50Z refers to cells transfected with 50 ng of each ZFP/nuclease expression plasmid, 100Z refers to cells transfected with 100 ng of each ZFP/nuclease expression plasmid, 0.5D refers to cells transfected with 0.5 μg of the donor plasmid, and 1D refers to cells transfected with 1.0 μg of the donor plasmid. "+" refers to cells that were exposed to 0.2 μM vinblastine; "-" refer to cells that were not exposed to vinblastine. "wt" refers to the fragment obtained after *Bsr*BI digestion of amplification products obtained from chromosomes containing the wild-type chromosomal IL-2Rγ gene; "rflp" refers to the two fragments (of approximately equal molecular weight) obtained after *BsrBI* digestion of amplification products obtained from chromosomes containing sequences from the donor plasmid which had integrated by homologous recombination.

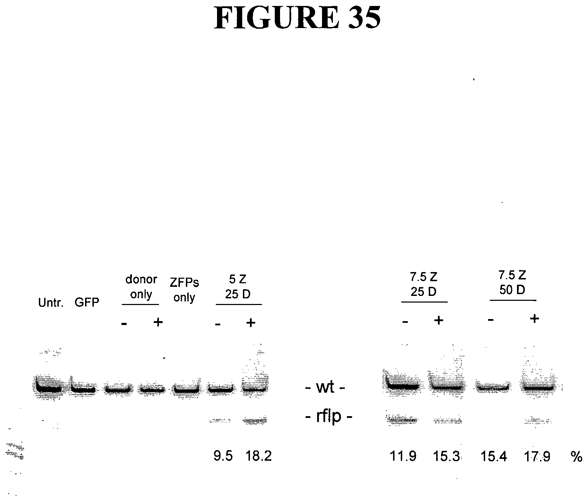

**Figure 35** shows an autoradiographic image of a four-hour exposure of a gel used in an assay to measure targeted recombination at the human IL-2Rγ locus in K562 cells. "wt" identifies a band that is diagnostic for chromosomal DNA containing the native K562 IL-2Rγ sequence; "rflp" identifies a doublet diagnostic for chromosomal DNA containing the altered IL-2Rγ sequence present in the donor DNA molecule. The symbol "+" above a lane indicates that cells were treated with 0.2 uM vinblastine; the symbol "-" indicates that cells were not treated with vinblastine. The numbers in the "ZFP + donor" lanes indicate the percentage of total chromosomal DNA containing sequence originally present in the donor DNA molecule, calculated using the "peak finder, automatic baseline" function of Molecular Dynamics' ImageQuant v. 5.1 software as described in Ch. 8 of the manufacturer's manual (Molecular Dynamics ImageQuant User's Guide; part 218-415). "Untr" indicates untransfected cells. See Example 15 for additional details.

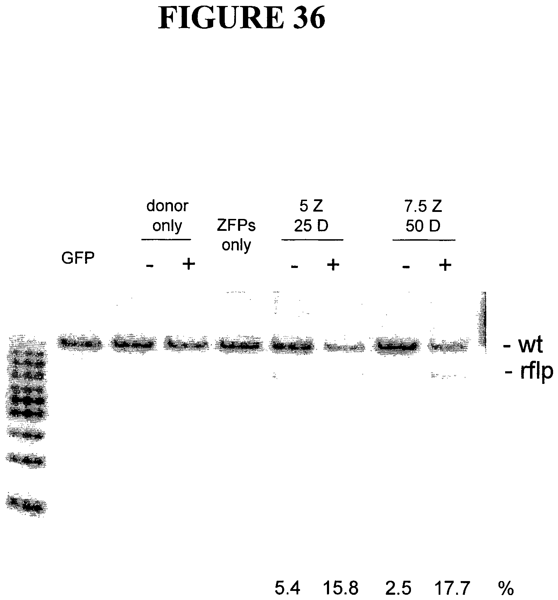

**Figure 36** shows an autoradiographic image of a four-hour exposure of a gel used in an assay to measure targeted recombination at the human IL-2Rγ locus in K562 cells. "wt" identifies a band that is diagnostic for chromosomal DNA containing the native K562 IL-2Rγ sequence; "rflp" identifies a band that is diagnostic for chromosomal DNA containing the altered IL-2Rγ sequence present in the donor DNA molecule. The symbol "+" above a lane indicates that cells were treated with 0.2 uM vinblastine; the symbol "-" indicates that cells were not treated with vinblastine. The numbers beneath the "ZFP + donor" lanes indicate the percentage of total chromosomal DNA containing sequence originally present in the donor DNA molecule, calculated as described in Example 35. See Example 15 for additional details.

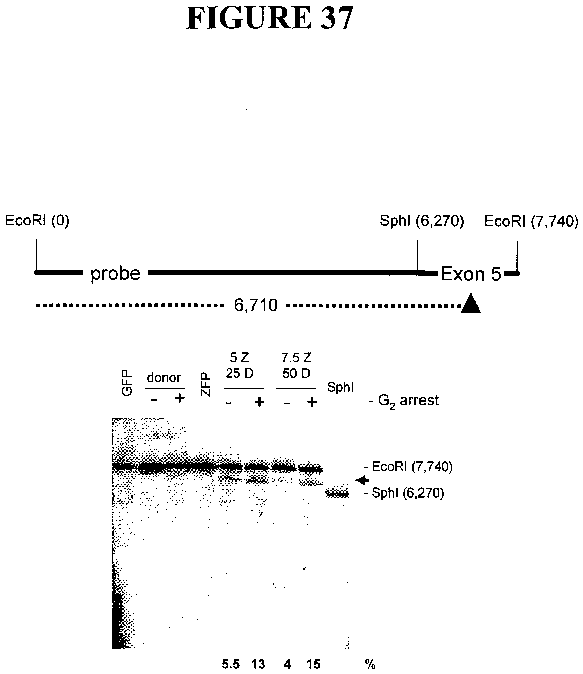

**Figure 37** shows an autoradiogram of a four-hour exposure of a DNA blot probed with a fragment specific to the human IL-2Rγ gene. The arrow to the right of the image indicates the position of a band corresponding to genomic DNA whose sequence has been altered by homologous recombination. The symbol "+" above a lane indicates that cells were treated with 0.2 uM vinblastine; the symbol "-" indicates that cells were not treated with vinblastine. The numbers beneath the "ZFP + donor" lanes indicate the percentage of total chromosomal DNA containing sequence originally present in the donor DNA molecule, calculated as described in Example 35. See Example 15 for additional details.

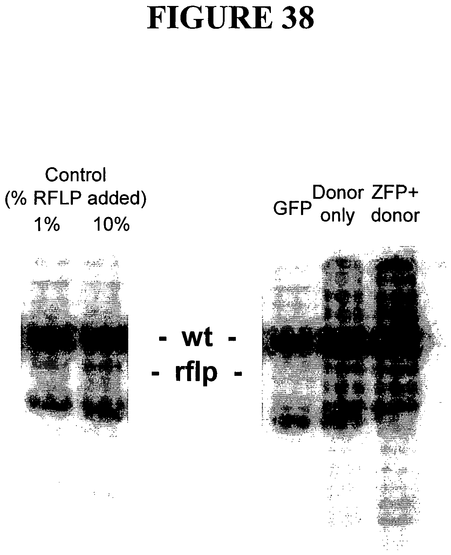

**Figure 38** shows autoradiographic images of gels used in an assay to measure targeted recombination at the human IL-2Rγ locus in CD34[+] human bone marrow cells. The left panel shows a reference standard in which the stated percentage of normal human genomic DNA (containing a *Mae*II site) was added to genomic DNA from Jurkat cells (lacking a *Mae*II site), the mixture was amplified by PCR to generate a radiolabelled amplification product, and the amplification product was digested with *Mae*II. "wt" identifies a band representing undigested DNA, and "rflp" identifies a band resulting from *Mae*II digestion.

The right panel shows results of an experiment in which CD34[+] cells were transfected with donor DNA containing a *Bsr*BI site and plasmids encoding zinc finger-*Fok*I fusion endonucleases. The relevant genomic region was then amplified and labeled, and the labeled amplification product was digested with *Bsr*BI. "GFP" indicates control cells that were transfected with a GFP-encoding plasmid; "Donor only" indicates control cells that were transfected only with donor DNA, and "ZFP + Donor" indicates cells that were transfected with donor DNA and with plasmids encoding the zinc finger/*Fok*I nucleases. "wt" identifies a band that is diagnostic for chromosomal DNA containing the native IL-2Rγ sequence; "rflp" identifies a band that is diagnostic for chromosomal DNA containing the altered IL-2Rγ sequence present in the donor DNA molecule. The rightmost lane contains DNA size markers. See Example 16 for additional details.

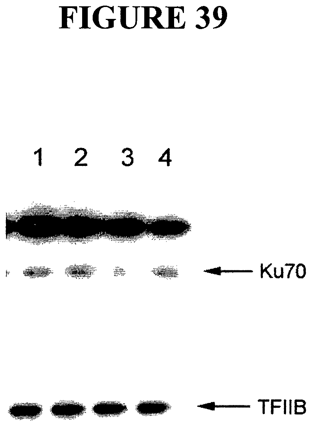

**Figure 39** shows an image of an immunoblot used to test for Ku70 protein levels in cells transfected with Ku70-targeted siRNA. The T7 cell line (Example 9, Figure 27) was transfected with two concentrations each of siRNA from two different siRNA pools (see Example 18). Lane 1: 70 ng of siRNA pool D; Lane 2: 140 ng of siRNA pool D; Lane 3: 70 ng of siRNA pool E; Lane 4: 140 ng of siRNA pool E.. "Ku70" indicates the band representing the Ku70 protein; "TFIIB" indicates a band representing the TFIIB transcription factor, used as a control.

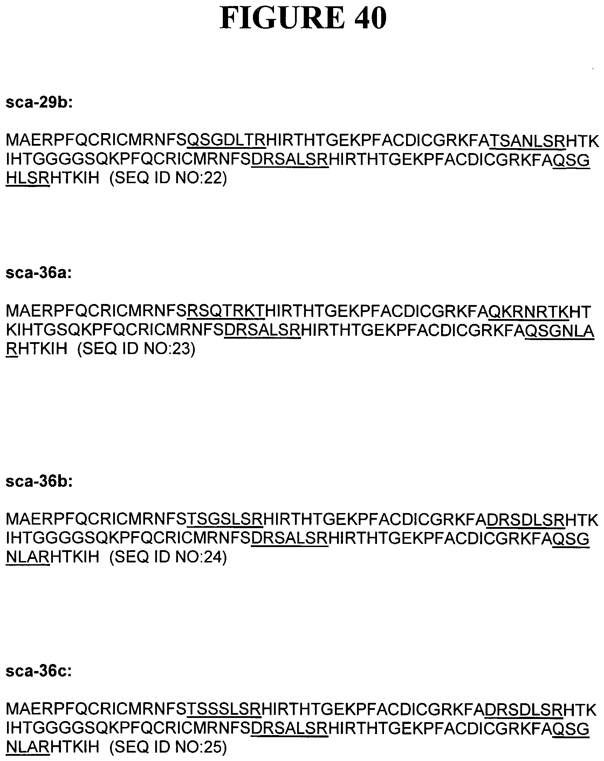

**Figure 40** shows the amino acid sequences of four zinc finger domains targeted to the human β-globin gene: sca-29b (SEQ ID NO:22); sca-36a (SEQ ID NO:23); sca-36b (SEQ ID NO:24) and sca-36c (SEQ ID NO:25). The target site for the sca-29b domain is on one DNA strand, and the target sites for the sca-36a, sca-36b and sca-36c domains are on the opposite strand. *See* Example 20.

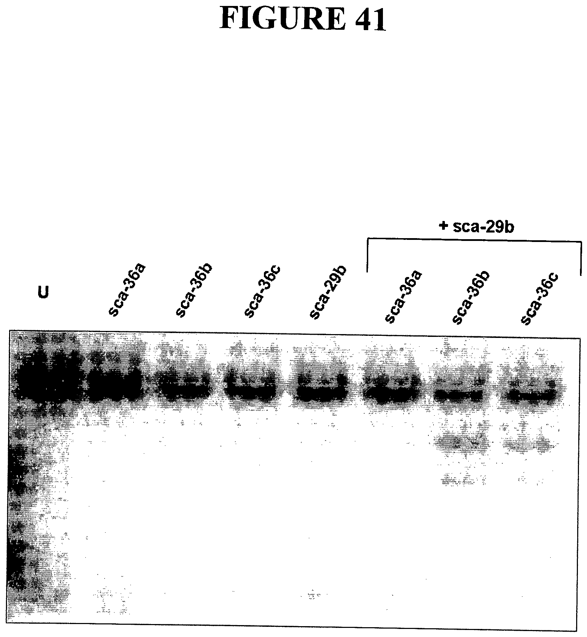

**Figure 41** shows results of an *in vitro* assay, in which different combinations of zinc finger/*Fok*I fusion nucleases (ZFNs) were tested for sequence-specific DNA cleavage. The lane labeled "U" shows a sample of the DNA template. The next four lanes show results of incubation of the DNA template with each of four β-globin-targeted ZFNs (see Example 20 for characterization of these ZFNs). The rightmost three lanes show results of incubation of template DNA with the sca-29b ZFN and one of the sca-36a, sca-36b or sca-36c ZFNs (all of which are targeted to the strand opposite that to which sca-29b is targeted).

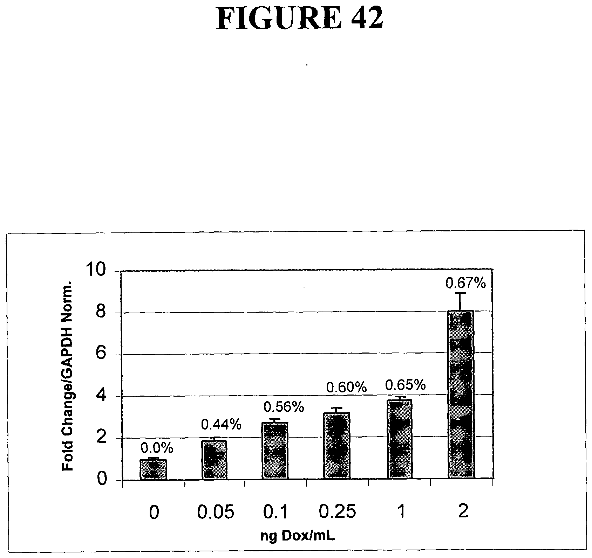

**Figure 42** shows levels of eGFP mRNA in T18 cells (bars) as a function of doxycycline concentration (provided on the abscissa). The number above each bar represents the percentage correction of the eGFP mutation, in cells transfected with donor DNA and plasmids encoding eGFP-targeted zinc finger nucleases, as a function of doxycycline concentration.

DETAILED DESCRIPTION

Disclosed herein are compositions and methods useful for targeted cleavage of cellular chromatin and for targeted alteration of a cellular nucleotide sequence, *e.g.*, by targeted cleavage followed by non-homologous end joining or by targeted cleavage followed by homologous recombination between an exogenous polynucleotide (comprising one or more regions of homology with the cellular nucleotide sequence) and a genomic sequence. Genomic sequences include those present in chromosomes, episomes, organellar genomes (e.g., mitochondria, chloroplasts), artificial chromosomes and any other type of nucleic acid present in a cell such as, for example, amplified sequences, double minute chromosomes and the genomes of endogenous or infecting bacteria and viruses. Genomic sequences can be normal (*i.e.*, wild-type) or mutant; mutant sequences can comprise, for example, insertions, deletions, translocations, rearrangements, and/or point mutations. A genomic sequence can also comprise one of a number of different alleles.

Compositions useful for targeted cleavage and recombination include fusion proteins comprising a cleavage domain (or a cleavage half-domain) and a zinc finger binding domain, polynucleotides encoding these proteins and combinations of polypeptides and polypeptide-encoding polynucleotides. A zinc finger binding domain can comprise one or more zinc fingers (*e.g.*, 2, 3, 4, 5, 6, 7, 8, 9 or more zinc fingers), and can be engineered to bind to any genomic sequence. Thus, by identifying a target genomic region of interest at which cleavage or recombination is desired, one can, according to the methods disclosed herein, construct one or more fusion proteins comprising a cleavage domain (or cleavage half-domain) and a zinc finger domain engineered to recognize a target sequence in said genomic region. The presence of such a fusion protein (or proteins) in a cell will result in binding of the fusion protein(s) to its (their) binding site(s) and cleavage within or near said genomic region. Moreover, if an exogenous polynucleotide homologous to the genomic region is also present in such a cell, homologous recombination occurs at a high rate between the genomic region and the exogenous polynucleotide.

**General**

Practice of the methods, as well as preparation and use of the compositions disclosed herein employ, unless otherwise indicated, conventional techniques in molecular biology, biochemistry, chromatin structure and analysis, computational chemistry, cell culture, recombinant DNA and related fields as are within the skill of the art. These techniques are fully explained in the literature. *See,* for example, Sambrook et al. MOLECULAR CLONING: A LABORATORY MANUAL, Second edition, Cold Spring Harbor Laboratory Press, 1989 and Third edition, 2001; Ausubel et al., CURRENT PROTOCOLS IN MOLECULAR BIOLOGY, John Wiley & Sons, New York, 1987 and periodic updates; the series METHODS IN ENZYMOLOGY, Academic Press, San Diego; Wolffe, CHROMATIN STRUCTURE AND FUNCTION, Third edition, Academic Press, San Diego, 1998; METHODS IN ENZYMOLOGY, Vol. 304, "Chromatin" (P.M. Wassarman and A. P. Wolffe, eds.), Academic Press, San Diego, 1999; and METHODS IN MOLECULAR BIOLOGY, Vol. 119, "Chromatin Protocols" (P.B. Becker, ed.) Humana Press, Totowa, 1999.

**Definitions**

The terms "nucleic acid," "polynucleotide," and "oligonucleotide" are used interchangeably and refer to a deoxyribonucleotide or ribonucleotide polymer, in linear or circular conformation, and in either single- or double-stranded form. For the purposes of the present disclosure, these terms are not to be construed as limiting with respect to the length of a polymer. The terms can encompass known analogues of natural nucleotides, as well as nucleotides that are modified in the base, sugar and/or phosphate moieties (e.g., phosphorothioate backbones). In general, an analogue of a particular nucleotide has the same base-pairing specificity; *i.e.,* an analogue of A will base-pair with T.

The terms "polypeptide," "peptide" and "protein" are used interchangeably to refer to a polymer of amino acid residues. The term also applies to amino acid polymers in which one or more amino acids are chemical analogues or modified derivatives of a corresponding naturally-occurring amino acids.

"Binding" refers to a sequence-specific, non-covalent interaction between macromolecules (*e.g.*, between a protein and a nucleic acid). Not all components of a binding interaction need be sequence-specific (*e.g.*, contacts with phosphate residues in a DNA backbone), as long as the interaction as a whole is sequence-specific. Such interactions are generally characterized by a dissociation constant ($K_d$) of $10^{-6}$ $M^{-1}$ or lower. "Affinity" refers to the strength of binding: increased binding affinity being correlated with a lower $K_d$.

A "binding protein" is a protein that is able to bind non-covalently to another molecule. A binding protein can bind to, for example, a DNA molecule (a DNA-binding protein), an RNA molecule (an RNA-binding protein) and/or a protein molecule (a protein-binding protein). In the case of a protein-binding protein, it can bind to itself (to form homodimers, homotrimers, *etc.*) and/or it can bind to one or more molecules of a different protein or proteins. A binding protein can have more than one type of binding activity. For example, zinc finger proteins have DNA-binding, RNA-binding and protein-binding activity.

A "zinc finger DNA binding protein" (or binding domain) is a protein, or a domain within a larger protein, that binds DNA in a sequence-specific manner through one or more zinc fingers, which are regions of amino acid sequence within the binding domain whose structure is stabilized through coordination of a zinc ion. The term zinc finger DNA binding protein is often abbreviated as zinc finger protein or ZFP.

Zinc finger binding domains can be "engineered" to bind to a predetermined nucleotide sequence. Non-limiting examples of methods for engineering zinc finger proteins are design and selection. A designed zinc finger protein is a protein not occurring in nature whose design/composition results principally from rational criteria. Rational criteria for design include application of substitution rules and computerized algorithms for processing information in a database storing information of existing ZFP designs and binding data. See, for example, US Patents 6,140,081; 6,453,242; and 6,534,261; see also WO 98/53058; WO 98/53059; WO 98/53060; WO 02/016536 and WO 03/016496.

A "selected" zinc finger protein is a protein not found in nature whose production results primarily from an empirical process such as phage display, interaction trap or hybrid selection. See *e.g.,* US 5,789,538; US 5,925,523; US 6,007,988; US 6,013,453; US 6,200,759; WO 95/19431; WO 96/06166; WO 98/53057; WO 98/54311; WO 00/27878; WO 01/60970 WO 01/88197 and WO 02/099084.

The term "sequence" refers to a nucleotide sequence of any length, which can be DNA or RNA; can be linear, circular or branched and can be either single-stranded or double stranded. The term "donor sequence" refers to a nucleotide sequence that is inserted into a genome. A donor sequence can be of any length, for example between 2 and 10,000 nucleotides in length (or any integer value therebetween or thereabove), preferably between about 100 and 1,000 nucleotides in length (or any integer therebetween), more preferably between about 200 and 500 nucleotides in length.

A "homologous, non-identical sequence" refers to a first sequence which shares a degree of sequence identity with a second sequence, but whose sequence is not identical to that of the second sequence. For example, a polynucleotide comprising the wild-type sequence of a mutant gene is homologous and non-identical to the sequence of the mutant gene. In certain embodiments, the degree of homology between the two sequences is sufficient to allow homologous recombination therebetween, utilizing normal cellular mechanisms. Two homologous non-identical sequences can be any length and their degree of non-homology can be as small as a single nucleotide (*e.g.*, for correction of a genomic point mutation by targeted homologous recombination) or as large as 10 or more kilobases (*e.g.*, for insertion of a gene at a predetermined ectopic site in a chromosome). Two polynucleotides comprising the homologous non-identical sequences need not be the same length. For example, an exogenous polynucleotide (*i.e.,* donor polynucleotide) of between 20 and 10,000 nucleotides or nucleotide pairs can be used.

Techniques for determining nucleic acid and amino acid sequence identity are known in the art. Typically, such techniques include determining the nucleotide sequence of the mRNA for a gene and/or determining the amino acid sequence encoded thereby, and comparing these sequences to a second nucleotide or amino acid sequence. Genomic sequences can also be determined and compared in this fashion. In general, identity refers to an exact nucleotide-to-nucleotide or amino acid-to-amino acid correspondence of two polynucleotides or polypeptide sequences, respectively. Two or more sequences (polynucleotide or amino acid) can be compared by determining their percent identity. The percent identity of two sequences, whether nucleic acid or amino acid sequences, is the number of exact matches between two aligned sequences divided by the length of the shorter sequences and multiplied by 100. An approximate alignment for nucleic acid sequences is provided by the local homology algorithm of Smith and Waterman, Advances in Applied Mathematics 2:482-489 (1981). This algorithm can be applied to amino acid sequences by using the scoring matrix developed by Dayhoff, Atlas of Protein Sequences and Structure, M.O. Dayhoff ed., 5 suppl. 3:353-358, National Biomedical Research Foundation, Washington, D.C., USA, and normalized by Gribskov, Nucl. Acids Res. 14(6):6745-6763 (1986). An exemplary implementation of this algorithm to determine percent identity of a sequence is provided by the Genetics Computer Group (Madison, WI) in the "BestFit" utility application. The default parameters for this method are described in the Wisconsin Sequence Analysis Package Program Manual, Version 8 (1995) (available from Genetics Computer Group, Madison, WI). A preferred method of establishing percent identity in the context of the present disclosure is to use the MPSRCH package of programs copyrighted by the University of Edinburgh, developed by John F. Collins and Shane S. Sturrok, and distributed by IntelliGenetics, Inc. (Mountain View, CA). From this suite of packages the Smith-Waterman algorithm can be employed where default parameters are used for the scoring table (for example, gap open penalty of 12, gap extension penalty of one, and a gap of six). From the data generated the "Match" value reflects sequence identity. Other suitable programs for calculating the percent identity or similarity between sequences are generally known in the art, for example, another alignment program is BLAST, used with default parameters. For example, BLASTN and BLASTP can be used using the following default parameters: genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR. Details of these programs can be found at the following internet address: http://www.ncbi.nlm.gov/cgi-bin/BLAST. With respect to sequences described herein, the range of desired degrees of sequence identity is approximately 80% to 100% and any integer value therebetween. Typically the percent identities between sequences are at least 70-75%, preferably 80-82%, more preferably 85-90%, even more preferably 92%, still more preferably 95%, and most preferably 98% sequence identity.

Alternatively, the degree of sequence similarity between polynucleotides can be determined by hybridization of polynucleotides under conditions that allow formation of stable duplexes between homologous regions, followed by digestion with single-stranded-specific nuclease(s), and size determination of the digested fragments. Two nucleic acid, or two polypeptide sequences are substantially homologous to each other when the sequences exhibit at least about 70%-75%, preferably 80%-82%, more preferably 85%-90%, even more preferably 92%, still more preferably 95%, and most preferably 98% sequence identity over a defined length of the molecules, as determined using the methods above. As used herein, substantially homologous also refers to sequences showing complete identity to a specified DNA or polypeptide sequence. DNA sequences that are substantially homologous can be identified in a Southern hybridization experiment under, for example, stringent conditions, as defined for that particular system. Defining appropriate hybridization conditions is within the skill of the art. See, *e.g.,* Sambrook et al., supra; Nucleic Acid Hybridization: A Practical Approach, editors B.D. Hames and S.J. Higgins, (1985) Oxford; Washington, DC; IRL Press).

Selective hybridization of two nucleic acid fragments can be determined as follows. The degree of sequence identity between two nucleic acid molecules affects the efficiency and strength of hybridization events between such molecules. A partially identical nucleic acid sequence will at least partially inhibit the hybridization of a completely identical sequence to a target molecule. Inhibition of hybridization of the completely identical sequence can be assessed using hybridization assays that are well known in the art (*e.g.*, Southern (DNA) blot, Northern (RNA) blot, solution hybridization, or the like, see Sambrook, et al., Molecular Cloning: A Laboratory Manual, Second Edition, (1989) Cold Spring Harbor, N.Y.). Such assays can be conducted using varying degrees of selectivity, for example, using conditions varying from low to high stringency. If conditions of low stringency are employed, the absence of non-specific binding can be assessed using a secondary probe that lacks even a partial degree of sequence identity (for example, a probe having less than about 30% sequence identity with the target molecule), such that, in the absence of non-specific binding events, the secondary probe will not hybridize to the target.

When utilizing a hybridization-based detection system, a nucleic acid probe is chosen that is complementary to a reference nucleic acid sequence, and then by selection of appropriate conditions the probe and the reference sequence selectively hybridize, or bind, to each other to form a duplex molecule. A nucleic acid molecule that is capable of hybridizing selectively to a reference sequence under moderately stringent hybridization conditions typically hybridizes under conditions that allow detection of a target nucleic acid sequence of at least about 10-14 nucleotides in length having at least approximately 70% sequence identity with the sequence of the selected nucleic acid probe. Stringent hybridization conditions typically allow detection of target nucleic acid sequences of at least about 10-14 nucleotides in length having a sequence identity of greater than about 90-95% with the sequence of the selected nucleic acid probe. Hybridization conditions useful for probe/reference sequence hybridization, where the probe and reference sequence have a specific degree of sequence identity, can be determined as is known in the art (see, for example, Nucleic Acid Hybridization: A Practical Approach, editors B.D. Hames and S.J. Higgins, (1985) Oxford; Washington, DC; IRL Press).

Conditions for hybridization are well-known to those of skill in the art. Hybridization stringency refers to the degree to which hybridization conditions disfavor the formation of hybrids containing mismatched nucleotides, with higher stringency correlated with a lower tolerance for mismatched hybrids. Factors that affect the stringency of hybridization are well-known to those of skill in the art and include, but are not limited to, temperature, pH, ionic strength, and concentration of organic solvents such as, for example, formamide and dimethylsulfoxide. As is known to those of skill in the art, hybridization stringency is increased by higher temperatures, lower ionic strength and lower solvent concentrations.

With respect to stringency conditions for hybridization, it is well known in the art that numerous equivalent conditions can be employed to establish a particular stringency by varying, for example, the following factors: the length and nature of the sequences, base composition of the various sequences, concentrations of salts and other hybridization solution components, the presence or absence of blocking agents in the hybridization solutions (e.g., dextran sulfate, and polyethylene glycol), hybridization reaction temperature and time parameters, as well as, varying wash conditions. The selection of a particular set of hybridization conditions is selected following standard methods in the art (see, for example, Sambrook, et al., Molecular Cloning: A Laboratory Manual, Second Edition, (1989) Cold Spring Harbor, N.Y.).

"Recombination" refers to a process of exchange of genetic information between two polynucleotides. For the purposes of this disclosure, "homologous recombination (HR)" refers to the specialized form of such exchange that takes place, for example, during repair of double-strand breaks in cells. This process requires nucleotide sequence homology, uses a "donor" molecule to template repair of a "target" molecule (*i.e.,* the one that experienced the double-strand break), and is variously known as "non-crossover gene conversion" or "short tract gene conversion," because it leads to the transfer of genetic information from the donor to the target. Without wishing to be bound by any particular theory, such transfer can involve mismatch correction of heteroduplex DNA that forms between the broken target and the donor, and/or "synthesis-dependent strand annealing," in which the donor is used to resynthesize genetic information that will become part of the target, and/or related processes. Such specialized HR often results in an alteration of the sequence of the target molecule such that part or all of the sequence of the donor polynucleotide is incorporated into the target polynucleotide.

"Cleavage" refers to the breakage of the covalent backbone of a DNA molecule. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. DNA cleavage can result in the production of either blunt ends or staggered ends. Fusion polypeptides can be used for targeted double-stranded DNA cleavage.

A "cleavage domain" comprises one or more polypeptide sequences which possesses catalytic activity for DNA cleavage. A cleavage domain can be contained in a single polypeptide chain or cleavage activity can result from the association of two (or more) polypeptides.

A "cleavage half-domain" is a polypeptide sequence which, in conjunction with a second polypeptide (either identical or different) forms a complex having cleavage activity (preferably double-strand cleavage activity).

"Chromatin" is the nucleoprotein structure comprising the cellular genome. Cellular chromatin comprises nucleic acid, primarily DNA, and protein, including histones and non-histone chromosomal proteins. The majority of eukaryotic cellular chromatin exists in the form of nucleosomes, wherein a nucleosome core comprises approximately 150 base pairs of DNA associated with an octamer comprising two each of histones H2A, H2B, H3 and H4; and linker DNA (of variable length depending on the organism) extends between nucleosome cores. A molecule of histone H1 is generally associated with the linker DNA. For the purposes of the present disclosure, the term "chromatin" is meant to encompass all types of cellular nucleoprotein, both prokaryotic and eukaryotic. Cellular chromatin includes both chromosomal and episomal chromatin.

A "chromosome," is a chromatin complex comprising all or a portion of the genome of a cell. The genome of a cell is often characterized by its karyotype, which is the collection of all the chromosomes that comprise the genome of the cell. The genome of a cell can comprise one or more chromosomes.

An "episome" is a replicating nucleic acid, nucleoprotein complex or other structure comprising a nucleic acid that is not part of the chromosomal karyotype of a cell. Examples of episomes include plasmids and certain viral genomes.

An "accessible region" is a site in cellular chromatin in which a target site present in the nucleic acid can be bound by an exogenous molecule which recognizes the target site. Without wishing to be bound by any particular theory, it is believed that an accessible region is one that is not packaged into a nucleosomal structure. The distinct structure of an accessible region can often be detected by its sensitivity to chemical and enzymatic probes, for example, nucleases.

A "target site" or "target sequence" is a nucleic acid sequence that defines a portion of a nucleic acid to which a binding molecule will bind, provided sufficient conditions for binding exist. For example, the sequence 5'-GAATTC-3' is a target site for the Eco RI restriction endonuclease.

An "exogenous" molecule is a molecule that is not normally present in a cell, but can be introduced into a cell by one or more genetic, biochemical or other methods. "Normal presence in the cell" is determined with respect to the particular developmental stage and environmental conditions of the cell. Thus, for example, a molecule that is present only during embryonic development of muscle is an exogenous molecule with respect to an adult muscle cell. Similarly, a molecule induced by heat shock is an exogenous molecule with respect to a non-heat-shocked cell. An exogenous molecule can comprise, for example, a functioning version of a malfunctioning endogenous molecule or a malfunctioning version of a normally-functioning endogenous molecule.

An exogenous molecule can be, among other things, a small molecule, such as is generated by a combinatorial chemistry process, or a macromolecule such as a protein, nucleic acid, carbohydrate, lipid, glycoprotein, lipoprotein, polysaccharide, any modified derivative of the above molecules, or any complex comprising one or more of the above molecules. Nucleic acids include DNA and RNA, can be single- or double-stranded; can be linear, branched or circular; and can be of any length. Nucleic acids include those capable of forming duplexes, as well as triplex-forming nucleic acids. See, for example, U.S. Patent Nos. 5,176,996 and 5,422,251. Proteins include, but are not limited to, DNA-binding proteins, transcription factors, chromatin remodeling factors, methylated DNA binding proteins, polymerases, methylases, demethylases, acetylases, deacetylases, kinases, phosphatases, integrases, recombinases, ligases, topoisomerases, gyrases and helicases.

An exogenous molecule can be the same type of molecule as an endogenous molecule, *e.g.*, an exogenous protein or nucleic acid. For example, an exogenous nucleic acid can comprise an infecting viral genome, a plasmid or episome introduced into a cell, or a chromosome that is not normally present in the cell. Methods for the introduction of exogenous molecules into cells are known to those of skill in the art and include, but are not limited to, lipid-mediated transfer (*i.e.*, liposomes, including neutral and cationic lipids), electroporation, direct injection, cell fusion, particle bombardment, calcium phosphate co-precipitation, DEAE-dextran-mediated transfer and viral vector-mediated transfer.

By contrast, an "endogenous" molecule is one that is normally present in a particular cell at a particular developmental stage under particular environmental conditions. For example, an endogenous nucleic acid can comprise a chromosome, the genome of a mitochondrion, chloroplast or other organelle, or a naturally-occurring episomal nucleic acid. Additional endogenous molecules can include proteins, for example, transcription factors and enzymes.

A "fusion" molecule is a molecule in which two or more subunit molecules are linked, preferably covalently. The subunit molecules can be the same chemical type of molecule, or can be different chemical types of molecules. Examples of the first type of fusion molecule include, but are not limited to, fusion proteins (for example, a fusion between a ZFP DNA-binding domain and a cleavage domain) and fusion nucleic acids (for example, a nucleic acid encoding the fusion protein described *supra*). Examples of the second type of fusion molecule include, but are not limited to, a fusion between a triplex-forming nucleic acid and a polypeptide, and a fusion between a minor groove binder and a nucleic acid.

Expression of a fusion protein in a cell can result from delivery of the fusion protein to the cell or by delivery of a polynucleotide encoding the fusion protein to a cell, wherein the polynucleotide is transcribed, and the transcript is translated, to generate the fusion protein. Trans-splicing, polypeptide cleavage and polypeptide ligation can also be involved in expression of a protein in a cell. Methods for polynucleotide and polypeptide delivery to cells are presented elsewhere in this disclosure.

A "gene," for the purposes of the present disclosure, includes a DNA region encoding a gene product (see *infra*), as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

"Gene expression" refers to the conversion of the information, contained in a gene, into a gene product. A gene product can be the direct transcriptional product of a gene (*e.g.*, mRNA, tRNA, rRNA, antisense RNA, ribozyme, structural RNA or any other type of RNA) or a protein produced by translation of a mRNA. Gene products also include RNAs which are modified, by processes such as capping, polyadenylation, methylation, and editing, and proteins modified by, for example, methylation, acetylation, phosphorylation, ubiquitination, ADP-ribosylation, myristilation, and glycosylation.

"Modulation" of gene expression refers to a change in the activity of a gene. Modulation of expression can include, but is not limited to, gene activation and gene repression.

"Eucaryotic" cells include, but are not limited to, fungal cells (such as yeast), plant cells, animal cells, mammalian cells and human cells.

A "region of interest" is any region of cellular chromatin, such as, for example, a gene or a non-coding sequence within or adjacent to a gene, in which it is desirable to bind an exogenous molecule. Binding can be for the purposes of targeted DNA cleavage and/or targeted recombination. A region of interest can be present in a chromosome, an episome, an organellar genome (*e.g.*, mitochondrial, chloroplast), or an infecting viral genome, for example. A region of interest can be within the coding region of a gene, within transcribed non-coding regions such as, for example, leader sequences, trailer sequences or introns, or within non-transcribed regions, either upstream or downstream of the coding region. A region of interest can be as small as a single nucleotide pair or up to 2,000 nucleotide pairs in length, or any integral value of nucleotide pairs.

The terms "operative linkage" and "operatively linked" (or "operably linked") are used interchangeably with reference to a juxtaposition of two or more components (such as sequence elements), in which the components are arranged such that both components function normally and allow the possibility that at least one of the components can mediate a function that is exerted upon at least one of the other components. By way of illustration, a transcriptional regulatory sequence, such as a promoter, is operatively linked to a coding sequence if the transcriptional regulatory sequence controls the level of transcription of the coding sequence in response to the presence or absence of one or more transcriptional regulatory factors. A transcriptional regulatory sequence is generally operatively linked in *cis* with a coding sequence, but need not be directly adjacent to it. For example, an enhancer is a transcriptional regulatory sequence that is operatively linked to a coding sequence, even though they are not contiguous.

With respect to fusion polypeptides, the term "operatively linked" can refer to the fact that each of the components performs the same function in linkage to the other component as it would if it were not so linked. For example, with respect to a fusion polypeptide in which a ZFP DNA-binding domain is fused to a cleavage domain, the ZFP DNA-binding domain and the cleavage domain are in operative linkage if, in the fusion polypeptide, the ZFP DNA-binding domain portion is able to bind its target site and/or its binding site, while the cleavage domain is able to cleave DNA in the vicinity of the target site.

A "functional fragment" of a protein, polypeptide or nucleic acid is a protein, polypeptide or nucleic acid whose sequence is not identical to the full-length protein, polypeptide or nucleic acid, yet retains the same function as the full-length protein, polypeptide or nucleic acid. A functional fragment can possess more, fewer, or the same number of residues as the corresponding native molecule, and/or can contain one ore more amino acid or nucleotide substitutions. Methods for determining the function of a nucleic acid (*e.g.,* coding function, ability to hybridize to another nucleic acid) are well-known in the art. Similarly, methods for determining protein function are well-known. For example, the DNA-binding function of a polypeptide can be determined, for example, by filter-binding, electrophoretic mobility-shift, or immunoprecipitation assays. DNA cleavage can be assayed by gel electrophoresis. See Ausubel *et al.*, *supra.* The ability of a protein to interact with another protein can be determined, for example, by co-immunoprecipitation, two-hybrid assays or complementation, both genetic and biochemical. See, for example, Fields et al. (1989) Nature 340:245-246; U.S. Patent No. 5,585,245 and PCT WO 98/44350.

**Target sites**

The disclosed methods and compositions include fusion proteins comprising a cleavage domain (or a cleavage half-domain) and a zinc finger domain, in which the zinc finger domain, by binding to a sequence in cellular chromatin (e.g., a target site or a binding site), directs the activity of the cleavage domain (or cleavage half-domain) to the vicinity of the sequence and, hence, induces cleavage in the vicinity of the target sequence. As set forth elsewhere in this disclosure, a zinc finger domain can be engineered to bind to virtually any desired sequence. Accordingly, after identifying a region of interest containing a sequence at which cleavage or recombination is desired, one or more zinc finger binding domains can be engineered to bind to one or more sequences in the region of interest. Expression of a fusion protein comprising a zinc finger binding domain and a cleavage domain (or of two fusion proteins, each comprising a zinc finger binding domain and a cleavage half-domain), in a cell, effects cleavage in the region of interest.

Selection of a sequence in cellular chromatin for binding by a zinc finger domain (*e.g.,* a target site) can be accomplished, for example, according to the methods disclosed in co-owned US Patent No. 6,453,242 (Sept. 17, 2002), which also discloses methods for designing ZFPs to bind to a selected sequence. It will be clear to those skilled in the art that simple visual inspection of a nucleotide sequence can also be used for selection of a target site. Accordingly, any means for target site selection can be used in the claimed methods.

Target sites are generally composed of a plurality of adjacent target subsites. A target subsite refers to the sequence (usually either a nucleotide triplet, or a nucleotide quadruplet that can overlap by one nucleotide with an adjacent quadruplet) bound by an individual zinc finger. *See,* for example, WO 02/077227. If the strand with which a zinc finger protein makes most contacts is designated the target strand "primary recognition strand," or "primary contact strand," some zinc finger proteins bind to a three base triplet in the target strand and a fourth base on the non-target strand. A target site generally has a length of at least 9 nucleotides and, accordingly, is bound by a zinc finger binding domain comprising at least three zinc fingers. However binding of, for example, a 4-finger binding domain to a 12-nucleotide target site, a 5-finger binding domain to a 15-nucleotide target site or a 6-finger binding domain to an 18-nucleotide target site, is also possible. As will be apparent, binding of larger binding domains (*e.g.*, 7-, 8-, 9-finger and more) to longer target sites is also possible.

It is not necessary for a target site to be a multiple of three nucleotides. For example, in cases in which cross-strand interactions occur (see, *e.g.,* US Patent 6,453,242 and WO 02/077227), one or more of the individual zinc fingers of a multi-finger binding domain can bind to overlapping quadruplet subsites. As a result, a three-finger protein can bind a 10-nucleotide sequence, wherein the tenth nucleotide is part of a quadruplet bound by a terminal finger, a four-finger protein can bind a 13-nucleotide sequence, wherein the thirteenth nucleotide is part of a quadruplet bound by a terminal finger, *etc.*

The length and nature of amino acid linker sequences between individual zinc fingers in a multi-finger binding domain also affects binding to a target sequence. For example, the presence of a so-called "non-canonical linker," "long linker" or "structured linker" between adjacent zinc fingers in a multi-finger binding domain can allow those fingers to bind subsites which are not immediately adjacent. Non-limiting examples of such linkers are described, for example, in US Patent No. 6,479,626 and WO 01/53480. Accordingly, one or more subsites, in a target site for a zinc finger binding domain, can be separated from each other by 1, 2, 3, 4, 5 or more nucleotides. To provide but one example, a four-finger binding domain can bind to a 13-nucleotide target site comprising, in sequence, two contiguous 3-nucleotide subsites, an intervening nucleotide, and two contiguous triplet subsites.

Distance between sequences (*e.g.*, target sites) refers to the number of nucleotides or nucleotide pairs intervening between two sequences, as measured from the edges of the sequences nearest each other.

Where cleavage depends on the binding of two zinc finger domain/cleavage half-domain fusion molecules to separate target sites, the two target sites can be on opposite DNA strands or both target sites can be on the same DNA strand.

### Zinc finger binding domains

A zinc finger binding domain comprises one or more zinc fingers. Miller et al. (1985) EMBO J. 4:1609-1614; Rhodes (1993) Scientific American Feb.:56-65; US Patent No. 6,453,242. Typically, a single zinc finger domain is about 30 amino acids in length. Structural studies have demonstrated that each zinc finger domain (motif) contains two beta sheets (held in a beta turn which contains the two invariant cysteine residues) and an alpha helix (containing the two invariant histidine residues), which are held in a particular conformation through coordination of a zinc atom by the two cysteines and the two histidines.

Zinc fingers include both canonical $C_2H_2$ zinc fingers (*i.e.,* those in which the zinc ion is coordinated by two cysteine and two histidine residues) and non-canonical zinc fingers such as, for example, $C_3H$ zinc fingers (those in which the zinc ion is coordinated by three cysteine residues and one histidine residue) and $C_4$ zinc fingers (those in which the zinc ion is coordinated by four cysteine residues). See also WO 02/057293.

Zinc finger binding domains can be engineered to bind to a sequence of choice. *See,* for example, Beerli et al. (2002) Nature Biotechnol. 20:135-141; Pabo et al. (2001) Ann. Rev. Biochem. 70:313-340; Isalan et al. (2001) Nature Biotechnol. 19:656-660; Segal et al. (2001) Curr. Opin. Biotechnol. 12:632-637; Choo et al. (2000) Curr. Opin. Struct. Biol. 10:411-416. An engineered zinc finger binding domain can have a novel binding specificity, compared to a naturally-occurring zinc finger protein. Engineering methods include, but are not limited to, rational design and various types of selection. Rational design includes, for example, using databases comprising triplet (or quadruplet) nucleotide sequences and individual zinc finger amino acid sequences, in which each triplet or quadruplet nucleotide sequence is associated with one or more amino acid sequences of zinc fingers which bind the particular triplet or quadruplet sequence. See, for example, co-owned U.S. Patents 6,453,242 and 6,534,261.

Exemplary selection methods, including phage display and two-hybrid systems, are disclosed in US Patents 5,789,538; 5,925,523; 6,007,988; 6,013,453; 6,410,248; 6,140,466; 6,200,759; and 6,242,568; as well as WO 98/37186; WO 98/53057; WO 00/27878; WO 01/88197 and GB 2,338,237.

Enhancement of binding specificity for zinc finger binding domains has been described, for example, in co-owned WO 02/077227.

Since an individual zinc finger binds to a three-nucleotide (*i.e.,* triplet) sequence (or a four-nucleotide sequence which can overlap, by one nucleotide, with the four-nucleotide binding site of an adjacent zinc finger), the length of a sequence to which a zinc finger binding domain is engineered to bind (*e.g.,* a target sequence) will determine the number of zinc fingers in an engineered zinc finger binding domain. For example, for ZFPs in which the finger motifs do not bind to overlapping subsites, a six-nucleotide target sequence is bound by a two-finger binding domain; a nine-nucleotide target sequence is bound by a three-finger binding domain, *etc.* As noted herein, binding sites for individual zinc fingers (*i.e.,* subsites) in a target site need not be contiguous, but can be separated by one or several nucleotides, depending on the length and nature of the amino acids sequences between the zinc fingers (*i.e.,* the inter-finger linkers) in a multi-finger binding domain.

In a multi-finger zinc finger binding domain, adjacent zinc fingers can be separated by amino acid linker sequences of approximately 5 amino acids (so-called "canonical" inter-finger linkers) or, alternatively, by one or more non-canonical linkers. See, *e.g.,* co-owned US Patent Nos. 6,453,242 and 6,534,261. For engineered zinc finger binding domains comprising more than three fingers, insertion of longer ("non-canonical") inter-finger linkers between certain of the zinc fingers may be preferred as it may increase the affinity and/or specificity of binding by the binding domain. See, for example, U.S. Patent No. 6,479,626 and WO 01/53480. Accordingly, multi-finger zinc finger binding domains can also be characterized with respect to the presence and location of non-canonical inter-finger linkers. For example, a six-finger zinc finger binding domain comprising three fingers (joined by two canonical inter-finger linkers), a long linker and three additional fingers (joined by two canonical inter-finger linkers) is denoted a 2x3 configuration. Similarly, a binding domain comprising two fingers (with a canonical linker therebetween), a long linker and two additional fingers (joined by a canonical linker) is denoted a 2x2 protein. A protein comprising three two-finger units (in each of which the two fingers are joined by a canonical linker), and in which each two-finger unit is joined to the adjacent two finger unit by a long linker, is referred to as a 3x2 protein.

The presence of a long or non-canonical inter-finger linker between two adjacent zinc fingers in a multi-finger binding domain often allows the two fingers to bind to subsites which are not immediately contiguous in the target sequence. Accordingly, there can be gaps of one or more nucleotides between subsites in a target site; *i.e.,* a target site can contain one or more nucleotides that are not contacted by a zinc finger. For example, a 2x2 zinc finger binding domain can bind to two six-nucleotide sequences separated by one nucleotide, *i.e.*, it binds to a 13-nucleotide target site. *See* also Moore et al. (2001a) Proc. Natl. Acad. Sci. USA 98:1432-1436; Moore et al. (2001b) Proc. Natl. Acad. Sci. USA 98:1437-1441 and WO 01/53480.

As mentioned previously, a target subsite is a three- or four-nucleotide sequence that is bound by a single zinc finger. For certain purposes, a two-finger unit is denoted a binding module. A binding module can be obtained by, for example, selecting for two adjacent fingers in the context of a multi-finger protein (generally three fingers) which bind a particular six-nucleotide target sequence. Alternatively, modules can be constructed by assembly of individual zinc fingers. See also WO 98/53057 and WO 01/53480.

**Cleavage domains**

The cleavage domain portion of the fusion proteins disclosed herein can be obtained from any endo- or exonuclease. Exemplary endonucleases from which a cleavage domain can be derived include, but are not limited to, restriction endonucleases and homing endonucleases. *See,* for example, 2002-2003 Catalogue, New England Biolabs, Beverly, MA; and Belfort et al. (1997) Nucleic Acids Res. 25:3379-3388. Additional enzymes which cleave DNA are known (*e.g.*, S1 Nuclease; mung bean nuclease; pancreatic DNase I; micrococcal nuclease; yeast HO endonuclease; *see* also Linn et al. (eds.) Nucleases, Cold Spring Harbor Laboratory Press, 1993). One or more of these enzymes (or functional fragments thereof) can be used as a source of cleavage domains and cleavage half-domains.

Similarly, a cleavage half-domain (*e.g.*, fusion proteins comprising a zinc finger binding domain and a cleavage half-domain) can be derived from any nuclease or portion thereof, as set forth above, that requires dimerization for cleavage activity. In general, two fusion proteins are required for cleavage if the fusion proteins comprise cleavage half-domains. The two cleavage half-domains can be derived from the same endonuclease (or functional fragments thereof), or each cleavage half-domain can be derived from a different endonuclease (or functional fragments thereof). In addition, the target sites for the two fusion proteins are preferably disposed, with respect to each other, such that binding of the two fusion proteins places the cleavage half-domains in a spatial orientation to each other that allows the cleavage half-domains to form a functional cleavage domain, *e.g.*, by dimerizing. Thus, in certain embodiments, the near edges of the target sites are separated by 5-8 nucleotides or by 15-18 nucleotides. However any integral number of nucleotides or nucleotide pairs can intervene between two target sites (*e.g.,* from 2 to 50 nucleotides or more). In general, the point of cleavage lies between the target sites.

In general, if two fusion proteins are used, each comprising a cleavage half-domain, the primary contact strand for the zinc finger portion of each fusion protein will be on a different DNA strands and in opposite orientation. That is, for a pair of ZFP/cleavage half-domain fusions, the target sequences are on opposite strands and the two proteins bind in opposite orientations.

Restriction endonucleases (restriction enzymes) are present in many species and are capable of sequence-specific binding to DNA (at a recognition site), and cleaving DNA at or near the site of binding. Certain restriction enzymes (*e.g.*, Type IIS) cleave DNA at sites removed from the recognition site and have separable binding and cleavage domains. For example, the Type IIS enzyme *Fok* I catalyzes double-stranded cleavage of DNA, at 9 nucleotides from its recognition site on one strand and 13 nucleotides from its recognition site on the other. See, for example, US Patents 5,356,802;

5,436,150 and 5,487,994; as well as Li et al. (1992) Proc. Natl. Acad. Sci. USA 89:4275-4279; Li et al. (1993) Proc. Natl. Acad. Sci. USA 90:2764-2768; Kim et al. (1994a) Proc. Natl. Acad. Sci. USA 91:883-887; Kim et al. (1994b) J. Biol. Chem. 269:31,978-31,982. Fusion proteins can comprise the cleavage domain (or cleavage half-domain) from at least one Type IIS restriction enzyme and one or more zinc finger binding domains, which may or may not be engineered.

An exemplary Type IIS restriction enzyme, whose cleavage domain is separable from the binding domain, is *Fok* I. This particular enzyme is active as a dimer. Bitinaite et al. (1998) Proc. Natl. Acad. Sci. USA 95: 10,570-10,575. Accordingly, for the purposes of the present disclosure, the portion of the Fok I enzyme used in the disclosed fusion proteins is considered a cleavage half-domain. Thus, for targeted double-stranded cleavage and/or targeted replacement of cellular sequences using zinc finger-*Fok* I fusions, two fusion proteins, each comprising a *Fok*I cleavage half-domain, can be used to reconstitute a catalytically active cleavage domain. Alternatively, a single polypeptide molecule containing a zinc finger binding domain and two *Fok* I cleavage half-domains can also be used. Parameters for targeted cleavage and targeted sequence alteration using zinc finger-Fok I fusions are provided elsewhere in this disclosure.

Exemplary Type IIS restriction enzymes are listed in Table 1. Additional restriction enzymes also contain separable binding and cleavage domains, and these are contemplated by the present disclosure. See, for example, Roberts et al. (2003) Nucleic Acids Res. 31:418-420.

**Table 1: Some Type IIS Restriction Enzymes**

| | | |
|---|---|---|
| Aar I | BsrB I | SspD5 I |
| Ace III | BsrD I | Sth132 I |
| Aci I | BstF5 I | Sts I |
| Alo I | Btr I | TspDT I |
| Bae I | Bts I | TspGW I |
| Bbr7 I | Cdi I | Tth111 II |
| Bbv I | CjeP I | UbaP I |
| Bbv II | Drd II | Bsa I |
| BbvC I | Eci I | BsmB I |
| Bcc I | Eco31 I | |
| Bce83 I | Eco57 I | |
| BceA I | Eco57M I | |
| Bcef I | Esp3 I | |
| Bcg I | Fau I | |
| BciV I | Fin I | |
| Bfi I | Fok I | |
| Bin I | Gdi II | |
| Bmg I | Gsu I | |
| Bpu10 I | Hga I | |
| BsaX I | Hin4 II | |
| Bsb I | Hph I | |
| BscA I | Ksp632 I | |
| BscG I | Mbo II | |
| BseR I | Mly I | |
| BseY I | Mme I | |
| Bsi I | Mnl I | |
| Bsm I | Pfl1108 I | |
| BsmA I | Ple I | |
| BsmF I | Ppi I | |
| Bsp24 I | Psr I | |
| BspG I | RleA I | |
| BspM I | Sap I | |
| BspNC I | SfaN I | |
| Bsr I | Sim I | |

**Zinc finger domain-cleavage domain fusions**

Methods for design and construction of fusion proteins (and polynucleotides encoding same) are known to those of skill in the art. For example, methods for the design and construction of fusion protein comprising zinc finger proteins (and polynucleotides encoding same) are described in co-owned US Patents 6,453,242 and 6,534,261. In certain embodiments, polynucleotides encoding such fusion proteins are constructed. These polynucleotides can be inserted into a vector and the vector can be introduced into a cell (see below for additional disclosure regarding vectors and methods for introducing polynucleotides into cells).

In certain methods described herein, a fusion protein comprises a zinc finger binding domain and a cleavage half-domain from the *Fok* I restriction enzyme, and two such fusion proteins are expressed in a cell. Expression of two fusion proteins in a cell can result from delivery of the two proteins to the cell; delivery of one protein and one nucleic acid encoding one of the proteins to the cell; delivery of two nucleic acids, each encoding one of the proteins, to the cell; or by delivery of a single nucleic acid, encoding both proteins, to the cell. A fusion protein may comprise a single polypeptide chain comprising two cleavage half domains and a zinc finger binding domain. In this case, a single fusion protein is expressed in a cell and, without wishing to be bound by theory, is believed to cleave DNA as a result of formation of an intramolecular dimer of the cleavage half-domains.

In general, the components of the fusion proteins (*e.g*, ZFP-*Fok* I fusions) are arranged such that the zinc finger domain is nearest the amino terminus of the fusion protein, and the cleavage half-domain is nearest the carboxy-terminus. This mirrors the relative orientation of the cleavage domain in naturally-occurring dimerizing cleavage domains such as those derived from the *Fok* I enzyme, in which the DNA-binding domain is nearest the amino terminus and the cleavage half-domain is nearest the carboxy terminus.

In the disclosed fusion proteins, the amino acid sequence between the zinc finger binding domain (which is delimited by the N-terminal most of the two conserved cysteine residues and the C-terminal-most of the two conserved histidine residues) and the cleavage domain (or half-domain) is denoted the "ZC linker." The ZC linker is to be distinguished from the inter-finger linkers discussed above. For instance, in a ZFP-*Fok* I fusion protein (in which the components are arranged: N terminus-zinc finger binding domain-*Fok* I cleavage half domain-C terminus), the ZC linker is located between the second histidine residue of the C-terminal-most zinc finger and the N-terminal-most amino acid residue of the cleavage half-domain (which is generally glutamine (Q) in the sequence QLV). The ZC linker can be any amino acid sequence. To obtain optimal cleavage, the length of the linker and the distance between the target sites (binding sites) are interrelated. *See,* for example, Smith et al. (2000) Nucleic Acids Res. 28:3361-3369; Bibikova et al. (2001) Mol. Cell. Biol. 21:289-297, noting that their notation for linker length differs from that given here. For example, for ZFP-*Fok* I fusions having a ZC linker length of four amino acids (as defined herein), optimal cleavage occurs when the binding sites for the fusion proteins are located 6 or 16 nucleotides apart (as measured from the near edge of each binding site).

**Methods for targeted cleavage**

The disclosed methods and compositions can be used to cleave DNA at a region of interest in cellular chromatin (*e.g.*, at a desired or predetermined site in a genome, for example, in a gene, either mutant or wild-type). For such targeted DNA cleavage, a zinc finger binding domain is engineered to bind a target site at or near the predetermined cleavage site, and a fusion protein comprising the engineered zinc finger binding domain and a cleavage domain is expressed in a cell. Upon binding of the zinc finger portion of the fusion protein to the target site, the DNA is cleaved near the target site by the cleavage domain. The exact site of cleavage can depend on the length of the ZC linker.

Alternatively, two fusion proteins, each comprising a zinc finger binding domain and a cleavage half-domain, are expressed in a cell, and bind to target sites which are juxtaposed in such a way that a functional cleavage domain is reconstituted and DNA is cleaved in the vicinity of the target sites. In one embodiment, cleavage occurs between the target sites of the two zinc finger binding domains. One or both of the zinc finger binding domains can be engineered.

For targeted cleavage using a zinc finger binding domain-cleavage domain fusion polypeptide, the binding site can encompass the cleavage site, or the near edge of the binding site can be 1, 2, 3, 4, 5, 6, 10, 25, 50 or more nucleotides (or any integral value between 1 and 50 nucleotides) from the cleavage site. The exact location of the binding site, with respect to the cleavage site, will depend upon the particular cleavage domain, and the length of the ZC linker. For methods in which two fusion polypeptides, each comprising a zinc finger binding domain and a cleavage half-domain, are used, the binding sites generally straddle the cleavage site. Thus the near edge of the first binding site can be 1, 2, 3, 4, 5, 6, 10, 25 or more nucleotides (or any integral value between 1 and 50 nucleotides) on one side of the cleavage site, and the near edge of the second binding site can be 1, 2, 3, 4, 5, 6, 10, 25 or more nucleotides (or any integral value between 1 and 50 nucleotides) on the other side of the cleavage site. Methods for mapping cleavage sites *in vitro* and *in vivo* are known to those of skill in the art.

Thus, the methods described herein can employ an engineered zinc finger binding domain fused to a cleavage domain. In these cases, the binding domain is engineered to bind to a target sequence, at or near which cleavage is desired. The fusion protein, or a polynucleotide encoding same, is introduced into a cell. Once introduced into, or expressed in, the cell, the fusion protein binds to the target sequence and cleaves at or near the target sequence. The exact site of cleavage depends on the nature of the cleavage domain and/or the presence and/or nature of linker sequences between the binding and cleavage domains. In cases where two fusion proteins, each comprising a cleavage half-domain, are used, the distance between the near edges of the binding sites can be 1, 2, 3, 4, 5, 6, 7, 8, 9, 10, 25 or more nucleotides (or any integral value between 1 and 50 nucleotides). Optimal levels of cleavage can also depend on both the distance between the binding sites of the two fusion proteins (*See,* for example, Smith et al. (2000) Nucleic Acids Res. 28:3361-3369; Bibikova et al. (2001) Mol. Cell. Biol. 21:289-297) and the length of the ZC linker in each fusion protein.

For ZFP-*Fok*I fusion nucleases, the length of the linker between the ZFP and the *Fok*I cleavage half-domain (*i.e.,* the ZC linker) can influence cleavage efficiency. In one experimental system utilizing a ZFP-*Fok*I fusion with a ZC linker of 4 amino acid residues, optimal cleavage was obtained when the near edges of the binding sites for two ZFP-*Fok*I nucleases were separated by 6 base pairs. This particular fusion nuclease comprised the following amino acid sequence between the zinc finger portion and the nuclease half-domain:

HQRTHQNKKQLV (SEQ ID NO:26)

in which the two conserved histidines in the C-terminal portion of the zinc finger and the first three residues in the *Fok*I cleavage half-domain are underlined. Accordingly, the linker sequence in this construct is QNKK. Bibikova et al. (2001) Mol. Cell. Biol. 21:289-297. The present inventors have constructed a number of ZFP-*Fok*I fusion nucleases having a variety of ZC linker lengths and sequences, and analyzed the cleavage efficiencies of these nucleases on a series of substrates having different distances between the ZFP binding sites. *See* Example 4.

The cleavage domain may comprise two cleavage half-domains, both of which are part of a single polypeptide comprising a binding domain, a first cleavage half-domain and a second cleavage half-domain. The cleavage half-domains can have the same amino acid sequence or different amino acid sequences, so long as they function to cleave the DNA.

Cleavage half-domains may also be provided in separate molecules. For example, two fusion polypeptides may be introduced into a cell, wherein each polypeptide comprises a binding domain and a cleavage half-domain. The cleavage half-domains can have the same amino acid sequence or different amino acid sequences, so long as they function to cleave the DNA. Further, the binding domains bind to target sequences which are typically disposed in such a way that, upon binding of the fusion polypeptides, the two cleavage half-domains are presented in a spatial orientation to each other that allows reconstitution of a cleavage domain (*e.g.*, by dimerization of the half-domains), thereby positioning the half-domains relative to each other to form a functional cleavage domain, resulting in cleavage of cellular chromatin in a region of interest. Generally, cleavage by the reconstituted cleavage domain occurs at a site located between the two target sequences. One or both of the proteins can be engineered to bind to its target site.

The two fusion proteins can bind in the region of interest in the same or opposite polarity, and their binding sites (*i.e.*, target sites) can be separated by any number of nucleotides, *e.g.*, from 0 to 200 nucleotides or any integral value therebetween. In certain embodiments, the binding sites for two fusion proteins, each comprising a zinc finger binding domain and a cleavage half-domain, can be located between 5 and 18 nucleotides apart, for example, 5-8 nucleotides apart, or 15-18 nucleotides apart, or 6 nucleotides apart, or 16 nucleotides apart, as measured from the edge of each binding site nearest the other binding site, and cleavage occurs between the binding sites.

The site at which the DNA is cleaved generally lies between the binding sites for the two fusion proteins. Double-strand breakage of DNA often results from two single-strand breaks, or "nicks," offset by 1, 2, 3, 4, 5, 6 or more nucleotides, (for example, cleavage of double-stranded DNA by native *Fok* I results from single-strand breaks offset by 4 nucleotides). Thus, cleavage does not necessarily occur at exactly opposite sites on each DNA strand. In addition, the structure of the fusion proteins and the distance between the target sites can influence whether cleavage occurs adjacent a single nucleotide pair, or whether cleavage occurs at several sites. However, for many applications, including targeted recombination (see *infra*) cleavage within a range of nucleotides is generally sufficient, and cleavage between particular base pairs is not required.

As noted above, the fusion protein(s) can be introduced as polypeptides and/or polynucleotides. For example, two polynucleotides, each comprising sequences encoding one of the aforementioned polypeptides, can be introduced into a cell, and when the polypeptides are expressed and each binds to its target sequence, cleavage occurs at or near the target sequence. Alternatively, a single polynucleotide comprising sequences encoding both fusion polypeptides is introduced into a cell. Polynucleotides can be DNA, RNA or any modified forms or analogues or DNA and/or RNA.

To enhance cleavage specificity, additional compositions may also be employed in the methods described herein. For example, single cleavage half-domains can exhibit limited double-stranded cleavage activity. In methods in which two fusion proteins, each containing a three-finger zinc finger domain and a cleavage half-domain, are introduced into the cell, either protein specifies an approximately 9-nucleotide target site. Although the aggregate target sequence of 18 nucleotides is likely to be unique in a mammalian genome, any given 9-nucleotide target site occurs, on average, approximately 23,000 times in the human genome. Thus, non-specific cleavage, due to the site-specific binding of a single half-domain, may occur. Accordingly, the methods described herein contemplate the use of a dominant-negative mutant of a cleavage half-domain such as *Fok* I (or a nucleic acid encoding same) that is expressed in a cell along with the two fusion proteins. The dominant-negative mutant is capable of dimerizing but is unable to cleave, and also blocks the cleavage activity of a half-domain to which it is dimerized. By providing the dominant-negative mutant in molar excess to the fusion proteins, only regions in which both fusion proteins are bound will have a high enough local concentration of functional cleavage half-domains for dimerization and cleavage to occur. At sites where only one of the two fusion proteins are bound, its cleavage half-domain forms a dimer with the dominant negative mutant half-domain, and undesirable, non-specific cleavage does not occur.

Three catalytic amino acid residues in the *Fok* I cleavage half-domain have been identified: Asp 450, Asp 467 and Lys 469. Bitinaite et al. (1998) Proc. Natl. Acad. Sci. USA 95: 10,570-10,575. Thus, one or more mutations at one of these residues can be used to generate a dominant negative mutation. Further, many of the catalytic amino acid residues of other Type IIS endonucleases are known and/or can be determined, for example, by alignment with *Fok* I sequences and/or by generation and testing of mutants for catalytic activity.

**Dimerization domain mutations in the cleavage half-domain**

Methods for targeted cleavage which involve the use of fusions between a ZFP and a cleavage half-domain (such as, *e.g.,* a ZFP/*Fok*I fusion) require the use of two such fusion molecules, each generally directed to a distinct target sequence. Target sequences for the two fusion proteins can be chosen so that targeted cleavage is directed to a unique site in a genome, as discussed above. A potential source of reduced cleavage specificity could result from homodimerization of one of the two ZFP/cleavage half-domain fusions. This might occur, for example, due to the presence, in a genome, of inverted repeats of the target sequences for one of the two ZFP/cleavage half-domain fusions, located so as to allow two copies of the same fusion protein to bind with an orientation and spacing that allows formation of a functional dimer.

One approach for reducing the probability of this type of aberrant cleavage at sequences other than the intended target site involves generating variants of the cleavage half-domain that minimize or prevent homodimerization. Preferably, one or more amino acids in the region of the half-domain involved in its dimerization are altered. In the crystal structure of the *FokI* protein dimer, the structure of the cleavage half-domains is reported to be similar to the arrangement of the cleavage half-domains during cleavage of DNA by *FokI.* Wah et al. (1998) Proc. Natl. Acad. Sci. USA 95:10564-10569. This structure indicates that amino acid residues at positions 483 and 487 play a key role in the dimerization of the *FokI* cleavage half-domains. The structure also indicates that amino acid residues at positions 446, 447, 479, 483, 484, 486, 487, 490, 491, 496, 498, 499, 500, 531, 534, 537, and 538 are all close enough to the dimerization interface to influence dimerization. Accordingly, amino acid sequence alterations at one or more of the aforementioned positions will likely alter the dimerization properties of the cleavage half-domain. Such changes can be introduced, for example, by constructing a library containing (or encoding) different amino acid residues at these positions and selecting variants with the desired properties, or by rationally designing individual mutants. In addition to preventing homodimerization, it is also possible that some of these mutations may increase the cleavage efficiency above that obtained with two wild-type cleavage half-domains.

Accordingly, alteration of a *FokI* cleavage half-domain at any amino acid residue which affects dimerization can be used to prevent one of a pair of ZFP/*Fok*I fusions from undergoing homodimerization which can lead to cleavage at undesired sequences. Thus, for targeted cleavage using a pair of ZFP/*Fok*I fusions, one or both of the fusion proteins can comprise one or more amino acid alterations that inhibit self-dimerization, but allow heterodimerization of the two fusion proteins to occur such that cleavage occurs at the desired target site. In certain embodiments, alterations are present in both fusion proteins, and the alterations have additive effects; *i.e.,* homodimerization of either fusion, leading to aberrant cleavage, is minimized or abolished, while heterodimerization of the two fusion proteins is facilitated compared to that obtained with wild-type cleavage half-domains. *See* Example 5.

**Methods for targeted alteration of genomic sequences and targeted recombination**

Also described herein are methods of replacing a genomic sequence (*e.g.,* a region of interest in cellular chromatin) with a homologous non-identical sequence (*i.e.,* targeted recombination). Previous attempts to replace particular sequences have involved contacting a cell with a polynucleotide comprising sequences bearing homology to a chromosomal region (*i.e.,* a donor DNA), followed by selection of cells in which the donor DNA molecule had undergone homologous recombination into the genome. The success rate of these methods is low, due to poor efficiency of homologous recombination and a high frequency of non-specific insertion of the donor DNA into regions of the genome other than the target site.

The present disclosure provides methods of targeted sequence alteration characterized by a greater efficiency of targeted recombination and a lower frequency of non-specific insertion events. The methods involve making and using engineered zinc finger binding domains fused to cleavage domains (or cleavage half-domains) to make one or more targeted double-stranded breaks in cellular DNA. Because double-stranded breaks in cellular DNA stimulate homologous recombination several thousand-fold in the vicinity of the cleavage site, such targeted cleavage allows for the alteration or replacement (*via* homologous recombination) of sequences at virtually any site in the genome.

In addition to the fusion molecules described herein, targeted replacement of a selected genomic sequence also requires the introduction of the replacement (or donor) sequence. The donor sequence can be introduced into the cell prior to, concurrently with, or subsequent to, expression of the fusion protein(s). The donor polynucleotide contains sufficient homology to a genomic sequence to support homologous recombination between it and the genomic sequence to which it bears homology. Approximately 25, 50 100 or 200 nucleotides or more of sequence homology between a donor and a genomic sequence (or any integral value between 10 and 200 nucleotides, or more) will support homologous recombination therebetween. Donor sequences can range in length from 10 to 5,000 nucleotides (or any integral value of nucleotides therebetween) or longer. It will be readily apparent that the donor sequence is typically not identical to the genomic sequence that it replaces. For example, the sequence of the donor polynucleotide can contain one or more single base changes, insertions, deletions, inversions or rearrangements with respect to the genomic sequence, so long as sufficient homology is present to support homologous recombination. Alternatively, a donor sequence can contain a non-homologous sequence flanked by two regions of homology. Additionally, donor sequences can comprise a vector molecule containing sequences that are not homologous to the region of interest in cellular chromatin. Generally, the homologous region(s) of a donor sequence will have at least 50% sequence identity to a genomic sequence with which recombination is desired. In certain embodiments, 60%, 70%, 80%, 90%, 95%, 98%, 99%, or 99.9% sequence identity is present. Any value between 1% and 100% sequence identity can be present, depending upon the length of the donor polynucleotide.

A donor molecule can contain several, discontinuous regions of homology to cellular chromatin. For example, for targeted insertion of sequences not normally present in a region of interest, said sequences can be present in a donor nucleic acid molecule and flanked by regions of homology to sequence in the region of interest.

To simplify assays (*e.g.*, hybridization, PCR, restriction enzyme digestion) for determining successful insertion of the donor sequence, certain sequence differences may be present in the donor sequence as compared to the genomic sequence. Preferably, if located in a coding region, such nucleotide sequence differences will not change the amino acid sequence, or will make silent amino acid changes (*i.e.,* changes which do not affect the structure or function of the protein). The donor polynucleotide can optionally contain changes in sequences corresponding to the zinc finger domain binding sites in the region of interest, to prevent cleavage of donor sequences that have been introduced into cellular chromatin by homologous recombination.

The donor polynucleotide can be DNA or RNA, single-stranded or double-stranded and can be introduced into a cell in linear or circular form. If introduced in linear form, the ends of the donor sequence can be protected (*e.g.*, from exonucleolytic degradation) by methods known to those of skill in the art. For example, one or more dideoxynucleotide residues are added to the 3' terminus of a linear molecule and/or self-complementary oligonucleotides are ligated to one or both ends. *See,* for example, Chang et al. (1987) Proc. Natl. Acad. Sci. USA 84:4959-4963; Nehls et al. (1996) Science 272:886-889. Additional methods for protecting exogenous polynucleotides from degradation include, but are not limited to, addition of terminal amino group(s) and the use of modified internucleotide linkages such as, for example, phosphorothioates, phosphoramidates, and O-methyl ribose or deoxyribose residues. A polynucleotide can be introduced into a cell as part of a vector molecule having additional sequences such as, for example, replication origins, promoters and genes encoding antibiotic resistance. Moreover, donor polynucleotides can be introduced as naked nucleic acid, as nucleic acid complexed with an agent such as a liposome or poloxamer, or can be delivered by viruses (*e.g.*, adenovirus, AAV).

Without being bound by one theory, it appears that the presence of a double-stranded break in a cellular sequence, coupled with the presence of an exogenous DNA molecule having homology to a region adjacent to or surrounding the break, activates cellular mechanisms which repair the break by transfer of sequence information from the donor molecule into the cellular (*e.g.,* genomic or chromosomal) sequence; *i.e.,* by a processes of homologous recombination. Applicants' methods advantageously combine the powerful targeting capabilities of engineered ZFPs with a cleavage domain (or cleavage half-domain) to specifically target a double-stranded break to the region of the genome at which recombination is desired.

For alteration of a chromosomal sequence, it is not necessary for the entire sequence of the donor to be copied into the chromosome, as long as enough of the donor sequence is copied to effect the desired sequence alteration.

The efficiency of insertion of donor sequences by homologous recombination is inversely related to the distance, in the cellular DNA, between the double-stranded break and the site at which recombination is desired. In other words, higher homologous recombination efficiencies are observed when the double-stranded break is closer to the site at which recombination is desired. In cases in which a precise site of recombination is not predetermined (*e.g.*, the desired recombination event can occur over an interval of genomic sequence), the length and sequence of the donor nucleic acid, together with the site(s) of cleavage, are selected to obtain the desired recombination event. In cases in which the desired event is designed to change the sequence of a single nucleotide pair in a genomic sequence, cellular chromatin is cleaved within 10,000 nucleotides on either side of that nucleotide pair. In certain embodiments, cleavage occurs within 500, 200, 100, 90, 80, 70, 60, 50, 40, 30, 20, 10, 5, or 2 nucleotides, or any integral value between 2 and 1,000 nucleotides, on either side of the nucleotide pair whose sequence is to be changed.

As detailed above, the binding sites for two fusion proteins, each comprising a zinc finger binding domain and a cleavage half-domain, can be located 5-8 or 15-18 nucleotides apart, as measured from the edge of each binding site nearest the other binding site, and cleavage occurs between the binding sites. Whether cleavage occurs at a single site or at multiple sites between the binding sites is immaterial, since the cleaved genomic sequences are replaced by the donor sequences. Thus, for efficient alteration of the sequence of a single nucleotide pair by targeted recombination, the midpoint of the region between the binding sites is within 10,000 nucleotides of that nucleotide pair, preferably within 1,000 nucleotides, or 500 nucleotides, or 200 nucleotides, or 100 nucleotides, or 50 nucleotides, or 20 nucleotides, or 10 nucleotides, or 5 nucleotide, or 2 nucleotides, or one nucleotide, or at the nucleotide pair of interest.

A homologous chromosome can serve as the donor polynucleotide. Thus, for example, correction of a mutation in a heterozygote can be achieved by engineering fusion proteins which bind to and cleave the mutant sequence on one chromosome, but do not cleave the wild-type sequence on the homologous chromosome. The double-stranded break on the mutation-bearing chromosome stimulates a homology-based "gene conversion" process in which the wild-type sequence from the homologous chromosome is copied into the cleaved chromosome, thus restoring two copies of the wild-type sequence.

Methods and compositions are also disclosed that may enhance levels of targeted recombination including, but not limited to, the use of additional ZFP-functional domain fusions to activate expression of genes involved in homologous recombination, such as, for example, members of the RAD52 epistasis group (*e.g., Rad50, Rad51, Rad51B, Rad51C, Rad51D, Rad52, Rad54, Rad54B, Mrell, XRCC2, XRCC3),* genes whose products interact with the aforementioned gene products (*e.g.*, BRCA1, BRCA2) and/or genes in the NBS1 complex. Similarly ZFP-functional domain fusions can be used, in combination with the methods and compositions disclosed herein, to repress expression of genes involved in non-homologous end joining (*e.g.*, Ku70/80, XRCC4, poly(ADP ribose) polymerase, DNA ligase 4). *See,* for example, Yanez et al. (1998) Gene Therapy 5:149-159; Hoeijmakers (2001) Nature 411:366-374; Johnson et al. (2001) Biochem. Soc. Trans. 29:196-201; Tauchi et al. (2002) Oncogene 21:8967-8980. Methods for activation and repression of gene expression using fusions between a zinc finger binding domain and a functional domain are disclosed in co-owned US Patent No. 6,534,261. Additional repression methods include the use of antisense oligonucleotides and/or small interfering RNA (siRNA or RNAi) targeted to the sequence of the gene to be repressed.

As an alternative to or, in addition to, activating expression of gene products involved in homologous recombination, fusions of these protein (or functional fragments thereof) with a zinc finger binding domain targeted to the region of interest, can be used to recruit these proteins (recombination proteins) to the region of interest, thereby increasing their local concentration and further stimulating homologous recombination processes. Alternatively, a polypeptide involved in homologous recombination as described above (or a functional fragment thereof) can be part of a triple fusion protein comprising a zinc finger binding domain, a cleavage domain (or cleavage half-domain) and the recombination protein (or functional fragment thereof). Additional proteins involved in gene conversion and recombination-related chromatin remodeling, which can be used in the aforementioned methods and compositions, include histone acetyltransferases (*e.g.,* Esalp, Tip60), histone methyltransferases (*e.g.,* Dotlp), histone kinases and histone phosphatases.

The p53 protein has been reported to play a central role in repressing homologous recombination (HR). *See,* for example, Valerie et al., (2003) Oncogene 22:5792-5812; Janz, et al. (2002) Oncogene 21:5929-5933. For example, the rate of HR in p53-deficient human tumor lines is 10,000-fold greater than in primary human fibroblasts, and there is a 100-fold increase in HR in tumor cells with a non-functional p53 compared to those with functional p53. Mekeel et al. (1997) Oncogene 14:1847-1857. In addition, overexpression of p53 dominant negative mutants leads to a 20-fold increase in spontaneous recombination. Bertrand et al. (1997) Oncogene 14:1117-1122. Analysis of different p53 mutations has revealed that the roles of p53 in transcriptional transactivation and G1 cell cycle checkpoint control are separable from its involvement in HR. Saintigny et al. (1999) Oncogene 18:3553-3563; Boehden et al. (2003) Oncogene 22:4111-4117. Accordingly, downregulation of p53 activity can serve to increase the efficiency of targeted homologous recombination using the methods and compositions disclosed herein. Any method for downregulation of p53 activity can be used, including but not limited to cotransfection and overexpression of a p53 dominant negative mutant or targeted repression of p53 gene expression according to methods disclosed, *e.g.,* in co-owned U.S. Patent No. 6,534,261.

Further increases in efficiency of targeted recombination, in cells comprising a zinc finger/nuclease fusion molecule and a donor DNA molecule, are achieved by blocking the cells in the $G_2$ phase of the cell cycle, when homology-driven repair processes are maximally active. Such arrest can be achieved in a number of ways. For example, cells can be treated with *e.g.*, drugs, compounds and/or small molecules which influence cell-cycle progression so as to arrest cells in $G_2$ phase. Exemplary molecules of this type include, but are not limited to, compounds which affect microtubule polymerization (*e.g.*, vinblastine, nocodazole, Taxol), compounds that interact with DNA (*e.g., cis*-platinum(II) diamine dichloride, Cisplatin, doxorubicin) and/or compounds that affect DNA synthesis (*e.g.*, thymidine, hydroxyurea, L-mimosine, etoposide, 5-fluorouracil). Additional increases in recombination efficiency are achieved by the use of histone deacetylase (HDAC) inhibitors (*e.g.*, sodium butyrate, trichostatin A) which alter chromatin structure to make genomic DNA more accessible to the cellular recombination machinery.

Additional methods for cell-cycle arrest include overexpression of proteins which inhibit the activity of the CDK cell-cycle kinases, for example, by introducing a cDNA encoding the protein into the cell or by introducing into the cell an engineered ZFP which activates expression of the gene encoding the protein. Cell-cycle arrest is also achieved by inhibiting the activity of cyclins and CDKs, for example, using RNAi methods (*e.g.*, U.S. Patent No. 6,506,559) or by introducing into the cell an engineered ZFP which represses expression of one or more genes involved in cell-cycle progression such as, for example, cyclin and/or CDK genes. See, *e.g.,* co- owned U.S. Patent No. 6,534,261 for methods for the synthesis of engineered zinc finger proteins for regulation of gene expression.

Alternatively, in certain cases, targeted cleavage is conducted in the absence of a donor polynucleotide (preferably in S or $G_2$ phase), and recombination occurs between homologous chromosomes.

**Methods to screen for cellular factors that facilitate homologous recombination**

Since homologous recombination is a multi-step process requiring the modification of DNA ends and the recruitment of several cellular factors into a protein complex, the addition of one or more exogenous factors, along with donor DNA and vectors encoding zinc finger-cleavage domain fusions, can be used to facilitate targeted homologous recombination. An exemplary method for identifying such a factor or factors employs analyses of gene expression using microarrays (*e.g.*, Affymetrix Gene Chip® arrays) to compare the mRNA expression patterns of different cells. For example, cells that exhibit a higher capacity to stimulate double strand break-driven homologous recombination in the presence of donor DNA and zinc finger-cleavage domain fusions, either unaided or under conditions known to increase the level of gene correction, can be analyzed for their gene expression patterns compared to cells that lack such capacity. Genes that are upregulated or downregulated in a manner that directly correlates with increased levels of homologous recombination are thereby identified and can be cloned into any one of a number of expression vectors. These expression constructs can be co-transfected along with zinc finger-cleavage domain fusions and donor constructs to yield improved methods for achieving high-efficiency homologous recombination. Alternatively, expression of such genes can be appropriately regulated using engineered zinc finger roteins which modulate expression (either activation or repression) of one or more these genes. See, *e.g.,* co- owned U.S. Patent No. 6,534,261 for methods for the synthesis of engineered zinc finger proteins for regulation of gene expression.

As an example, it was observed that the different clones obtained in the experiments described in Example 9 and Figure 27 exhibited a wide-range of homologous recombination frequencies, when transfected with donor DNA and plasmids encoding zinc finger-cleavage domain fusions. Gene expression in clones showing a high frequency of targeted recombination can thus be compared to that in clones exhibiting a low frequency, and expression patterns unique to the former clones can be identified.

As an additional example, studies using cell cycle inhibitors (*e.g.*, nocodazole or vinblastine, see *e.g.*, Examples 11, 14 and 15) showed that cells arrested in the G2 phase of the cell cycle carried out homologous recombination at higher rates, indicating that cellular factors responsible for homologous recombination may be preferentially expressed or active in G2. One way to identify these factors is to compare the mRNA expression patterns between the stably transfected HEK 293 cell clones that carry out gene correction at high and low levels (*e.g.,* clone T18 *vs.* clone T7). Similar comparisons are made between these cell lines in response to compounds that arrest the cells in G2 phase. Candidate genes that are differentially expressed in cells that carry out homologous recombination at a higher rate, either unaided or in response to compounds that arrest the cells in G2, are identified, cloned, and re-introduced into cells to determine whether their expression is sufficient to re-capitulate the improved rates. Alternatively, expression of said candidate genes is activated using engineered zinc finger transcription factors as described, for example, in co-owned U.S. Patent No. 6,534,261.

**Expression vectors**

A nucleic acid encoding one or more ZFPs or ZFP fusion proteins can be cloned into a vector for transformation into prokaryotic or eukaryotic cells for replication and/or expression. Vectors can be prokaryotic vectors, *e.g.*, plasmids, or shuttle vectors, insect vectors, or eukaryotic vectors. A nucleic acid encoding a ZFP can also be cloned into an expression vector, for administration to a plant cell, animal cell, preferably a mammalian cell or a human cell, fungal cell, bacterial cell, or protozoal cell.

To obtain expression of a cloned gene or nucleic acid, sequences encoding a ZFP or ZFP fusion protein are typically subcloned into an expression vector that contains a promoter to direct transcription. Suitable bacterial and eukaryotic promoters are well known in the art and described, *e.g.*, in Sambrook et al., Molecular Cloning, A Laboratory Manual (2nd ed. 1989; 3rd ed., 2001); Kriegler, Gene Transfer and Expression: A Laboratory Manual (1990); and Current Protocols in Molecular Biology (Ausubel *et al., supra.* Bacterial expression systems for expressing the ZFP are available in, e.g., *E. coli, Bacillus sp., and Salmonella* (Palva et al., Gene 22:229-235 (1983)). Kits for such expression systems are commercially available. Eukaryotic expression systems for mammalian cells, yeast, and insect cells are well known by those of skill in the art and are also commercially available.

The promoter used to direct expression of a ZFP-encoding nucleic acid depends on the particular application. For example, a strong constitutive promoter is typically used for expression and purification of ZFP. In contrast, when a ZFP is administered *in vivo* for gene regulation, either a constitutive or an inducible promoter is used, depending on the particular use of the ZFP. In addition, a preferred promoter for administration of a ZFP can be a weak promoter, such as HSV TK or a promoter having similar activity. The promoter typically can also include elements that are responsive to transactivation, *e.g.*, hypoxia response elements, Gal4 response elements, lac repressor response element, and small molecule control systems such as tet-regulated systems and the RU-486 system (*see, e.g.,* Gossen & Bujard, PNAS 89:5547 (1992); Oligino et al., Gene Ther. 5:491-496 (1998); Wang et al., Gene Ther. 4:432-441 (1997); Neering et al., Blood 88:1147-1155 (1996); and Rendahl et al., Nat. Biotechnol. 16:757-761 (1998)). The MNDU3 promoter can also be used, and is preferentially active in CD34$^+$ hematopoietic stem cells.

In addition to the promoter, the expression vector typically contains a transcription unit or expression cassette that contains all the additional elements required for the expression of the nucleic acid in host cells, either prokaryotic or eukaryotic. A typical expression cassette thus contains a promoter operably linked, *e.g.,* to a nucleic acid sequence encoding the ZFP, and signals required, *e.g.*, for efficient polyadenylation of the transcript, transcriptional termination, ribosome binding sites, or translation termination. Additional elements of the cassette may include, *e.g.*, enhancers, and heterologous splicing signals.

The particular expression vector used to transport the genetic information into the cell is selected with regard to the intended use of the ZFP, *e.g.*, expression in plants, animals, bacteria, fungus, protozoa, *etc.* (see expression vectors described below). Standard bacterial expression vectors include plasmids such as pBR322-based plasmids, pSKF, pET23D, and commercially available fusion expression systems such as GST and LacZ. An exemplary fusion protein is the maltose binding protein, "MBP." Such fusion proteins are used for purification of the ZFP. Epitope tags can also be added to recombinant proteins to provide convenient methods of isolation, for monitoring expression, and for monitoring cellular and subcellular localization, *e.g.*, c-myc or FLAG.

Expression vectors containing regulatory elements from eukaryotic viruses are often used in eukaryotic expression vectors, *e.g.*, SV40 vectors, papilloma virus vectors, and vectors derived from Epstein-Barr virus. Other exemplary eukaryotic vectors include pMSG, pAV009/A+, pMTO10/A+, pMAMneo-5, baculovirus pDSVE, and any other vector allowing expression of proteins under the direction of the SV40 early promoter, SV40 late promoter, metallothionein promoter, murine mammary tumor virus promoter, Rous sarcoma virus promoter, polyhedrin promoter, or other promoters shown effective for expression in eukaryotic cells.

Some expression systems have markers for selection of stably transfected cell lines such as thymidine kinase, hygromycin B phosphotransferase, and dihydrofolate reductase. High yield expression systems are also suitable, such as using a baculovirus vector in insect cells, with a ZFP encoding sequence under the direction of the polyhedrin promoter or other strong baculovirus promoters.

The elements that are typically included in expression vectors also include a replicon that functions in *E. coli,* a gene encoding antibiotic resistance to permit selection of bacteria that harbor recombinant plasmids, and unique restriction sites in nonessential regions of the plasmid to allow insertion of recombinant sequences.

Standard transfection methods are used to produce bacterial, mammalian, yeast or insect cell lines that express large quantities of protein, which are then purified using standard techniques (*see, e.g.,* Colley et al., J. Biol. Chem. 264:17619-17622 (1989); Guide to Protein Purification, in Methods in Enzymology, vol. 182 (Deutscher, ed., 1990)). Transformation of eukaryotic and prokaryotic cells are performed according to standard techniques (*see, e.g.,* Morrison, J. Bact. 132:349-351 (1977); Clark-Curtiss & Curtiss, Methods in Enzymology 101:347-362 (Wu et al., eds, 1983).

Any of the well known procedures for introducing foreign nucleotide sequences into host cells may be used. These include the use of calcium phosphate transfection, polybrene, protoplast fusion, electroporation, ultrasonic methods (e.g., sonoporation), liposomes, microinjection, naked DNA, plasmid vectors, viral vectors, both episomal and integrative, and any of the other well known methods for introducing cloned genomic DNA, cDNA, synthetic DNA or other foreign genetic material into a host cell (*see, e.g.,* Sambrook *et al., supra*). It is only necessary that the particular genetic engineering procedure used be capable of successfully introducing at least one gene into the host cell capable of expressing the protein of choice.

**Nucleic acids encoding fusion proteins and delivery to cells**

Conventional viral and non-viral based gene transfer methods can be used to introduce nucleic acids encoding engineered ZFPs in cells (*e.g.*, mammalian cells) and target tissues. Such methods can also be used to administer nucleic acids encoding ZFPs to cells *in vitro.* Nucleic acids encoding ZFPs can be administered for *in vivo* or *ex vivo* gene therapy uses. Non-viral vector delivery systems include DNA plasmids, naked nucleic acid, and nucleic acid complexed with a delivery vehicle such as a liposome or poloxamer. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Böhm (eds) (1995); and Yu et al., Gene Therapy 1:13-26 (1994).

Methods of non-viral delivery of nucleic acids encoding engineered ZFPs include electroporation, lipofection, microinjection, biolistics, virosomes, liposomes, immunoliposomes, polycation or lipid:nucleic acid conjugates, naked DNA, artificial virions, and agent-enhanced uptake of DNA. Sonoporation using, *e.g.,* the Sonitron 2000 system (Rich-Mar) can also be used for delivery of nucleic acids.

Additional exemplary nucleic acid delivery systems include those provided by Amaxa Biosystems (Cologne, Germany), Maxcyte, Inc. (Rockville, Maryland) and BTX Molecular Delivery Systems (Holliston, MA).

Lipofection is described in e.g., US 5,049,386, US 4,946,787; and US 4,897,355) and lipofection reagents are sold commercially (e.g., Transfectam™ and Lipofectin™). Cationic and neutral lipids that are suitable for efficient receptor-recognition lipofection of polynucleotides include those of Felgner, WO 91/17424, WO 91/16024. Delivery can be to cells (*ex vivo* administration) or target tissues (*in vivo* administration).

The preparation of lipid:nucleic acid complexes, including targeted liposomes such as immunolipid complexes, is well known to one of skill in the art (*see, e.g.,* Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids encoding engineered ZFPs take advantage of highly evolved processes for targeting a virus to specific cells in the body and trafficking the viral payload to the nucleus. Viral vectors can be administered directly to patients (*in vivo*) or they can be used to treat cells *in vitro* and the modified cells are administered to patients (*ex vivo*). Conventional viral based systems for the delivery of ZFPs include, but are not limited to, retroviral, lentivirus, adenoviral, adeno-associated, vaccinia and herpes simplex virus vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system depends on the target tissue. Retroviral vectors are comprised of *cis*-acting long terminal repeats with packaging capacity for up to 6-10 kb of foreign sequence. The minimum *cis*-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immunodeficiency virus (SIV), human immunodeficiency virus (HIV), and combinations thereof (*see, e.g.,* Buchscher et al., J. Virol. 66:2731-2739 (1992); Johann et al., J. Virol. 66:1635-1640 (1992); Sommerfelt et al., Virol. 176:58-59 (1990); Wilson et al., J. Virol. 63:2374-2378 (1989); Miller et al., J. Virol. 65:2220-2224 (1991); PCT/US94/05700).

In applications in which transient expression of a ZFP fusion protein is preferred, adenoviral based systems can be used. Adenoviral based vectors are capable of very high transduction efficiency in many cell types and do not require cell division. With such vectors, high titer and high levels of expression have been obtained. This vector can be produced in large quantities in a relatively simple system. Adeno-associated virus ("AAV") vectors are also used to transduce cells with target nucleic acids, e.g., in the *in vitro* production of nucleic acids and peptides, and for *in vivo* and *ex vivo* gene therapy procedures (*see, e.g.,* West et al., Virology 160:38-47 (1987); U.S. Patent No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

At least six viral vector approaches are currently available for gene transfer in clinical trials, which utilize approaches that involve complementation of defective vectors by genes inserted into helper cell lines to generate the transducing agent.

pLASN and MFG-S are examples of retroviral vectors that have been used in clinical trials (Dunbar et al., Blood 85:3048-305 (1995); Kohn et al., Nat. Med. 1:1017-102 (1995); Malech et al., PNAS 94:22 12133-12138 (1997)).

PA317/pLASN was the first therapeutic vector used in a gene therapy trial. (Blaese et al., Science 270:475-480 (1995)). Transduction efficiencies of 50% or greater have been observed for MFG-S packaged vectors. (Ellem et al., Immunol Immunother. 44(1):10-20 (1997); Dranoff et al., Hum. Gene Ther. 1:111-2 (1997).

Recombinant adeno-associated virus vectors (rAAV) are a promising alternative gene delivery systems based on the defective and nonpathogenic parvovirus adeno-associated type 2 virus. All vectors are derived from a plasmid that retains only the AAV 145 bp inverted terminal repeats flanking the transgene expression cassette. Efficient gene transfer and stable transgene delivery due to integration into the genomes of the transduced cell are key features for this vector system. (Wagner et al., Lancet 351:9117 1702-3 (1998), Kearns et al., Gene Ther. 9:748-55 (1996)).

Replication-deficient recombinant adenoviral vectors (Ad) can be produced at high titer and readily infect a number of different cell types. Most adenovirus vectors are engineered such that a transgene replaces the Ad E1a, E1b, and/or E3 genes; subsequently the replication defective vector is propagated in human 293 cells that supply deleted gene function in *trans.* Ad vectors can transduce multiple types of tissues *in vivo,* including nondividing, differentiated cells such as those found in liver, kidney and muscle. Conventional Ad vectors have a large carrying capacity. An example of the use of an Ad vector in a clinical trial involved polynucleotide therapy for antitumor immunization with intramuscular injection (Sterman et al., Hum. Gene Ther. 7:1083-9 (1998)). Additional examples of the use of adenovirus vectors for gene transfer in clinical trials include Rosenecker et al., Infection 24:1 5-10 (1996); Sterman et al., Hum. Gene Ther. 9:7 1083-1089 (1998); Welsh et al., Hum. Gene Ther. 2:205-18 (1995); Alvarez et al., Hum. Gene Ther. 5:597-613 (1997); Topf et al., Gene Ther. 5:507-513 (1998); Sterman et al., Hum. Gene Ther. 7:1083-1089 (1998).

Packaging cells are used to form virus particles that are capable of infecting a host cell. Such cells include 293 cells, which package adenovirus, and ψ2 cells or PA317 cells, which package retrovirus. Viral vectors used in gene therapy are usually generated by a producer cell line that packages a nucleic acid vector into a viral particle. The vectors typically contain the minimal viral sequences required for packaging and subsequent integration into a host (if applicable), other viral sequences being replaced by an expression cassette encoding the protein to be expressed. The missing viral functions are supplied in *trans* by the packaging cell line. For example, AAV vectors used in gene therapy typically only possess inverted terminal repeat (ITR) sequences from the AAV genome which are required for packaging and integration into the host genome. Viral DNA is packaged in a cell line, which contains a helper plasmid encoding the other AAV genes, namely *rep* and *cap,* but lacking ITR sequences. The cell line is also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV.

In many gene therapy applications, it is desirable that the gene therapy vector be delivered with a high degree of specificity to a particular tissue type. Accordingly, a viral vector can be modified to have specificity for a given cell type by expressing a ligand as a fusion protein with a viral coat protein on the outer surface of the virus. The ligand is chosen to have affinity for a receptor known to be present on the cell type of interest. For example, Han et al., Proc. Natl. Acad. Sci. USA 92:9747-9751 (1995), reported that Moloney murine leukemia virus can be modified to express human heregulin fused to gp70, and the recombinant virus infects certain human breast cancer cells expressing human epidermal growth factor receptor. This principle can be extended to other virus-target cell pairs, in which the target cell expresses a receptor and the virus expresses a fusion protein comprising a ligand for the cell-surface receptor. For example, filamentous phage can be engineered to display antibody fragments (e.g., FAB or Fv) having specific binding affinity for virtually any chosen cellular receptor. Although the above description applies primarily to viral vectors, the same principles can be applied to nonviral vectors. Such vectors can be engineered to contain specific uptake sequences which favor uptake by specific target cells.

Gene therapy vectors can be delivered *in vivo* by administration to an individual patient, typically by systemic administration (e.g., intravenous, intraperitoneal, intramuscular, subdermal, or intracranial infusion) or topical application, as described below. Alternatively, vectors can be delivered to cells *ex vivo,* such as cells explanted from an individual patient (e.g., lymphocytes, bone marrow aspirates, tissue biopsy) or universal donor hematopoietic stem cells, followed by reimplantation of the cells into a patient, usually after selection for cells which have incorporated the vector.

*Ex vivo* cell transfection for diagnostics, research, or for gene therapy (e.g., via re-infusion of the transfected cells into the host organism) is well known to those of skill in the art. Cells can be isolated from the subject organism, transfected with a ZFP nucleic acid (gene or cDNA), and re-infused back into the subject organism (*e.g.*, patient). Various cell types suitable for *ex vivo* transfection are well known to those of skill in the art (*see, e.g.,* Freshney et al., Culture of Animal Cells, A Manual of Basic Technique (3rd ed. 1994)) and the references cited therein for a discussion of how to isolate and culture cells from patients).

Stem cells can be used in *ex vivo* procedures for cell transfection and gene therapy. The advantage to using stem cells is that they can be differentiated into other cell types *in vitro,* or can be introduced into a mammal (such as the donor of the cells) where they will engraft in the bone marrow. Methods for differentiating CD34+ cells *in vitro* into clinically important immune cell types using cytokines such a GM-CSF, IFN-γ and TNF-α are known (*see* Inaba et al., J. Exp. Med. 176:1693-1702 (1992)).

Stem cells are isolated for transduction and differentiation using known methods. For example, stem cells are isolated from bone marrow cells by panning the bone marrow cells with antibodies which bind unwanted cells, such as CD4+ and CD8+ (T cells), CD45+ (panB cells), GR-1 (granulocytes), and lad (differentiated antigen presenting cells) (*see* Inaba et al., J. Exp. Med. 176:1693-1702 (1992)).

Vectors (*e.g.*, retroviruses, adenoviruses, liposomes, etc.) containing therapeutic ZFP nucleic acids can also be administered directly to an organism for transduction of cells *in vivo.* Alternatively, naked DNA can be administered. Administration is by any of the routes normally used for introducing a molecule into ultimate contact with blood or tissue cells including, but not limited to, injection, infusion, topical application and electroporation. Suitable methods of administering such nucleic acids are available and well known to those of skill in the art, and, although more than one route can be used to administer a particular composition, a particular route can often provide a more immediate and more effective reaction than another route.

Methods for introduction of DNA into hematopoietic stem cells are disclosed, for example, in U.S. Patent No. 5,928,638.

Pharmaceutically acceptable carriers are determined in part by the particular composition being administered, as well as by the particular method used to administer the composition. Accordingly, there is a wide variety of suitable formulations of pharmaceutical compositions available, as described below (*see, e.g.,* Remington's Pharmaceutical Sciences, 17th ed., 1989).

DNA constructs may be introduced into the genome of a desired plant host by a variety of conventional techniques. For reviews of such techniques see, for example, Weissbach & Weissbach Methods for Plant Molecular Biology (1988, Academic Press, N.Y.) Section VIII, pp. 421-463; and Grierson & Corey, Plant Molecular Biology (1988, 2d Ed.), Blackie, London, Ch. 7-9. For example, the DNA construct may be introduced directly into the genomic DNA of the plant cell using techniques such as electroporation and microinjection of plant cell protoplasts, or the DNA constructs can be introduced directly to plant tissue using biolistic methods, such as DNA particle bombardment (see, e.g., Klein et al (1987) Nature 327:70-73). Alternatively, the DNA constructs may be combined with suitable T-DNA flanking regions and introduced into a conventional *Agrobacterium tumefaciens* host vector. *Agrobacterium tumefaciens*-mediated transformation techniques, including disarming and use of binary vectors, are well described in the scientific literature. See, for example Horsch et al (1984) Science 233:496-498, and Fraley et al (1983) Proc. Nat'l. Acad. Sci. USA 80:4803. The virulence functions of the *Agrobacterium tumefaciens* host will direct the insertion of the construct and adjacent marker into the plant cell DNA when the cell is infected by the bacteria using binary T DNA vector (Bevan (1984) Nuc. Acid Res. 12:8711-8721) or the co-cultivation procedure (Horsch et al (1985) Science 227:1229-1231). Generally, the *Agrobacterium* transformation system is used to engineer dicotyledonous plants (Bevan et al (1982) Ann. Rev. Genet 16:357-384; Rogers et al (1986) Methods Enzymol. 118:627-641). The *Agrobacterium* transformation system may also be used to transform, as well as transfer, DNA to monocotyledonous plants and plant cells. *See* Hernalsteen et al (1984) EMBO J 3:3039-3041; Hooykass-Van Slogteren et al (1984) Nature 311:763-764; Grimsley et al (1987) Nature 325:1677-179; Boulton et al (1989) Plant Mol. Biol. 12:31-40.; and Gould et al (1991) Plant Physiol. 95:426-434.

Alternative gene transfer and transformation methods include, but are not limited to, protoplast transformation through calcium-, polyethylene glycol (PEG)- or electroporation-mediated uptake of naked DNA (see Paszkowski et al. (1984) EMBO J 3:2717-2722, Potrykus et al. (1985) Molec. Gen. Genet. 199:169-177; Fromm et al. (1985) Proc. Nat. Acad. Sci. USA 82:5824-5828; and Shimamoto (1989) Nature 338:274-276) and electroporation of plant tissues (D'Halluin et al. (1992) Plant Cell 4:1495-1505). Additional methods for plant cell transformation include microinjection, silicon carbide mediated DNA uptake (Kaeppler et al. (1990) Plant Cell Reporter 9:415-418), and microprojectile bombardment (see Klein et al. (1988) Proc. Nat. Acad. Sci. USA 85:4305-4309; and Gordon-Kamm et al. (1990) Plant Cell 2:603-618).

The disclosed methods and compositions can be used to insert exogenous sequences into a predetermined location in a plant cell genome. This is useful inasmuch as expression of an introduced transgene into a plant genome depends critically on its integration site. Accordingly, genes encoding, *e.g.*, nutrients, antibiotics or therapeutic molecules can be inserted, by targeted recombination, into regions of a plant genome favorable to their expression.

Transformed plant cells which are produced by any of the above transformation techniques can be cultured to regenerate a whole plant which possesses the transformed genotype and thus the desired phenotype. Such regeneration techniques rely on manipulation of certain phytohormones in a tissue culture growth medium, typically relying on a biocide and/or herbicide marker which has been introduced together with the desired nucleotide sequences. Plant regeneration from cultured protoplasts is described in Evans, et al., "Protoplasts Isolation and Culture" in Handbook of Plant Cell Culture, pp. 124-176, Macmillian Publishing Company, New York, 1983; and Binding, Regeneration of Plants, Plant Protoplasts, pp. 21-73, CRC Press, Boca Raton, 1985. Regeneration can also be obtained from plant callus, explants, organs, pollens, embryos or parts thereof. Such regeneration techniques are described generally in Klee et al (1987) Ann. Rev. of Plant Phys. 38:467-486.

Nucleic acids introduced into a plant cell can be used to confer desired traits on essentially any plant. A wide variety of plants and plant cell systems may be engineered for the desired physiological and agronomic characteristics described herein using the nucleic acid constructs of the present disclosure and the various transformation methods mentioned above. In preferred embodiments, target plants and plant cells for engineering include, but are not limited to, those monocotyledonous and dicotyledonous plants, such as crops including grain crops (e.g., wheat, maize, rice, millet, barley), fruit crops (e.g., tomato, apple, pear, strawberry, orange), forage crops (e.g., alfalfa), root vegetable crops (e.g., carrot, potato, sugar beets, yam), leafy vegetable crops (e.g., lettuce, spinach); flowering plants (e.g., petunia, rose, chrysanthemum), conifers and pine trees (e.g., pine fir, spruce); plants used in phytoremediation (e.g., heavy metal accumulating plants); oil crops (e.g., sunflower, rape seed) and plants used for experimental purposes (e.g., *Arabidopsis*). Thus, the disclosed methods and compositions have use over a broad range of plants, including, but not limited to, species from the genera Asparagus, Avena, Brassica, Citrus, Citrullus, Capsicum, Cucurbita, Daucus, Glycine, Hordeum, Lactuca, Lycopersicon, Malus, Manihot, Nicotiana, Oryza, Persea, Pisum, Pyrus, Prunus, Raphanus, Secale, Solanum, Sorghum, Triticum, Vitis, Vigna, and Zea.

One of skill in the art will recognize that after the expression cassette is stably incorporated in transgenic plants and confirmed to be operable, it can be introduced into other plants by sexual crossing. Any of a number of standard breeding techniques can be used, depending upon the species to be crossed.

A transformed plant cell, callus, tissue or plant may be identified and isolated by selecting or screening the engineered plant material for traits encoded by the marker genes present on the transforming DNA. For instance, selection may be performed by growing the engineered plant material on media containing an inhibitory amount of the antibiotic or herbicide to which the transforming gene construct confers resistance. Further, transformed plants and plant cells may also be identified by screening for the activities of any visible marker genes (e.g., the β-glucuronidase, luciferase, B or C1 genes) that may be present on the recombinant nucleic acid constructs. Such selection and screening methodologies are well known to those skilled in the art.

Physical and biochemical methods also may be used to identify plant or plant cell transformants containing inserted gene constructs. These methods include but are not limited to: 1) Southern analysis or PCR amplification for detecting and determining the structure of the recombinant DNA insert; 2) Northern blot, S1 RNase protection, primer-extension or reverse transcriptase-PCR amplification for detecting and examining RNA transcripts of the gene constructs; 3) enzymatic assays for detecting enzyme or ribozyme activity, where such gene products are encoded by the gene construct; 4) protein gel electrophoresis, Western blot techniques, immunoprecipitation, or enzyme-linked immunoassays, where the gene construct products are proteins. Additional techniques, such as *in situ* hybridization, enzyme staining, and immunostaining, also may be used to detect the presence or expression of the recombinant construct in specific plant organs and tissues. The methods for doing all these assays are well known to those skilled in the art.

Effects of gene manipulation using the methods disclosed herein can be observed by, for example, northern blots of the RNA (*e.g.*, mRNA) isolated from the tissues of interest. Typically, if the amount of mRNA has increased, it can be assumed that the corresponding endogenous gene is being expressed at a greater rate than before. Other methods of measuring gene and/or CYP74B activity can be used. Different types of enzymatic assays can be used, depending on the substrate used and the method of detecting the increase or decrease of a reaction product or by-product. In addition, the levels of and/or CYP74B protein expressed can be measured immunochemically, i.e., ELISA, RIA, EIA and other antibody based assays well known to those of skill in the art, such as by electrophoretic detection assays (either with staining or western blotting). The transgene may be selectively expressed in some tissues of the plant or at some developmental stages, or the transgene may be expressed in substantially all plant tissues, substantially along its entire life cycle. However, any combinatorial expression mode is also applicable.

The present disclosure also encompasses seeds of the transgenic plants described above wherein the seed has the transgene or gene construct. The present disclosure further encompasses the progeny, clones, cell lines or cells of the transgenic plants described above wherein said progeny, clone, cell line or cell has the transgene or gene construct.

**Delivery vehicles**

An important factor in the administration of polypeptide compounds, such as ZFP fusion proteins, is ensuring that the polypeptide has the ability to traverse the plasma membrane of a cell, or the membrane of an intra-cellular compartment such as the nucleus. Cellular membranes are composed of lipid-protein bilayers that are freely permeable to small, nonionic lipophilic compounds and are inherently impermeable to polar compounds, macromolecules, and therapeutic or diagnostic agents. However, proteins and other compounds such as liposomes have been described, which have the ability to translocate polypeptides such as ZFPs across a cell membrane.

For example, "membrane translocation polypeptides" have amphiphilic or hydrophobic amino acid subsequences that have the ability to act as membrane-translocating carriers. In one embodiment, homeodomain proteins have the ability to translocate across cell membranes. The shortest internalizable peptide of a homeodomain protein, Antennapedia, was found to be the third helix of the protein, from amino acid position 43 to 58 (*see, e.g.,* Prochiantz, Current Opinion in Neurobiology 6:629-634 (1996)). Another subsequence, the h (hydrophobic) domain of signal peptides, was found to have similar cell membrane translocation characteristics (*see, e.g.,* Lin et al., J. Biol. Chem. 270:1 4255-14258 (1995)).

Examples of peptide sequences which can be linked to a protein, for facilitating uptake of the protein into cells, include, but are not limited to: an 11 amino acid peptide of the tat protein of HIV; a 20 residue peptide sequence which corresponds to amino acids 84-103 of the p16 protein (*see* Fahraeus et al., Current Biology 6:84 (1996)); the third helix of the 60-amino acid long homeodomain of Antennapedia (Derossi et al., J. Biol. Chem. 269:10444 (1994)); the h region of a signal peptide such as the Kaposi fibroblast growth factor (K-FGF) h region (Lin *et al., supra*); or the VP22 translocation domain from HSV (Elliot & O'Hare, Cell 88:223-233 (1997)). Other suitable chemical moieties that provide enhanced cellular uptake may also be chemically linked to ZFPs. Membrane translocation domains *(i.e.,* internalization domains) can also be selected from libraries of randomized peptide sequences. *See,* for example, Yeh et al. (2003) Molecular Therapy 7(5):S461, Abstract #1191.

Toxin molecules also have the ability to transport polypeptides across cell membranes. Often, such molecules (called "binary toxins") are composed of at least two parts: a translocation/binding domain or polypeptide and a separate toxin domain or polypeptide. Typically, the translocation domain or polypeptide binds to a cellular receptor, and then the toxin is transported into the cell. Several bacterial toxins, including *Clostridium perfringens* iota toxin, diphtheria toxin (DT), *Pseudomonas* exotoxin A (PE), pertussis toxin (PT), *Bacillus anthracis* toxin, and pertussis adenylate cyclase (CYA), have been used to deliver peptides to the cell cytosol as internal or amino-terminal fusions (Arora et al., J. Biol. Chem., 268:3334-3341 (1993); Perelle et al., Infect. Immun., 61:5147-5156 (1993); Stenmark et al., J. Cell Biol. 113:1025-1032 (1991); Donnelly et al., PNAS 90:3530-3534 (1993); Carbonetti et al., Abstr. Annu. Meet. Am. Soc. Microbiol. 95:295 (1995); Sebo et al., Infect. Immun. 63:3851-3857 (1995); Klimpel et al., PNAS U.S.A. 89:10277-10281 (1992); and Novak et al., J. Biol. Chem. 267:17186-17193 1992)).

Such peptide sequences can be used to translocate ZFPs across a cell membrane. ZFPs can be conveniently fused to or derivatized with such sequences. Typically, the translocation sequence is provided as part of a fusion protein. Optionally, a linker can be used to link the ZFP and the translocation sequence. Any suitable linker can be used, e.g., a peptide linker.

The ZFP can also be introduced into an animal cell, preferably a mammalian cell, via a liposomes and liposome derivatives such as immunoliposomes. The term "liposome" refers to vesicles comprised of one or more concentrically ordered lipid bilayers, which encapsulate an aqueous phase. The aqueous phase typically contains the compound to be delivered to the cell, i.e., a ZFP.

The liposome fuses with the plasma membrane, thereby releasing the drug into the cytosol. Alternatively, the liposome is phagocytosed or taken up by the cell in a transport vesicle. Once in the endosome or phagosome, the liposome either degrades or fuses with the membrane of the transport vesicle and releases its contents.

In current methods of drug delivery via liposomes, the liposome ultimately becomes permeable and releases the encapsulated compound (in this case, a ZFP) at the target tissue or cell. For systemic or tissue specific delivery, this can be accomplished, for example, in a passive manner wherein the liposome bilayer degrades over time through the action of various agents in the body. Alternatively, active drug release involves using an agent to induce a permeability change in the liposome vesicle. Liposome membranes can be constructed so that they become destabilized when the environment becomes acidic near the liposome membrane (*see, e.g.,* PNAS 84:7851 (1987); Biochemistry 28:908 (1989)). When liposomes are endocytosed by a target cell, for example, they become destabilized and release their contents. This destabilization is termed fusogenesis. Dioleoylphosphatidylethanolamine (DOPE) is the basis of many "fusogenic" systems.

Such liposomes typically comprise a ZFP and a lipid component, e.g., a neutral and/or cationic lipid, optionally including a receptor-recognition molecule such as an antibody that binds to a predetermined cell surface receptor or ligand (*e.g.,* an antigen). A variety of methods are available for preparing liposomes as described in, *e.g.,* Szoka et al., Ann. Rev. Biophys. Bioeng. 9:467 (1980), U.S. Pat. Nos. 4,186,183, 4,217,344, 4,235,871, 4,261,975, 4,485,054, 4,501,728, 4,774,085, 4,837,028, 4,235,871, 4,261,975, 4,485,054, 4,501,728, 4,774,085, 4,837,028, 4,946,787, PCT Publication No. WO 91 17424, Deamer & Bangham, Biochim. Biophys. Acta 443:629-634 (1976); Fraley, et al., PNAS 76:3348-3352 (1979); Hope et al., Biochim. Biophys. Acta 812:55-65 (1985); Mayer et al., Biochim. Biophys. Acta 858:161-168 (1986); Williams et al., PNAS 85:242-246 (1988); Liposomes (Ostro (ed.), 1983, Chapter 1); Hope et al., Chem. Phys. Lip. 40:89 (1986); Gregoriadis, Liposome Technology (1984) and Lasic, Liposomes: from Physics to Applications (1993)). Suitable methods include, for example, sonication, extrusion, high pressure/homogenization, micro fluidization, detergent dialysis, calcium-induced fusion of small liposome vesicles and ether-fusion methods, all of which are known to those of skill in the art.

In certain embodiments, it is desirable to target liposomes using targeting moieties that are specific to a particular cell type, tissue, and the like. Targeting of liposomes using a variety of targeting moieties (e.g., ligands, receptors, and monoclonal antibodies) has been described. *See, e.g.,* U.S. Patent Nos. 4,957,773 and 4,603,044.

Examples of targeting moieties include monoclonal antibodies specific to antigens associated with neoplasms, such as prostate cancer specific antigen and MAGE. Tumors can also be diagnosed by detecting gene products resulting from the activation or overexpression of oncogenes, such as ras or c-erbB2. In addition, many tumors express antigens normally expressed by fetal tissue, such as the alphafetoprotein (AFP) and carcinoembryonic antigen (CEA). Sites of viral infection can be diagnosed using various viral antigens such as hepatitis B core and surface antigens (HBVc, HBVs) hepatitis C antigens, Epstein-Barr virus antigens, human immunodeficiency type-1 virus (HIV1) and papilloma virus antigens. Inflammation can be detected using molecules specifically recognized by surface molecules which are expressed at sites of inflammation such as integrins (*e.g.,* VCAM-1), selectin receptors (*e.g.,* ELAM-1) and the like.

Standard methods for coupling targeting agents to liposomes can be used. These methods generally involve incorporation into liposomes of lipid components, *e.g.*, phosphatidylethanolamine, which can be activated for attachment of targeting agents, or derivatized lipophilic compounds, such as lipid derivatized bleomycin. Antibody targeted liposomes can be constructed using, for instance, liposomes which incorporate protein A (*see* Renneisen et al., J. Biol. Chem., 265:16337-16342 (1990) and Leonetti et al., PNAS 87:2448-2451 (1990).

**Dosages**

For therapeutic applications, the dose administered to a patient, or to a cell which will be introduced into a patient, in the context of the present disclosure, should be sufficient to effect a beneficial therapeutic response in the patient over time. In addition, particular dosage regimens can be useful for determining phenotypic changes in an experimental setting, *e.g.,* in functional genomics studies, and in cell or animal models. The dose will be determined by the efficacy and $K_d$ of the particular ZFP employed, the nuclear volume of the target cell, and the condition of the patient, as well as the body weight or surface area of the patient to be treated. The size of the dose also will be determined by the existence, nature, and extent of any adverse side-effects that accompany the administration of a particular compound or vector in a particular patient.

The maximum therapeutically effective dosage of ZFP for approximately 99% binding to target sites is calculated to be in the range of less than about $1.5 \times 10^5$ to $1.5 \times 10^6$ copies of the specific ZFP molecule per cell. The number of ZFPs per cell for this level of binding is calculated as follows, using the volume of a HeLa cell nucleus (approximately 1000 $\mu m^3$ or $10^{-12}$ L; Cell Biology, (Altman & Katz, eds. (1976)). As the HeLa nucleus is relatively large, this dosage number is recalculated as needed using the volume of the target cell nucleus. This calculation also does not take into account competition for ZFP binding by other sites. This calculation also assumes that essentially all of the ZFP is localized to the nucleus. A value of 100x $K_d$ is used to calculate approximately 99% binding of to the target site, and a value of 10x $K_d$ is used to calculate approximately 90% binding of to the target site. For this example, $K_d$ = 25 nM

$$ZFP + \text{target site} \leftrightarrow \text{complex}$$

i.e., DNA + protein $\leftrightarrow$ DNA:protein complex

$$K_d = \frac{[\text{DNA}]\,[\text{protein}]}{[\text{DNA:protein complex}]}$$

When 50% of ZFP is bound, $K_d$ = [protein]
So when [protein] = 25 nM and the nucleus volume is $10^{-12}$ L
[protein] = $(25 \times 10^{-9}$ moles/L) $(10^{-12}$ L/nucleus) $(6 \times 10^{23}$ molecules/mole)
= 15,000 molecules/nucleus for 50% binding
When 99% target is bound; 100x $K_d$ = [protein]
100x $K_d$ = [protein] = 2.5 $\mu$M
$(2.5 \times 10^{-6}$ moles/L) $(10^{-12}$ L/nucleus) $(6 \times 10^{23}$ molecules/mole)
= about 1,500,000 molecules per nucleus for 99% binding of target site.

The appropriate dose of an expression vector encoding a ZFP can also be calculated by taking into account the average rate of ZFP expression from the promoter and the average rate of ZFP degradation in the cell. In certain embodiments, a weak promoter such as a wild-type or mutant HSV TK promoter is used, as described above. The dose of ZFP in micrograms is calculated by taking into account the molecular weight of the particular ZFP being employed.

In determining the effective amount of the ZFP to be administered in the treatment or prophylaxis of disease, the physician evaluates circulating plasma levels of the ZFP or nucleic acid encoding the ZFP, potential ZFP toxicities, progression of the disease, and the production of anti-ZFP antibodies. Administration can be accomplished via single or divided doses.

**Pharmaceutical compositions and administration**

ZFPs and expression vectors encoding ZFPs can be administered directly to the patient for targeted cleavage and/or recombination, and for therapeutic or prophylactic applications, for example, cancer, ischemia, diabetic retinopathy, macular degeneration, rheumatoid arthritis, psoriasis, HIV infection, sickle cell anemia, Alzheimer's disease, muscular dystrophy, neurodegenerative diseases, vascular disease, cystic fibrosis, stroke, and the like. Examples of microorganisms that can be inhibited by ZFP gene therapy include pathogenic bacteria, e.g., chlamydia, rickettsial bacteria, mycobacteria, staphylococci, streptococci, pneumococci, meningococci and conococci, klebsiella, proteus, serratia, pseudomonas, legionella, diphtheria, salmonella, bacilli, cholera, tetanus, botulism, anthrax, plague, leptospirosis, and Lyme disease bacteria; infectious fungus, e.g., *Aspergillus, Candida* species; protozoa such as sporozoa (e.g., *Plasmodia*), rhizopods (e.g., *Entamoeba*) and flagellates (*Trypanosoma, Leishmania, Trichomonas, Giardia,* etc.);viral diseases, e.g., hepatitis (A, B, or C), herpes virus *(e.g.,* VZV, HSV-1, HSV-6, HSV-II, CMV, and EBV), HIV, Ebola, adenovirus, influenza virus, flaviviruses, echovirus, rhinovirus, coxsackie virus, coronavirus, respiratory syncytial virus, mumps virus, rotavirus, measles virus, rubella virus, parvovirus, vaccinia virus, HTLV virus, dengue virus, papillomavirus, poliovirus, rabies virus, and arboviral encephalitis virus, *etc.*

Administration of therapeutically effective amounts is by any of the routes normally used for introducing ZFP into ultimate contact with the tissue to be treated. The ZFPs are administered in any suitable manner, preferably with pharmaceutically acceptable carriers. Suitable methods of administering such modulators are available and well known to those of skill in the art, and, although more than one route can be used to administer a particular composition, a particular route can often provide a more immediate and more effective reaction than another route.

Pharmaceutically acceptable carriers are determined in part by the particular composition being administered, as well as by the particular method used to administer the composition. Accordingly, there is a wide variety of suitable formulations of pharmaceutical compositions that are available (*see, e.g.,* Remington's Pharmaceutical Sciences, 17th ed. 1985)).

The ZFPs, alone or in combination with other suitable components, can be made into aerosol formulations (i.e., they can be "nebulized") to be administered via inhalation. Aerosol formulations can be placed into pressurized acceptable propellants, such as dichlorodifluoromethane, propane, nitrogen, and the like.

Formulations suitable for parenteral administration, such as, for example, by intravenous, intramuscular, intradermal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The disclosed compositions can be administered, for example, by intravenous infusion, orally, topically, intraperitoneally, intravesically or intrathecally. The formulations of compounds can be presented in unit-dose or multi-dose sealed containers, such as ampules and vials. Injection solutions and suspensions can be prepared from sterile powders, granules, and tablets of the kind previously described.

**Applications**

The disclosed methods and compositions for targeted cleavage can be used to induce mutations in a genomic sequence, *e.g.*, by cleaving at two sites and deleting sequences in between, by cleavage at a single site followed by non-homologous end joining, and/or by cleaving at a site so as to remove one or two or a few nucleotides. Targeted cleavage can also be used to create gene knock-outs (*e.g.*, for functional genomics or target validation) and to facilitate targeted insertion of a sequence into a genome (*i.e.,* gene knock-in); *e.g.,* for purposes of cell engineering or protein overexpression. Insertion can be by means of replacements of chromosomal sequences through homologous recombination or by targeted integration, in which a new sequence (*i.e.,* a sequence not present in the region of interest), flanked by sequences homologous to the region of interest in the chromosome, is inserted at a predetermined target site.

The same methods can also be used to replace a wild-type sequence with a mutant sequence, or to convert one allele to a different allele.

Targeted cleavage of infecting or integrated viral genomes can be used to treat viral infections in a host. Additionally, targeted cleavage of genes encoding receptors for viruses can be used to block expression of such receptors, thereby preventing viral infection and/or viral spread in a host organism. Targeted mutagenesis of genes encoding viral receptors (*e.g.,* the CCR5 and CXCR4 receptors for HIV) can be used to render the receptors unable to bind to virus, thereby preventing new infection and blocking the spread of existing infections. Non-limiting examples of viruses or viral receptors that may be targeted include herpes simplex virus (HSV), such as HSV-1 and HSV-2, varicella zoster virus (VZV), Epstein-Barr virus (EBV) and cytomegalovirus (CMV), HHV6 and HHV7. The hepatitis family of viruses includes hepatitis A virus (HAV), hepatitis B virus (HBV), hepatitis C virus (HCV), the delta hepatitis virus (HDV), hepatitis E virus (HEV) and hepatitis G virus (HGV). Other viruses or their receptors may be targeted, including, but not limited to, Picornaviridae (*e.g.*, polioviruses, etc.); Caliciviridae; Togaviridae (*e.g.,* rubella virus, dengue virus, *etc.*); Flaviviridae;

Coronaviridae; Reoviridae; Birnaviridae; Rhabodoviridae (*e.g.*, rabies virus, etc.); Filoviridae; Paramyxoviridae (*e.g.*, mumps virus, measles virus, respiratory syncytial virus, *etc.*); Orthomyxoviridae (*e.g.,* influenza virus types A, B and C, *etc.*); Bunyaviridae; Arenaviridae; Retroviradae; lentiviruses (*e.g.*, HTLV-I; HTLV-II; HIV-1 (also known as HTLV-III, LAV, ARV, hTLR, *etc.*) HIV-II); simian immunodeficiency virus (SIV), human papillomavirus (HPV), influenza virus and the tick-borne encephalitis viruses. See, *e.g.* Virology, 3rd Edition (W. K. Joklik ed. 1988); Fundamental Virology, 2nd Edition (B. N. Fields and D. M. Knipe, eds. 1991), for a description of these and other viruses. Receptors for HIV, for example, include CCR-5 and CXCR-4.

In similar fashion, the genome of an infecting bacterium can be mutagenized by targeted DNA cleavage followed by non-homologous end joining, to block or ameliorate bacterial infections.

The disclosed methods for targeted recombination can be used to replace any genomic sequence with a homologous, non-identical sequence. For example, a mutant genomic sequence can be replaced by its wild-type counterpart, thereby providing methods for treatment of *e.g.,* genetic disease, inherited disorders, cancer, and autoimmune disease. In like fashion, one allele of a gene can be replaced by a different allele using the methods of targeted recombination disclosed herein.

Exemplary genetic diseases include, but are not limited to, achondroplasia, achromatopsia, acid maltase deficiency, adenosine deaminase deficiency (OMIM No.102700), adrenoleukodystrophy, aicardi syndrome, alpha-1 antitrypsin deficiency, alpha-thalassemia, androgen insensitivity syndrome, apert syndrome, arrhythmogenic right ventricular, dysplasia, ataxia telangictasia, barth syndrome, beta-thalassemia, blue rubber bleb nevus syndrome, canavan disease, chronic granulomatous diseases (CGD), cri du chat syndrome, cystic fibrosis, dercum's disease, ectodermal dysplasia, fanconi anemia, fibrodysplasia ossificans progressive, fragile X syndrome, galactosemis, Gaucher's disease, generalized gangliosidoses (*e.g.*, GM1), hemochromatosis, the hemoglobin C mutation in the 6$^{th}$ codon of beta-globin (HbC), hemophilia, Huntington's disease, Hurler Syndrome, hypophosphatasia, Klinefleter syndrome, Krabbes Disease, Langer-Giedion Syndrome, leukocyte adhesion deficiency (LAD, OMIM No. 116920), leukodystrophy, long QT syndrome, Marfan syndrome, Moebius syndrome, mucopolysaccharidosis (MPS), nail patella syndrome, nephrogenic diabetes insipdius, neurofibromatosis, Neimann-Pick disease, osteogenesis imperfecta, porphyria, Prader-Willi syndrome, progeria, Proteus syndrome, retinoblastoma, Rett syndrome, Rubinstein-Taybi syndrome, Sanfilippo syndrome, severe combined immunodeficiency (SCID), Shwachman syndrome, sickle cell disease (sickle cell anemia), Smith-Magenis syndrome, Stickler syndrome, Tay-Sachs disease, Thrombocytopenia Absent Radius (TAR) syndrome, Treacher Collins syndrome, trisomy, tuberous sclerosis, Turner's syndrome, urea cycle disorder, von Hippel-Landau disease, Waardenburg syndrome, Williams syndrome, Wilson's disease, Wiskott-Aldrich syndrome, X-linked lymphoproliferative syndrome (XLP, OMIM No. 308240).

Additional exemplary diseases that can be treated by targeted DNA cleavage and/or homologous recombination include acquired immunodeficiencies, lysosomal storage diseases (*e.g.*, Gaucher's disease, GM1, Fabry disease and Tay-Sachs disease), mucopolysaccahidosis (*e.g.* Hunter's disease, Hurler's disease), hemoglobinopathies (*e.g.,* sickle cell diseases, HbC, $\alpha$-thalassemia, $\beta$-thalassemia) and hemophilias.

In certain cases, alteration of a genomic sequence in a pluripotent cell (*e.g.,* a hematopoietic stem cell) is desired. Methods for mobilization, enrichment and culture of hematopoietic stem cells are known in the art. *See* for example, U.S. Patents 5,061,620; 5,681,559; 6,335,195; 6,645,489 and 6,667,064. Treated stem cells can be returned to a patient for treatment of various diseases including, but not limited to, SCID and sickle-cell anemia.

In many of these cases, a region of interest comprises a mutation, and the donor polynucleotide comprises the corresponding wild-type sequence. Similarly, a wild-type genomic sequence can be replaced by a mutant sequence, if such is desirable. For example, overexpression of an oncogene can be reversed either by mutating the gene or by replacing its control sequences with sequences that support a lower, non-pathologic level of expression. As another example, the wild-type allele of the ApoAI gene can be replaced by the ApoAI Milano allele, to treat atherosclerosis. Indeed, any pathology dependent upon a particular genomic sequence, in any fashion, can be corrected or alleviated using the methods and compositions disclosed herein.

Targeted cleavage and targeted recombination can also be used to alter non-coding sequences (*e.g.*, regulatory sequences such as promoters, enhancers, initiators, terminators, splice sites) to alter the levels of expression of a gene product. Such methods can be used, for example, for therapeutic purposes, functional genomics and/or target validation studies.

The compositions and methods described herein also allow for novel approaches and systems to address immune reactions of a host to allogeneic grafts. In particular, a major problem faced when allogeneic stem cells (or any type of allogeneic cell) are grafted into a host recipient is the high risk of rejection by the host's immune system, primarily mediated through recognition of the Major Histocompatibility Complex (MHC) on the surface of the engrafted cells. The MHC comprises the HLA class I protein(s) that function as heterodimers that are comprised of a common $\beta$ subunit and variable $\alpha$ subunits. It has been demonstrated that tissue grafts derived from stem cells that are devoid of HLA escape the host's immune response. *See, e.g.,* Coffman et al. J Immunol 151, 425-35. (1993); Markmann et al. Transplantation 54, 1085-9. (1992); Koller et al. Science 248, 1227-30. (1990). Using the compositions and methods described herein, genes encoding HLA proteins involved in graft rejection can be cleaved, mutagenized or altered by recombination, in either their coding or regulatory sequences, so that their expression is blocked or they express a non-functional product. For example, by inactivating the gene encoding the common β subunit gene (β2 microglobulin) using ZFP fusion proteins as described herein, HLA class I can be removed from the cells to rapidly and reliably generate HLA class I *null* stem cells from any donor, thereby reducing the need for closely matched donor/recipient MHC haplotypes during stem cell grafting.

Inactivation of any gene (*e.g.,* the β2 microglobulin gene) can be achieved, for example, by a single cleavage event, by cleavage followed by non-homologous end joining, by cleavage at two sites followed by joining so as to delete the sequence between the two cleavage sites, by targeted recombination of a missense or nonsense codon into the coding region, or by targeted recombination of an irrelevant sequence *(i.e.,* a "stuffer" sequence) into the gene or its regulatory region, so as to disrupt the gene or regulatory region.

Targeted modification of chromatin structure, as disclosed in co-owned WO 01/83793, can be used to facilitate the binding of fusion proteins to cellular chromatin.

In additional embodiments, one or more fusions between a zinc finger binding domain and a recombinase (or functional fragment thereof) can be used, in addition to or instead of the zinc finger-cleavage domain fusions disclosed herein, to facilitate targeted recombination. See, for example, co-owned US patent No. 6,534,261 and Akopian et al. (2003) Proc. Natl. Acad. Sci. USA 100:8688-8691.

In additional embodiments of the disclosure, the disclosed methods and compositions are used to provide fusions of ZFP binding domains with transcriptional activation or repression domains that require dimerization (either homodimerization or heterodimerization) for their activity. In these cases, a fusion polypeptide comprises a zinc finger binding domain and a functional domain monomer (*e.g.*, a monomer from a dimeric transcriptional activation or repression domain). Binding of two such fusion polypeptides to properly situated target sites allows dimerization so as to reconstitute a functional transcription activation or repression domain.

## EXAMPLES

### Example 1: Editing of a Chromosomal hSMC1L1 Gene by Targeted Recombination

The hSMC1L1 gene is the human orthologue of the budding yeast gene *structural maintenance of chromosomes 1.* A region of this gene encoding an amino-terminal portion of the protein which includes the Walker ATPase domain was mutagenized by targeted cleavage and recombination. Cleavage was targeted to the region of the methionine initiation codon (nucleotides 24-26, Figure 1), by designing chimeric nucleases, comprising a zinc finger DNA-binding domain and a *Fok*I cleavage half-domain, which bind in the vicinity of the codon. Thus, two zinc finger binding domains were designed, one of which recognizes nucleotides 23-34 (primary contacts along the top strand as shown in Figure 1), and the other of which recognizes nucleotides 5-16 (primary contacts along the bottom strand). Zinc finger proteins were designed as described in co-owned US Patents 6,453,242 and 6,534,261. *See* Table 2 for the amino acid sequences of the recognition regions of the zinc finger proteins.

Sequences encoding each of these two ZFP binding domains were fused to sequences encoding a *Fok*I cleavage half-domain (amino acids 384-579 of the native *Fok*I sequence; Kita et al. (1989) J. Biol. Chem. 264:5751-5756), such that the encoded protein contained *Fok*I sequences at the carboxy terminus and ZFP sequences at the amino terminus. Each of these fusion sequences was then cloned in a modified mammalian expression vector pcDNA3 (Figure 2).

### Table 2: Zinc Finger Designs for the hSMC1L1 Gene

| Target sequence | F1 | F2 | F3 | F4 |
|---|---|---|---|---|
| CATGGGGTTCCT (SEQ ID NO: 27) | RSHDLIE (SEQ ID NO: 28) | TSSSLSR (SEQ ID NO: 29) | RSDHLST (SEQ ID NO: 30) | TNSNRIT (SEQ ID NO: 31) |
| GCGGCGCCGGCG (SEQ ID NO: 32) | RSDDLSR (SEQ ID NO: 33) | RSDDRKT (SEQ ID NO: 34) | RSEDLIR (SEQ ID NO: 35) | RSDTLSR (SEQ ID NO: 36) |
| Note: The zinc finger amino acid sequences shown above (in one-letter code) represent residues -1 through +6, with respect to the start of the alpha-helical portion of each zinc finger. Finger F1 is closest to the amino terminus of the protein, and Finger F4 is closest to the carboxy terminus. | | | | |

A donor DNA molecule was obtained as follows. First, a 700 base pair fragment of human genomic DNA representing nucleotides 52415936-52416635 of the "-" strand of the X chromosome (UCSC human genome release July, 2003), which includes the first exon of the human hSMC1L1 gene, was amplified, using genomic DNA from HEK293 cells as template. Sequences of primers used for amplification are shown in Table 3 ("Initial amp 1" and "Initial amp 2"). The PCR product was then altered, using standard overlap extension PCR methodology (see, *e.g.,* Ho, et al. (1989) Gene 77:51-59), resulting in replacement of the sequence ATGGGG (nucleotides 24-29 in Figure 1) to ATAAGAAGC. This change resulted in conversion of the ATG codon (methionine) to an ATA codon (isoleucine) and replacement of GGG (nucleotides 27-29 in Figure 1) by the sequence AGAAGC, allowing discrimination between donor-derived sequences and endogenous chromosomal sequences following recombination. A schematic diagram of the hSMC1 gene, including sequences of the chromosomal DNA in the region of the initiation codon, and sequences in the donor DNA that differ from the chromosomal sequence, is given in Figure 3. The resulting 700 base pair donor fragment was cloned into pCR4BluntTopo, which does not contain any sequences homologous to the human genome. *See* Figure 4.

For targeted mutation of the chromosomal hSMC1L1 gene, the two plasmids encoding ZFP-*Fok*I fusions and the donor plasmid were introduced into $1 \times 10^6$ HEK293 cells by transfection using Lipofectamine 2000® (Invitrogen). Controls included cells transfected only with the two plasmids encoding the ZFP-*Fok*I fusions, cells transfected only with the donor plasmid and cells transfected with a control plasmid (pEGFP-N1, Clontech). Cells were cultured in 5% $CO_2$ at 37°C. At 48 hours after transfection, genomic DNA was isolated from the cells, and 200 ng was used as template for PCR amplification, using one primer complementary to a region of the gene outside of its region of homology with the donor sequences (nucleotides 52416677-52416701 on the "-" STRAND of the X chromosome; UCSC July 2003), and a second primer complementary to a region of the donor molecule into which distinguishing mutations were introduced. Using these two primers, an amplification product of 400 base pairs will be obtained from genomic DNA if a targeted recombination event has occurred. The sequences of these primers are given in Table 3 (labeled "chromosome-specific" and "donor-specific," respectively). Conditions for amplification were: 94°C, 2 min, followed by 40 cycles of 94°C, 30 sec, 60°C, 1 min, 72°C, 1 min; and a final step of 72°C, 7min.

The results of this analysis (Figure 5) indicate that a 400 base pair amplification product (labeled "Chimeric DNA" in the Figure) was obtained only with DNA extracted from cells which had been transfected with the donor plasmid and both ZFP-*Fok*I plasmids.

**Table 3: Amplification Primers for the hSMC1L1 Gene**

| Initial amp 1 | AGCAACAACTCCTCCGGGGATC (SEQ ID NO: 37) |
|---|---|
| Initial amp 2 | TTCCAGACGCGACTCTTTGGC (SEQ ID NO: 38) |
| Chromosome-specific | CTCAGCAAGCGTGAGCTCAGGTCTC (SEQ ID NO: 39) |
| Donor-specific | CAATCAGTTTCAGGAAGCTTCTT (SEQ ID NO: 40) |
| Outside 1 | CTCAGCAAGCGTGAGCTCAGGTCTC (SEQ ID NO: 41) |
| Outside 2 | GGGGTCAAGTAAGGCTGGGAAGC (SEQ ID NO: 42) |

To confirm this result, two additional experiments were conducted. First, the amplification product was cloned into pCR4Blunt-Topo (Invitrogen) and its nucleotide sequence was determined. As shown in Figure 6 (SEQ ID NO: 6), the amplified sequence obtained from chromosomal DNA of cells transfected with the two ZFP-*Fok*I-encoding plasmids and the donor plasmid contains the AAGAAGC sequence that is unique to the donor (nucleotides 395-401 of the sequence presented in Figure 6) covalently linked to chromosomal sequences not present in the donor molecule (nucleotides 32-97 of Figure 6), indicating that donor sequences have been recombined into the chromosome. In particular, the G→A mutation converting the initiation codon to an isoleucine codon is observed at position 395 in the sequence.

In a second experiment, chromosomal DNA from cells transfected only with donor plasmid, cells transfected with both ZFP-*Fok*I fusion plasmids, cells transfected with the donor plasmid and both ZFP-*Fok*I fusion plasmids or cells transfected with the EGFP control plasmid was used as template for amplification, using primers complementary to sequences outside of the 700-nucleotide region of homology between donor and chromosomal sequences (identified as "Outside 1" and "Outside 2" in Table 3). The resulting amplification product was purified and used as template for a second amplification reaction using the donor-specific and chromosome-specific primers described above (Table 3). This amplification yielded a 400 nucleotide product only from cells transfected with the donor construct and both ZFP-*Fok*I fusion constructs, a result consistent with the replacement of genomic sequences by targeted recombination in these cells.

**Example 2: Editing of a Chromosomal IL2Rγ Gene by Targeted Recombination**

The IL-2Rγ gene encodes a protein, known as the "common cytokine receptor gamma chain," that functions as a subunit of several interleukin receptors (including IL-2R, IL-4R, IL-7R, IL-9R, IL-15R and IL-21R). Mutations in this gene, including those surrounding the 5' end of the third exon (*e.g.* the tyrosine 91 codon), can cause X-linked severe combined immunodeficiency (SCID). See, for example, Puck et al. (1997) Blood 89:1968-1977. A mutation in the tyrosine 91 codon (nucleotides 23-25 of SEQ ID NO: 7; Figure 7), was introduced into the IL2Rγ gene by targeted cleavage and recombination. Cleavage was targeted to this region by designing two pairs of zinc finger proteins. The first pair (first two rows of Table 4) comprises a zinc finger protein designed to bind to nucleotides 29-40 (primary contacts along the top strand as shown in Figure 7) and a zinc finger protein designed to bind to nucleotides 8-20 (primary contacts along the bottom strand). The second pair (third and fourth rows of Table 4) comprises two zinc finger proteins, the first of which recognizes nucleotides 23-34 (primary contacts along the top strand as shown in Figure 7) and the second of which recognizes nucleotides 8-16 (primary contacts along the bottom strand). Zinc finger proteins were designed as described in co-owned US Patents 6,453,242 and 6,534,261. *See* Table 4 for the amino acid sequences of the recognition regions of the zinc finger proteins.

Sequences encoding the ZFP binding domains were fused to sequences encoding a *Fok*I cleavage half-domain (amino acids 384-579 of the native *Fok*I sequence, Kita *et al., supra*), such that the encoded protein contained *Fok*I sequences at the carboxy terminus and ZFP sequences at the amino terminus. Each of these fusion sequences was then cloned in a modified mammalian expression vector pcDNA3. See Figure 8 for a schematic diagram of the constructs.

**Table 4: Zinc Finger Designs for the IL2Rγ Gene**

| Target sequence | F1 | F2 | F3 | F4 |
|---|---|---|---|---|
| AACTCGGATAAT (SEQ ID NO: 43) | DRSTLIE (SEQ ID NO:44) | SSSNLSR (SEQ ID NO:45) | RSDDLSK (SEQ ID NO:46) | DNSNRIK (SEQ ID NO:47) |
| TAGAGGaGAAAGG (SEQ ID NO:48) | RSDNLSN (SEQ ID NO:49) | TSSSRIN (SEQ ID NO:50) | RSDHLSQ (SEQ ID NO:51) | RNADRKT (SEQ ID NO:52) |
| TACAAGAACTCG (SEQ ID NO:53) | RSDDLSK (SEQ ID NO:54) | DNSNRIK (SEQ ID NO:55) | RSDALSV (SEQ ID NO:56) | DNANRTK (SEQ ID NO:57) |
| GGAGAAAGG (SEQ ID NO:58) | RSDHLTQ (SEQ ID NO:59) | QSGNLAR (SEQ ID NO:60) | RSDHLSR (SEQ ID NO:61) | |
| Note: The zinc finger amino acid sequences shown above (in one-letter code) represent residues -1 through +6, with respect to the start of the alpha-helical portion of each zinc finger. Finger F1 is closest to the amino terminus of the protein. | | | | |

A donor DNA molecule was obtained as follows. First, a 700 base pair fragment of human DNA corresponding to positions 69196910-69197609 on the "-" strand of the X chromosome (UCSC, July 2003), which includes exon 3 of the of the IL2Rγ gene, was amplified, using genomic DNA from K562 cells as template. *See* Figure 9. Sequences of primers used for amplification are shown in Table 5 (labeled initial amp 1 and initial amp 2). The PCR product was then altered via standard overlap extension PCR methodology (Ho, *et al., supra*) to replace the sequence TACAAGAACTCGGATAAT (SEQ ID NO: 62) with the sequence TAAAAGAATTCCGACAAC (SEQ ID NO: 63). This replacement results in the introduction of a point mutation at nucleotide 25 (Figure 7), converting the tyrosine 91 codon TAC to a TAA termination codon and enables discrimination between donor-derived and endogenous chromosomal sequences following recombination, because of differences in the sequences downstream of codon 91. The resulting 700 base pair fragment was cloned into pCR4BluntTopo which does not contain any sequences homologous to the human genome. *See* Figure 10.

For targeted mutation of the chromosomal IL2Rγ gene, the donor plasmid, along with two plasmids each encoding one of a pair of ZFP-*Fok*I fusions, were introduced into $2 \times 10^6$ K652 cells using mixed lipofection/electroporation (Amaxa). Each of the ZFP/*Fok*I pairs (see Table 4) was tested in separate experiments. Controls included cells transfected only with two plasmids encoding ZFP-*Fok*I fusions, and cells transfected only with the donor plasmid. Cells were cultured in 5% $CO_2$ at 37°C. At 48 hours after transfection, genomic DNA was isolated from the cells, and 200 ng was used as template for PCR amplification, using one primer complementary to a region of the gene outside of its region of homology with the donor sequences (nucleotides 69196839-69196863 on the "+" strand of the X chromosome; UCSC, July 2003), and a second primer complementary to a region of the donor molecule into which distinguishing mutations were introduced (see above) and whose sequence therefore diverges from that of chromosomal DNA. *See* Table 5 for primer sequences, labeled "chromosome-specific" and "donor-specific," respectively. Using these two primers, an amplification product of 500 bp is obtained from genomic DNA in which a targeted recombination event has occurred. Conditions for amplification were: 94°C, 2 min, followed by 35 cycles of 94°C, 30 sec, 62°C, 1 min, 72°C, 45 sec; and a final step of 72°C, 7min.

The results of this analysis (Figure 11) indicate that an amplification product of the expected size (500 base pairs) is obtained with DNA extracted from cells which had been transfected with the donor plasmid and either of the pairs of ZFP-*Fok*I-encoding plasmids. DNA from cells transfected with plasmids encoding a pair of ZFPs only (no donor plasmid) did not result in generation of the 500 bp product, nor did DNA from cells transfected only with the donor plasmid..

**Table 5: Amplification Primers for the IL2Rγ Gene**

| Initial amp 1 | TGTCGAGTACATGAATTGCACTTGG (SEQ ID NO:64) |
|---|---|
| Initial amp 2 | TTAGGTTCTCTGGAGCCCAGGG (SEQ ID NO:65) |
| Chromosome-specific | CTCCAAACAGTGGTTCAAGAATCTG (SEQ ID NO:66) |
| Donor-specific | TCCTCTAGGTAAAGAATTCCGACAAC (SEQ ID NO:67) |

To confirm this result, the amplification product obtained from the experiment using the second pair of ZFP/*Fok*I fusions was cloned into pCR4Blunt-Topo (Invitrogen) and its nucleotide sequence was determined. As shown in Figure 12 (SEQ ID NO: 12), the sequence consists of a fusion between chromosomal sequences and sequences from the donor plasmid. In particular, the G to A mutation converting tyrosine 91 to a stop codon is observed at position 43 in the sequence. Positions 43-58 contain nucleotides unique to the donor; nucleotides 32-42 and 59-459 are sequences common to the donor and the chromosome, and nucleotides 460-552 are unique to the chromosome. The presence of donor-unique sequences covalently linked to sequences present in the chromosome but not in the donor indicates that DNA from the donor plasmid was introduced into the chromosome by homologous recombination.

**Example 3: Editing of a Chromosomal β-globin Gene by Targeted Recombination**

The human beta globin gene is one of two gene products responsible for the structure and function of hemoglobin in adult human erythrocytes. Mutations in the beta-globin gene can result in sickle cell anemia. Two zinc finger proteins were designed to bind within this sequence, near the location of a nucleotide which, when mutated, causes sickle cell anemia. Figure 13 shows the nucleotide sequence of a portion of the human beta-globin gene, and the target sites for the two zinc finger proteins are underlined in the sequence presented in Figure 13. Amino acid sequences of the recognition regions of the two zinc finger proteins are shown in Table 6. Sequences encoding each of these two ZFP binding domains were fused to sequences encoding a *Fok*I cleavage half-domain, as described above, to create engineered ZFP-nucleases that targeted the endogenous beta globin gene. Each of these fusion sequences was then cloned in the mammalian expression vector pcDNA3.1 (Figure 14).

**Table 6: Zinc Finger Designs for the beta-globin Gene**

| Target sequence | F1 | F2 | F3 | F4 |
|---|---|---|---|---|
| GGGCAGTAACGG (SEQ ID NO: 68 | RSDHLSE (SEQ ID NO: 69) | QSANRTK (SEQ ID NO: 70) | RSDNLSA (SEQ ID NO: 71) | RSQNRTR (SEQ ID NO: 72) |
| AAGGTGAACGTG (SEQ ID NO: 73) | RSDSLSR (SEQ ID NO: 74) | DSSNRKT (SEQ ID NO: 75) | RSDSLSA (SEQ ID NO: 76) | RNDNRKT (SEQ ID NO: 77) |
| Note: The zinc finger amino acid sequences shown above (in one-letter code) represent residues -1 through +6, with respect to the start of the alpha-helical portion of each zinc finger. Finger F1 is closest to the amino terminus of the protein, and Finger F4 is closest to the carboxy terminus. | | | | |

A donor DNA molecule was obtained as follows. First, a 700 base pair fragment of human genomic DNA corresponding to nucleotides 5212134 - 5212833 on the "-" strand of Chromosome 11 (BLAT, UCSC Human Genome site) was amplified by PCR, using genomic DNA from K562 cells as template. Sequences of primers used for amplification are shown in Table 7 (labeled initial amp 1 and initial amp 2). The resulting amplified fragment contains sequences corresponding to the promoter, the first two exons and the first intron of the human beta globin gene. *See* Figure 15 for a schematic illustrating the locations of exons 1 and 2, the first intron, and the primer binding sites in the beta globin sequence. The cloned product was then further modified by PCR to introduce a set of sequence changes between nucleotides 305-336 (as shown in Figure 13), which replaced the sequence CCGTTACTGCCCTGTGGGGCAAGGTGAACGTG (SEQ ID NO: 78) with **g**CGTTA**g**TGCCC**GAATTCCG**A**tc**GT**c**AAC**cac** (SEQ ID NO: 79) (changes in bold). Certain of these changes (shown in lowercase) were specifically engineered to prevent the ZFP/*Fok*I fusion proteins from binding to and cleaving the donor sequence, once integrated into the chromosome. In addition, all of the sequence changes enable discrimination between donor and endogenous chromosomal sequences following recombination. The resulting 700 base pair fragment was cloned into pCR4-TOPO, which does not contain any sequences homologous to the human genome (Figure 16).

For targeted mutation of the chromosomal beta globin gene, the two plasmids encoding ZFP-*Fok*I fusions and the donor plasmid (pCR4-TOPO-HBBdonor) were introduced into 1 X $10^6$ K562 cells by transfection using Nucleofector™ Solution (Amaxa Biosystems). Controls included cells transfected only with 100 ng (low) or 200 ng (high) of the two plasmids encoding the ZFP-*Fok*I fusions, cells transfected only with 200 ng (low) or 600 ng (high) of the donor plasmid, cells transfected with a GFP-encoding plasmid, and mock transfected cells. Cells were cultured in RPMI Medium 1640 (Invitrogen), supplemented with 10% fetal bovine serum (FBS) (Hyclone) and 2 mM L-glutamine. Cells were maintained at 37°C in an atmosphere of 5% $CO_2$. At 72 hours after transfection, genomic DNA was isolated from the cells, and 200 ng was used as template for PCR amplification, using one primer complementary to a region of the gene outside of its region of homology with the donor sequences (nucleotides 5212883-5212905 on the "-" strand of chromosome 11), and a second primer complementary to a region of the donor molecule into which distinguishing mutations were introduced into the donor sequence (see *supra*). The sequences of these primers are given in Table 7 (labeled "chromosome-specific" and "donor-specific," respectively). Using these two primers, an amplification product of 415 base pairs will be obtained from genomic DNA if a targeted recombination event has occurred. As a control for DNA loading, PCR reactions were also carried out using the Initial amp 1 and Initial amp 2 primers to ensure that similar levels of genomic DNA were added to each PCR reaction. Conditions for amplification were: 95°C, 2 min, followed by 40 cycles of 95°C, 30 sec, 60°C, 45 sec, 68°C, 2 min; and a final step of 68°C, 10 min.

The results of this analysis (Figure 17) indicate that a 415 base pair amplification product was obtained only with DNA extracted from cells which had been transfected with the "high" concentration of donor plasmid and both ZFP-*Fok*I plasmids, consistent with targeted recombination of donor sequences into the chromosomal beta-globin locus.

**Table 7: Amplification Primers for the human beta globin gene**

| Initial amp 1 | TACTGATGGTATGGGGCCAAGAG (SEQ ID NO:80) |
|---|---|
| Initial amp 2 | CACGTGCAGCTTGTCACAGTGC (SEQ ID NO:81) |
| Chromosome-specific | TGCTTACCAAGCTGTGATTCCA (SEQ ID NO:82) |
| Donor-specific | GGTTGACGATCGGAATTC (SEQ ID NO:83) |

To confirm this result, the amplification product was cloned into pCR4-TOPO (Invitrogen) and its nucleotide sequence was determined. As shown in Figure 18 (SEQ ID NO: 14), the sequence consists of a fusion between chromosomal sequences not present on the donor plasmid and sequences unique to the donor plasmid. For example, two C→G mutations which disrupt ZFP-binding are observed at positions 377 and 383 in the sequence. Nucleotides 377-408 represent sequence obtained from the donor plasmid containing the sequence changes described above; nucleotides 73-376 are sequences common to the donor and the chromosome, and nucleotides 1-72 are unique to the chromosome. The covalent linkage of donor-specific and chromosome-specific sequences in the genome confirms the successful recombination of the donor sequence at the correct locus within the genome of K562 cells.

**Example 4: ZFP-FokI linker (ZC linker) optimization**

In order to test the effect of ZC linker length on cleavage efficiency, a four-finger ZFP binding domain was fused to a *Fok*I cleavage half-domain, using ZC linkers of various lengths. The target site for the ZFP is 5'-AACTCGGATAAT-3' (SEQ ID NO:84) and the amino acid sequences of the recognition regions (positions -1 through +6 with respect to the start of the alpha-helix) of each of the zinc fingers were as follows (wherein F1 is the N-most, and F4 is the C-most zinc finger):

F1: DRSTLIE (SEQ ID NO:85)
F2: SSSNLSR (SEQ ID NO:86)
F3: RSDDLSK (SEQ ID NO:87)
F4: DNSNRIK (SEQ ID NO:88)

ZFP-*Fok*I fusions, in which the aforementioned ZFP binding domain and a *Fok*I cleavage half-domain were separated by 2, 3, 4, 5, 6, or 10 amino acid residues, were constructed. Each of these proteins was tested for cleavage of substrates having an inverted repeat of the ZFP target site, with repeats separated by 4, 5, 6, 7, 8, 9, 12, 15, 16, 17, 22, or 26 basepairs.

The amino acid sequences of the fusion constructs, in the region of the ZFP-*Fok*I junction (with the ZC linker sequence underlined), are as follows:

10-residue linker HTKIH<u>LRQKDAARGS</u>QLV (SEQ ID NO:89)
6-residue linker HTKIH<u>LRQKGS</u>QLV (SEQ ID NO:90)
5-residue linker HTKIH<u>LRQGS</u>QLV (SEQ ID NO:91)
4-residue linker HTKIH<u>LRGS</u>QLV (SEQ ID NO:92)
3-residue linker HTKIH<u>LGS</u>QLV (SEQ ID NO:93)
2-residue linker HTKIHGSQLV (SEQ ID NO:94)

The sequences of the various cleavage substrates, with the ZFP target sites underlined, are as follows:

4bp separation

```
CTAGCATTATCCGAGTTACACAACTCGGATAATGCTAG
GATCGTAATAGGCTCAATGTGTTGAGCCTATTACGATC
(SEQ ID NO:95)
```

5bp separation

```
CTAGCATTATCCGAGTTCACACAACTCGGATAATGCTAG
GATCGTAATAGGCTCAAGTGTGTTGAGCCTATTACGATC
(SEQ ID NO:96)
```

6bp separation

```
CTAGGCATTATCCGAGTTCACCACAACTCGGATAATGACTAG
GATCCGTAATAGGCTCAAGTGGTGTTGAGCCTATTACTGATC
(SEQ ID NO:97)
```

7bp separation

```
CTAGCATTATCCGAGTTCACACACAACTCGGATAATGCTAG
GATCGTAATAGGCTCAAGTGTGTGTTGAGCCTATTACGATC
(SEQ ID NO:98)
```

8bp separation

```
CTAGCATTATCCGAGTTCACCACACAACTCGGATAATGCTAG
GATCGTAATAGGCTCAAGTGGTGTGTTGAGCCTATTACGATC
(SEQ ID NO:99)
```

9bp separation

```
CTAGCATTATCCGAGTTCACACACACAACTCGGATAATGCTAG
GATCGTAATAGGCTCAAGTGTGTGTGTTGAGCCTATTACGATC
(SEQ ID NO:100)
```

12bp separation

```
CTAGCATTATCCGAGTTCACCACCAACACAACTCGGATAATGCTAG
GATCGTAATAGGCTCAAGTGGTGGTTGTGTTGAGCCTATTACGATC
(SEQ ID NO:101)
```

15bp separation

```
CTAGCATTATCCGAGTTCACCACCAACCACACAACTCGGATAATGCTAG
GATCGTAATAGGCTCAAGTGGTGGTTGGTGTGTTGAGCCTATTACGATC
(SEQ ID NO:102)
```

16bp separation

```
CTAGCATTATCCGAGTTCACCACCAACCACACCAACTCGGATAATGCTAG
GATCGTAATAGGCTCAAGTGGTGGTTGGTGTGGTTGAGCCTATTACGATC
(SEQ ID NO:103)
```

17bp separation

```
CTAGCATTATCCGAGTTCAACCACCAACCACACCAACTCGGATAATGCTAG
GATCGTAATAGGCTCAAGTTGGTGGTTGGTGTGGTTGAGCCTATTACGATC
(SEQ ID NO:104)
```

22bp separation

```
CTAGCATTATCCGAGTTCAACCACCAACCACACCAACACAACTCGGATAATGCTAG
GATCGTAATAGGCTCAAGTTGGTGGTTGGTGTGGTTGTGTTGAGCCTATTACGATC
(SEQ ID NO:105)
```

26bp separation

```
CTAGCATTATCCGAGTTCAACCACCAACCACACCAACACCACCAACTCGGATAATGCTAG
GATCGTAATAGGCTCAAGTTGGTGGTTGGTGTGGTTGTGGTGGTTGAGCCTATTACGATC
(SEQ ID NO:106)
```

Plasmids encoding the different ZFP-*Fok*I fusion proteins (see above) were constructed by standard molecular biological techniques, and an *in vitro* coupled transcription/translation system was used to express the encoded proteins. For each construct, 200 ng linearized plasmid DNA was incubated in 20 $\mu$L TnT mix and incubated at 30° C for 1 hour and 45 minutes. TnT mix contains 100 $\mu$l TnT lysate (Promega, Madison, WI) with 4 $\mu$l T7 RNA polymerase (Promega) + 2 $\mu$l Methionine (1 mM) + 2.5 $\mu$l ZnCl$_2$ (20 mM).

For analysis of DNA cleavage by the different ZFP-*Fok*I fusions, 1 ul of the coupled transcription/translation reaction mixture was combined with approximately 1 ng DNA substrate (end-labeled with $^{32}$P using T4 polynucleotide kinase), and the mixture was diluted to a final volume of 19 $\mu$l with *Fok*I Cleavage Buffer. *Fok*I Cleavage buffer contains 20 mM Tris-HCl pH 8.5, 75 mM NaCl, 10 $\mu$M ZnCl$_2$, 1 mM DTT, 5% glycerol, 500 $\mu$g/ml BSA. The mixture was incubated for 1 hour at 37° C. 6.5 $\mu$l of FokI buffer, also containing 8 mM MgCl$_2$, was then added and incubation was continued for one hour at 37° C. Protein was extracted by adding 10 $\mu$l phenol-chloroform solution to each reaction, mixing, and centrifuging to separate the phases. Ten microliters of the aqueous phase from each reaction was analyzed by electrophoresis on a 10% polyacrylamide gel.

The gel was subjected to autoradiography, and the cleavage efficiency for each ZFP-*Fok*I fusion/substrate pair was calculated by quantifying the radioactivity in bands corresponding to uncleaved and cleaved substrate, summing to obtain total radioactivity, and determining the percentage of the total radioactivity present in the bands representing cleavage products.

The results of this experiment are shown in Table 8. This data allows the selection of a ZC linker that provides optimum cleavage efficiency for a given target site separation. This data also allows the selection of linker lengths that allow cleavage at a selected pair of target sites, but discriminate against cleavage at the same or similar ZFP target sites that have a separation that is different from that at the intended cleavage site.

**Table 8: DNA cleavage efficiency for various ZC linker lengths and various binding site separations\***

|  | 2-residue | 3-residue | 4-residue | 5-residue | 6-residue | 10-residue |
|---|---|---|---|---|---|---|
| 4 bp | 74% | 81% | 74% | 12% | 6% | 4% |
| 5 bp | 61% | 89% | 92% | 80% | 53% | 40% |
| 6 bp | 78% | 89% | 95% | 91% | 93% | 76% |
| 7 bp | 15% | 55% | 80% | 80% | 70% | 80% |
| 8 bp | 0% | 0% | 8% | 11% | 22% | 63% |
| 9 bp | 2% | 6% | 23% | 9% | 13% | 51% |
| 12 bp | 8% | 12% | 22% | 40% | 69% | 84% |
| 15 bp | 73% | 78% | 97% | 92% | 95% | 88% |
| 16 bp | 59% | 89% | 100% | 97% | 90% | 86% |
| 17 bp | 5% | 22% | 77% | 71% | 85% | 82% |
| 22 bp | 1% | 3% | 5% | 8% | 18% | 58% |
| 26 bp | 1% | 2% | 35% | 36% | 84% | 78% |

\* The columns represent different ZFP-*Fok*I fusion constructs with the indicated number of residues separating the ZFP and the *Fok*I cleavage half-domain. The rows represent different DNA substrates with the indicated number of basepairs separating the inverted repeats of the ZFP target site.

For ZFP-*Fok*I fusions with four residue linkers, the amino acid sequence of the linker was also varied. In separate constructs, the original LRGS linker sequence (SEQ ID NO:107) was changed to LGGS (SEQ ID NO: 108), TGGS (SEQ ID NO:109), GGGS (SEQ ID NO:110), LPGS (SEQ ID NO:111), LRKS (SEQ ID NO:112), and LRWS (SEQ ID NO:113); and the resulting fusions were tested on substrates having a six-basepair separation between binding sites. Fusions containing the LGGS (SEQ ID NO:108) linker sequence were observed to cleave more efficiently than those containing the original LRGS sequence(SEQ ID NO:107). Fusions containing the LRKS(SEQ ID NO:112) and LRWS(SEQ ID NO:113) sequences cleaved with less efficiency than the LRGS sequence(SEQ ID NO: 107), while the cleavage efficiencies of the remaining fusions were similar to that of the fusion comprising the original LRGS sequence(SEQ ID NO:107).

**Example 5: Increased cleavage specificity resulting from alteration of the *Fok*I cleavage half-domain in the dimerization interface**

A pair of ZFP/*Fok*I fusion proteins (denoted 5-8 and 5-10) were designed to bind to target sites in the fifth exon of the IL-2R$\gamma$ gene, to promote cleavage in the region between the target sites. The relevant region of the gene, including the target sequences of the two fusion proteins, is shown in Figure 19. The amino acid sequence of the 5-8 protein is shown in Figure 20, and the amino acid sequence of the 5-10 protein is shown in Figure 21. Both proteins contain a 10 amino acid ZC linker. With respect to the zinc finger portion of these proteins, the DNA target sequences, as well as amino acid sequences of the recognition regions in the zinc fingers, are given in Table 9.

**Table 9: Zinc Finger Designs for the IL2Rγ Gene**

| Fusion | Target sequence | F1 | F2 | F3 | F4 |
|---|---|---|---|---|---|
| 5-8 | ACTCTGTGGAAG (SEQ ID NO:114) | RSDNLSE (SEQ ID NO:115) | RNAHRIN (SEQ ID NO:116) | RSDTLSE (SEQ ID NO:117) | ARSTRTT (SEQ ID NO:118) |
| 5-10 | AACACGaAACGTG (SEQ ID NO:119) | RSDSLSR (SEQ ID NO:120) | DSSNRKT (SEQ ID NO:121) | RSDSLSV (SEQ ID NO:122) | DRSNRIT (SEQ ID NO:123) |
| Note: The zinc finger amino acid sequences shown above (in one-letter code) represent residues -1 through +6, with respect to the start of the alpha-helical portion of each zinc finger. Finger F1 is closest to the amino terminus of the protein. | | | | | |

The ability of this pair of fusion proteins to catalyze specific cleavage of DNA between their target sequences (see Figure 19) was tested *in vitro* using a labeled DNA template containing the target sequence and assaying for the presence of diagnostic digestion products. Specific cleavage was obtained when both proteins were used (Table 10, first row). However, the 5-10 fusion protein (comprising a wild-type *Fok*I cleavage half-domain) was also capable of aberrant cleavage at a non-target site in the absence of the 5-8 protein (Table 10, second row), possibly due to self-dimerization.

Accordingly, 5-10 was modified in its *Fok*I cleavage half-domain by converting amino acid residue 490 from glutamic acid (E) to lysine (K). (Numbering of amino acid residues in the *Fok*I protein is according to Wah *et al., supra*.) This modification was designed to prevent homodimerization by altering an amino acid residue in the dimerization interface. The 5-10 (E490K) mutant, unlike the parental 5-10 protein, was unable to cleave at aberrant sites in the absence of the 5-8 fusion protein (Table 10, Row 3). However, the 5-10 (E490K) mutant, together with the 5-8 protein, catalyzed specific cleavage of the substrate (Table 10, Row 4). Thus, alteration of a residue in the cleavage half-domain of 5-10, that is involved in dimerization, prevented aberrant cleavage by this fusion protein due to self-dimerization. An E490R mutant also exhibits lower levels of homodimerization than the parent protein.

In addition, the 5-8 protein was modified in its dimerization interface by replacing the glutamine (Q) residue at position 486 with glutamic acid (E). This 5-8 (Q486E) mutant was tested for its ability to catalyze targeted cleavage in the presence of either the wild-type 5-10 protein or the 5-10 (E490K) mutant. DNA cleavage was not observed when the labeled substrate was incubated in the presence of both 5-8 (Q486E) and wild-type 5-10 (Table 10, Row 5). However, cleavage was obtained when the 5-8 (Q486E) and 5-10 (E490K) mutants were used in combination (Table 10, Row 6).

These results indicate that DNA cleavage by a ZFP/*Fok*I fusion protein pair, at regions other than that defined by the target sequences of the two fusion proteins, can be minimized or abolished by altering the amino acid sequence of the cleavage half-domain in one or both of the fusion proteins.

**Table 10: DNA cleavage by ZFP/*Fok*I fusion protein pairs containing wild-type and mutant cleavage half-domains**

| | | ZFP 5-8 binding domain | ZFP 5-10 binding domain | DNA cleavage |
|---|---|---|---|---|
| | 1 | Wild-type *Fok*I | Wild-type *Fok*I | Specific |
| | 2 | Not present | Wild-type *Fok*I | Non-specific |
| | 3 | Not present | *Fok*I E490K | None |
| | 4 | Wild-type *Fok*I | *Fok*I E490K | Specific |
| | 5 | *Fok*I Q486E | Wild-type *Fok*I | None |

(continued)

| | ZFP 5-8 binding domain | ZFP 5-10 binding domain | DNA cleavage |
|---|---|---|---|
| 6 | *Fok*I Q486E | *Fok*I E490K | Specific |

Note: Each row of the table presents results of a separate experiment in which ZFP/*Fok*I fusion proteins were tested for cleavage of a labeled DNA substrate. One of the fusion proteins contained the 5-8 DNA binding domain, and the other fusion protein contained the 5-10 DNA binding domain (See Table 9 and Figure 19). The cleavage half-domain portion of the fusion proteins was as indicated in the Table. Thus, the entries in the ZFP 5-8 column indicate the type of *Fok*I cleavage domain fused to ZFP 5-8; and the entries in the ZFP 5-10 column indicates the type of *Fok*I cleavage domain fused to ZFP 5-10. For the *Fok*I cleavage half-domain mutants, the number refers to the amino acid residue in the *Fok*I protein; the letter preceding the number refers to the amino acid present in the wild-type protein and the letter following the number denotes the amino acid to which the wild-type residue was changed in generating the modified protein. 'Not present' indicates that the entire ZFP/*Fok*I fusion protein was omitted from that particular experiment.

The DNA substrate used in this experiment was an approximately 400 bp PCR product containing the target sites for both ZFP 5-8 and ZFP 5-10. See Figure 19 for the sequences and relative orientation of the two target sites.

**Example 6: Generation of a defective enhanced Green Fluorescent Protein (eGFP) gene**

The enhanced Green Fluorescent Protein (eGFP) is a modified form of the Green Fluorescent Protein (GFP; *see, e.g.,* Tsien (1998) Ann. Rev. Biochem. 67:509-544) containing changes at amino acid 64 (phe to leu) and 65 (ser to thr). Heim et al. (1995) Nature 373:663-664; Cormack et al. (1996) Gene 173:33-38. An eGFP-based reporter system was constructed by generating a defective form of the eGFP gene, which contained a stop codon and a 2-bp frameshift mutation. The sequence of the eGFP gene is shown in Figure 22. The mutations were inserted by overlapping PCR mutagenesis, using the Platinum® *Taq* DNA Polymerase High Fidelity kit (Invitrogen) and the oligonucleotides GFP-Bam, GFP-Xba, stop sense2, and stop anti2 as primers (oligonucleotide sequences are listed below in Table 11). GFP-Bam and GFP-Xba served as the external primers, while the primers stop sense2 and stop anti2 served as the internal primers encoding the nucleotide changes. The peGFP-NI vector (BD Biosciences), encoding a full-length eGFP gene, was used as the DNA template in two separate amplification reactions, the first utilizing the GFP-Bam and stop anti2 oligonucleotides as primers and the second using the GFP-Xba and stop sense2 oligonucleotides as primers. This generated two amplification products whose sequences overlapped. These products were combined and used as template in a third amplification reaction, using the external GFP-Bam and GFP-Xba oligonucleotides as primers, to regenerate a modified eGFP gene in which the sequence GACCACAT (SEQ ID NO: 124) at nucleotides 280-287 was replaced with the sequence TAACAC (SEQ ID NO: 125). The PCR conditions for all amplification reactions were as follows: the template was initially denatured for 2 minutes at 94 degrees and followed by 25 cycles of amplification by incubating the reaction for 30 sec. at 94 degrees C, 45 sec. at 46 degrees C, and 60 sec. at 68 degrees C. A final round of extension was carried out at 68 degrees C for 10 minutes. The sequence of the final amplification product is shown in Figure 23. This 795 bp fragment was cloned into the pCR(R)4-TOPO vector using the TOPO-TA cloning kit (Invitrogen) to generate the pCR(R)4-TOPO-GFPmut construct.

**Table 11: Oligonucleotide sequences for GFP**

| Oligo | sequence 5'-3' |
|---|---|
| GFP-Bam | CGAATTCTGCAGTCGAC (SEQ ID NO:126) |
| GFP-Xba | GATTATGATCTAGAGTCG (SEQ ID NO:127) |
| stop sense2 | AGCCGCTACCCCTAACACGAAGCAG (SEQ ID NO:128) |
| stop anti2 | CTGCTTCGTGTTAGGGGTAGCGGCT (SEQ ID NO:129) |

**Example 7: Design and assembly of Zinc Finger Nucleases targeting eGFP**

Two three-finger ZFPs were designed to bind a region of the mutated GFP gene (Example 6) corresponding to nucleotides 271-294 (numbering according to Figure 23). The binding sites for these proteins occur in opposite orientation with 6 base pairs separating the two binding sites. *See* Figure 23. ZFP 287A binds nucleotides 271-279 on the non-coding strand, while ZFP 296 binds nucleotides 286-294 on the coding strand. The DNA target and amino acid sequence for the recognition regions of the ZFPs are listed below, and in Table 12:
287A:

F1 (GCGg)      RSDDLTR (SEQ ID NO: 130)
F2 (GTA)       QSGALAR (SEQ ID NO:131)
F3 (GGG)       RSDHLSR (SEQ ID NO:132)

296S:

F1 (GCA)       QSGSLTR (SEQ ID NO:133)
F2 (GCA)       QSGDLTR (SEQ ID NO:134)
F3 (GAA)       QSGNLAR (SEQ ID NO:135)

**Table 12: Zinc finger designs for the GFP gene**

| Protein | Target sequence | F1 | F2 | F3 |
|---|---|---|---|---|
| 287A | GGGGTAGCGg (SEQ ID NO:136) | RSDDLTR (SEQ ID NO: 137) | QSGALAR (SEQ ID NO: 138) | RSDHLSR (SEQ ID NO: 139) |
| 296S | GAAGCAGCA(SEQ ID NO:140) | QSGSLTR (SEQ ID NO: 141) | QSGDLTR (SEQ ID NO: 142) | QSGNLAR (SEQ ID NO: 143) |
| Note: The zinc finger amino acid sequences shown above (in one-letter code) represent residues -1 through +6, with respect to the start of the alpha-helical portion of each zinc finger. Finger F1 is closest to the amino terminus of the protein, and Finger F3 is closest to the carboxy terminus. | | | | |

Sequences encoding these proteins were generated by PCR assembly (*e.g.*, U.S. Patent No. 6,534,261), cloned between the *Kph*I and *Bam*HI sites of the pcDNA3.1 vector (Invitrogen), and fused in frame with the catalytic domain of the *Fok*I endonuclease (amino acids 384-579 of the sequence of Looney et al. (1989) Gene 80:193-208). The resulting constructs were named pcDNA3.1-GFP287-FokI and pcDNA3.1-GFP296-FokI (Figure 24).

**Example 8: Targeted *in vitro* DNA cleavage by designed Zinc Finger Nucleases**

The pCR(R)4-TOPO-GFPmut construct (Example 6) was used to provide a template for testing the ability of the 287 and 296 zinc finger proteins to specifically recognize their target sites and cleave this modified form of eGFP *in vitro.*
A DNA fragment containing the defective eGFP-encoding insert was obtained by PCR amplification, using the T7 and T3 universal primers and pCR(R)4-TOPO-GFPmut as template. This fragment was end-labeled using $\gamma$-$^{32}$P-ATP and T4 polynucleotide kinase. Unincorporated nucleotide was removed using a microspin G-50 column (Amersham).
An *in vitro* coupled transcription/translation system was used to express the 287 and 296 zinc finger nucleases described in Example 7. For each construct, 200 ng linearized plasmid DNA was incubated in 20 $\mu$L TnT mix and incubated at 30° C for 1 hour and 45 minutes. TnT mix contains 100 $\mu$l TnT lysate (which includes T7 RNA polymerase, Promega, Madison, WI) supplemented with 2 $\mu$l Methionine (1 mM) and 2.5 $\mu$l ZnCl$_2$ (20 mM).
For analysis of DNA cleavage, aliquots from each of the 287 and 296 coupled transcription/translation reaction mixtures were combined, then serially diluted with cleavage buffer. Cleavage buffer contains 20 mM Tris-HCl pH 8.5, 75 mM NaCl, 10 mM MgCl$_2$, 10 $\mu$M ZnCl$_2$, 1 mM DTT, 5% glycerol, 500 $\mu$g/ml BSA. 5$\mu$l of each dilution was combined with approximately 1 ng DNA substrate (end-labeled with $^{32}$P using T4 polynucleotide kinase as described above), and each mixture was further diluted to generate a 20 $\mu$l cleavage reaction having the following composition: 20 mM Tris-HCl pH 8.5, 75 mM NaCl, 10 mM MgCl$_2$, 10 $\mu$M ZnCl$_2$, 1 mM DTT, 5% glycerol, 500 $\mu$g/ml BSA. Cleavage reactions were incubated for 1 hour at 37°C. Protein was extracted by adding 10 $\mu$l phenol-chloroform solution to each reaction, mixing, and centrifuging to separate the phases. Ten microliters of the aqueous phase from each reaction was analyzed by electrophoresis on a 10% polyacrylamide gel.
The gel was subjected to autoradiography, and the results of this experiment are shown in Figure 25. The four left-most lanes show the results of reactions in which the final dilution of each coupled transcription/translation reaction mixture (in the cleavage reaction) was 1/156.25, 1/31.25, 1/12.5 and 1/5, respectively, resulting in effective volumes of 0.032, 0.16, 04. and 1 ul, respectively of each coupled transcription/translation reaction. The appearance of two DNA fragments having lower molecular weights than the starting fragment (lane labeled "uncut control" in Figure 25) is correlated with increasing amounts of the 287 and 296 zinc finger endonucleases in the reaction mixture, showing that DNA cleavage at the expected target site was obtained.

**Example 9: Generation of stable cell lines containing an integrated defective eGFP gene**

A DNA fragment encoding the mutated eGFP, eGFPmut, was cleaved out of the pCR(R)4-TOPO-GFPmut vector (Example 6) and cloned into the *Hind*III and *Not*I sites of pcDNA4/TO, thereby placing this gene under control of a tetracycline-inducible CMV promoter. The resulting plasmid was named pcDNA4/TO/GFPmut (Figure 26). T-Rex 293 cells (Invitrogen) were grown in Dulbecco's modified Eagle's medium (DMEM) (Invitrogen) supplemented with 10% Tet-free fetal bovine serum (FBS) (HyClone). Cells were plated into a 6-well dish at 50% confluence, and two wells were each transfected with pcDNA4/TO/GFPmut. The cells were allowed to recover for 48 hours, then cells from both wells were combined and split into 10x15-cm$^2$ dishes in selective medium, *i.e.,* medium supplemented with 400 ug/ml Zeocin (Invitrogen). The medium was changed every 3 days, and after 10 days single colonies were isolated and expanded further. Each clonal line was tested individually for doxycycline(dox)-inducible expression of the eGFPmut gene by quantitative RT-PCR (TaqMan®).

For quantitative RT-PCR analysis, total RNA was isolated from dox-treated and untreated cells using the High Pure Isolation Kit (Roche Molecular Biochemicals), and 25 ng of total RNA from each sample was subjected to real time quantitative RT-PCR to analyze endogenous gene expression, using TaqMan® assays. Probe and primer sequences are shown in Table 13. Reactions were carried out on an ABI 7700 SDS machine (PerkinElmer Life Sciences) under the following conditions. The reverse transcription reaction was performed at 48° C for 30 minutes with MultiScribe reverse transcriptase (PerkinElmer Life Sciences), followed by a 10-minute denaturation step at 95°C. Polymerase chain reaction (PCR) was carried out with AmpliGold DNA polymerase (PerkinElmer Life Sciences) for 40 cycles at 95°C for 15 seconds and 60°C for 1 minute. Results were analyzed using the SDS version 1.7 software and are shown in Figure 27, with expression of the eGFPmut gene normalized to the expression of the human GAPDH gene. A number of cell lines exhibited doxycycline-dependent expression of eGFP; line 18 (T18) was chosen as a model cell line for further studies.

**Table 13: Oligonucleotides for mRNA analysis**

| Oligonucleotide | Sequence |
|---|---|
| eGFP primer 1 (5T) | CTGCTGCCCGACAACCA (SEQ ID NO:144) |
| eGFP primer 2 (3T) | CCATGTGATCGCGCTTCTC (SEQ ID NO:145) |
| eGFP probe | CCCAGTCCGCCCTGAGCAAAGA (SEQ ID NO:146) |
| GAPDH primer 1 | CCATGTTCGTCATGGGTGTGA (SEQ ID NO:147) |
| GAPDH primer 2 | CATGGACTGTGGTCATGAGT (SEQ ID NO:148) |
| GAPDH probe | TCCTGCACCACCAACTGCTTAGCA (SEQ ID NO:149) |

**Example 10: Generation of a donor sequence for correction of a defective chromosomal eGFP gene**

A donor construct containing the genetic information for correcting the defective eGFPmut gene was constructed by PCR. The PCR reaction was carried out as described above, using the peGFP-NI vector as the template. To prevent background expression of the donor construct in targeted recombination experiments, the first 12 bp and start codon were removed from the donor by PCR using the primers GFPnostart and GFP-Xba (sequences provided in Table 14). The resulting PCR fragment (734 bp) was cloned into the pCR(R)4-TOPO vector, which does not contain a mammalian cell promoter, by TOPO-TA cloning to create pCR(R)4-TOPO-GFPdonor5 (Figure 28). The sequence of the eGFP insert of this construct (corresponding to nucleotides 64-797 of the sequence shown in Figure 22) is shown in Figure 29 (SEQ ID NO:20).

**Table 14: Oligonucleotides for construction of donor molecule**

| Oligonucleotide | Sequence 5'-3' |
|---|---|
| GFPnostart | GGCGAGGAGCTGTTCAC (SEQ ID NO:150) |
| GFP-Xba | GATTATGATCTAGAGTCG (SEQ ID NO:151) |

**Example 11: Correction of a mutation in an integrated chromosomal eGFP gene by targeted cleavage and recombination**

The T18 stable cell line (Example 9) was transfected with one or both of the ZFP-FokI expression plasmid (pcDNA3.1-GFP287-FokI and pcDNA3.1-GFP296-FokI, Example 7) and 300 ng of the donor plasmid pCR(R)4-TOPO-GFPdonor5 (Example 10) using LipofectAMINE 2000 Reagent (Invitrogen) in Opti-MEM I reduced serum medium, according to the manufacturer's protocol. Expression of the defective chromosomal eGFP gene was induced 5-6 hours after transfection by the addition of 2 ng/ml doxycycline to the culture medium. The cells were arrested in the G2 phase of the cell cycle by the addition, at 24 hours post-transfection, of 100 ng/ml Nocodazole (Figure 30) or 0.2 uM Vinblastine (Figure 31). G2 arrest was allowed to continue for 24-48 hours, and was then released by the removal of the medium. The cells were washed with PBS and the medium was replaced with DMEM containing tetracycline-free FBS and 2 ng/ml doxycycline. The cells were allowed to recover for 24-48 hours, and gene correction efficiency was measured by monitoring the number of cells exhibiting eGFP fluorescence, by fluorescence-activated cell sorting (FACS) analysis. FACS analysis was carried out using a Beckman-Coulter EPICS XL-MCL instrument and System II Data Acquisition and Display software, version 2.0. eGFP fluorescence was detected by excitation at 488 nm with an argon laser and monitoring emissions at 525 nm (x-axis). Background or autofluorescence was measured by monitoring emissions at 570 nm (y-axis). Cells exhibiting high fluorescent emission at 525 nm and low emission at 570 nm (region E) were scored positive for gene correction.

The results are summarized in Table 15 and Figures 30 and 31. Figures 30 and 31 show results in which T18 cells were transfected with the pcDNA3.1-GFP287-FokI and pcDNA3.1-GFP296-FokI plasmids encoding ZFP nucleases and the pCR(R)4-TOPO-GFPdonor5 plasmid, eGFP expression was induced with doxycycline, and cells were arrested in G2 with either nocodazole (Figure 30) or vinblastine (Figure 31). Both figures show FACS traces, in which cells exhibiting eGFP fluorescence are represented in the lower right-hand portion of the trace (identified as Region E, which is the portion of Quadrant 4 underneath the curve). For transfected cells that had been treated with nocodazole, 5.35% of the cells exhibited GFP fluorescence, indicative of correction of the mutant chromosomal eGFP gene (Figure 30), while 6.7% of cells treated with vinblastine underwent eGFP gene correction (Figure 31). These results are summarized, along with additional control experiments, in Rows 1-8 of Table 15.

In summary, these experiments show that, in the presence of two ZFP nucleases and a donor sequence, approximately 1% of treated cells underwent gene correction, and that this level of correction was increased 4-5 fold by arresting treated cells in the G2 phase of the cell cycle.

**Table 15: Correction of a defective chromosomal eGFP gene**

| Expt. | Treatment[1] | Percent cells with corrected eGFP gene[2] |
|---|---|---|
| 1 | 300 ng donor only | 0.01 |
| 2 | 100 ng ZFP 287 + 300 ngdonor | 0.16 |
| 3 | 100 ng ZFP 296 + 300 ng donor | 0.6 |
| 4 | 50 ng ZFP 287 + 50 ng ZFP 296 + 300 ng donor | 1.2 |
| 5 | as 4 + 100 ng/ml nocodazole | 5.35 |
| 6 | as 4 + 0.2 uM vinblastine | 6.7 |
| 7 | no donor, no ZFP, 100 ng/ml nocodazole | 0.01 |
| 8 | no donor, no ZFP, 0.2 uM vinblastine | 0.0 |
| 9 | 100 ng ZFP287/Q486E + 300 ng donor | 0.0 |
| 10 | 100 ng ZFP296/E490K + 300 ng donor | 0.01 |
| 11 | 50 ng 287/Q486E + 50 ng 296/E490K + 300 ng donor | 0.62 |
| 12 | as 11 + 100 ng/ml nocodazole | 2.37 |

(continued)

| Expt. | Treatment[1] | Percent cells with corrected eGFP gene[2] |
|---|---|---|
| 13 | as 11 + 0.2 uM vinblastine | 2.56 |

**Notes:**

1: T18 cells, containing a defective chromosomal eGFP gene, were transfected with plasmids encoding one or two ZFP nucleases and/or a donor plasmid encoding a nondefective eGFP sequence, and expression of the chromosomal eGFP gene was induced with doxycycline. Cells were optionally arrested in G2 phase of the cell cycle after eGFP induction. FACS analysis was conducted 5 days after transfection.

2: The number is the percent of total fluorescence exhibiting high emission at 525 nm and low emission at 570 nm (region E of the FACS trace).

**Example 12: Correction of a defective chromosomal gene using zinc finger nucleases with sequence alterations in the dimerization interface**

Zinc finger nucleases whose sequences had been altered in the dimerization interface were tested for their ability to catalyze correction of a defective chromosomal eGFP gene. The protocol described in Example 11 was used, except that the nuclease portion of the ZFP nucleases (*i.e.,* the *FokI* cleavage half-domains) were altered as described in Example 5. Thus, an E490K cleavage half-domain was fused to the GFP296 ZFP domain (Table 12), and a Q486E cleavage half-domain was fused to the GFP287 ZFP (Table 12).

The results are shown in Rows 9-11 of Table 15 and indicate that a significant increase in the frequency of gene correction was obtained in the presence of two ZFP nucleases having alterations in their dimerization interfaces, compared to that obtained in the presence of either of the nucleases alone. Additional experiments, in which T18 cells were transfected with donor plasmid and plasmids encoding the 287/Q486E and 296/E490K zinc finger nucleases, then arrested in G2 with nocodazole or vinblastine, showed a further increase in frequency of gene correction, with over 2% of cells exhibiting eGFP fluorescence, indicative of a corrected chromosomal eGFP gene (Table 15, Rows 12 and 13).

**Example 13: Effect of donor length on frequency of gene correction**

In an experiment similar to those described in Example 11, the effect of the length of donor sequence on frequency of targeted recombination was tested. T18 cells were transfected with the two ZFP nucleases, and eGFP expression was induced with doxycycline, as in Example 11. Cells were also transfected with either the pCR(R)4-TOPO-GFPdonor5 plasmid (Figure 28) containing a 734 bp eGFP insert (Figure 29) as in Example 11, or a similar plasmid containing a 1527 bp sequence insert (Figure 32) homologous to the mutated chromosomal eGFP gene. Additionally, the effect of G2 arrest with nocodazole on recombination frequency was assessed.

In a second experiment, donor lengths of 0.7, 1.08 and 1.5 kbp were compared. T18 cells were transfected with 50 ng of the 287-FokI and 296-FokI expression plasmids (Example 7, Table 12) and 500ng of a 0.7 kbp , 1.08 kbp, or 1.5 kbp donors, as described in Example 11. Four days after transfection, cells were assayed for correction of the defective eGFP gene by FACS, monitoring GFP fluorescence.

The results of these two experiments, shown in Table 16, show that longer donor sequence increases the frequency of targeted recombination (and, hence, of gene correction) and confirm that arrest of cells in the G2 phase of the cell cycle also increases the frequency of targeted recombination.

**Table 16: Effect of donor length and cell-cycle arrest on targeted recombination frequency**

| Donor length (kb) | Experiment 1 Nocodazole concentration: | | Experiment 2 |
|---|---|---|---|
| | 0 ng/ml | 100 ng/ml | - |
| 0.7 | 1.41 | 5.84 | 1.2 |
| 1.08 | not done | not done | 2.2 |
| 1.5 | 2.16 | 8.38 | 2.3 |

**Note:** Numbers represent percentage of total fluorescence in Region E of the FACS trace (see Example 11) which is an indication of the fraction of cells that have undergone targeted recombination to correct the defective chromosomal eGFP gene.

**Example 14: Editing of the endogenous human IL-2Rγ gene by targeted cleavage and recombination using zinc finger nucleases**

Two expression vectors, each encoding a ZFP-nuclease targeted to the human IL-2Rγ gene, were constructed. Each ZFP-nuclease contained a zinc finger protein-based DNA binding domain (see Table 17) fused to the nuclease domain of the type IIS restriction enzyme *Fok*I (amino acids 384-579 of the sequence of Wah et al. (1998) Proc. Natl. Acad. Sci. USA 95:10564-10569) via a four amino acid ZC linker (see Example 4). The nucleases were designed to bind to positions in exon 5 of the chromosomal IL-2Rγ gene surrounding codons 228 and 229 (a mutational hotspot in the gene) and to introduce a double-strand break in the DNA between their binding sites.

**Table 17: Zinc Finger Designs for exon 5 of the IL2Rγ Gene**

| Target sequence | F1 | F2 | F3 | F4 |
|---|---|---|---|---|
| ACTCTGTGGAAG (SEQ ID NO:152) 5-8G | RSDNLSV (SEQ ID NO:153) | RNAHRIN (SEQ ID NO:154) | RSDTLSE (SEQ ID NO:155) | ARSTRTN (SEQ ID NO:156) |
| AAAGCGGCTCCG (SEQ ID NO:157) 5-9D | RSDTLSE (SEQ ID NO:158) | ARSTRTT (SEQ ID NO:159) | RSDSLSK (SEQ ID NO:160) | QRSNLKV (SEQ ID NO:161) |
| Note: The zinc finger amino acid sequences shown above (in one-letter code) represent residues -1 through +6, with respect to the start of the alpha-helical portion of each zinc finger. Finger F1 is closest to the amino terminus of the protein. | | | | |

The complete DNA-binding portion of each of the chimeric endonucleases was as follows:

Nuclease targeted to ACTCTGTGGAAG (SEQ ID NO:152)

MAERPFQCRICMRNFSRSDNLSVHIRTHTGEKPFACDICGRKFARNAHRIN HTKIHTGSQKPFQCRICMRNFSRSDTLSEHIRTHTGEKPFACDICGRKFAARSTRT NHTKIHLRGS     (SEQ ID NO:162)

Nuclease targeted to AAAGCGGCTCCG (SEQ ID NO:157)

MAERPFQCRICMRNFSRSDTLSEHIRTHTGEKPFACDICGRKFAARSTRTTHTKIH TGSQKPFQCRICMRNFSRSDSLSKHIRTHTGEKPFACDICGRKFAQRSNLKVHTKI HLRGS     (SEQ ID NO:163)

Human embryonic kidney 293 cells were transfected (Lipofectamine 2000; Invitrogen) with two expression constructs, each encoding one of the ZFP-nucleases described in the preceding paragraph. The cells were also transfected with a donor construct carrying as an insert a 1,543 bp fragment of the IL2Rγ locus corresponding to positions 69195166-69196708 of the "minus" strand of the X chromosome (UCSC human genome release July 2003), in the pCR4Blunt Topo (Invitrogen) vector. The IL-2Rγ insert sequence contained the following two point mutations in the sequence of exon 5 (underlined):

F R V R S R F N P L C G S (SEQ ID NO:164)

TTTCGTGTTCGGAGCCGGTTTAACCCGCTCTGTGGAAGT (SEQ ID NO:165)

The first mutation (CGC→CGG) does not change the amino acid sequence (upper line) and serves to adversely affect the ability of the ZFP-nuclease to bind to the donor DNA, and to chromosomal DNA following recombination. The second mutation (CCA→CCG) does not change the amino acid sequence and creates a recognition site for the restriction enzyme *Bsr*BI.

Either 50 or 100 nanograms of each ZFP-nuclease expression construct and 0.5 or 1 microgram of the donor construct were used in duplicate transfections. The following control experiments were also performed: transfection with an expression plasmid encoding the eGFP protein; transfection with donor construct only; and transfection with plasmids expressing the ZFP nucleases only. Twenty four hours after transfection, vinblastine (Sigma) was added to 0.2 $\mu$M final concentration to one sample in each set of duplicates, while the other remained untreated. Vinblastine affects the cell's ability to assemble the mitotic spindle and therefore acts as a potent $G_2$ arresting agent. This treatment was performed to enhance the frequency of targeting because the homology-directed double-stranded break repair pathway is more active than non-homologous end-joining in the $G_2$ phase of the cell cycle. Following a 48 hr period of treatment with 0.2 $\mu$M vinblastine, growth medium was replaced, and the cells were allowed to recover from vinblastine treatment for an additional 24 hours. Genomic DNA was then isolated from all cell samples using the DNEasy Tissue Kit (Qiagen). Five hundred nanograms of genomic DNA from each sample was then assayed for frequency of gene targeting, by testing for the presence of a new *Bsr*BI site in the chromosomal IL-2R$\gamma$ locus, using the assay described schematically in Figure 33.

In brief, 20 cycles of PCR were performed using the primers shown in Table 18, each of which hybridizes to the chromosomal IL-2R$\gamma$ locus immediately outside of the region homologous to the 1.5 kb donor sequence. Twenty microcuries each of $\alpha$-$^{32}$P-dCTP and $\alpha$-$^{32}$P-dATP were included in each PCR reaction to allow detection of PCR products. The PCR reactions were desalted on a G-50 column (Amersham), and digested for 1 hour with 10 units of *Bsr*BI (New England Biolabs). The digestion products were resolved on a 10% non-denaturing polyacrylamide gel (BioRad), and the gel was dried and autoradiographed (Figure 34). In addition to the major PCR product, corresponding to the 1.55 kb amplifed fragment of the IL2R$\gamma$ locus ("wt" in Figure 34), an additional band ("rflp" in Figure 34) was observed in lanes corresponding to samples from cells that were transfected with the donor DNA construct and both ZFP-nuclease constructs. This additional band did not appear in any of the control lanes, indicating that ZFP nuclease-facilitated recombination of the *BsrBI* RFLP-containing donor sequence into the chromosome occurred in this experiment.

Additional experiments, in which trace amounts of a RFLP-containing IL-2R$\gamma$ DNA sequence was added to human genomic DNA (containing the wild-type IL-2R$\gamma$ gene), and the resultant mixture was amplified and subjected to digestion with a restriction enzyme which cleaves at the RFLP, have indicated that as little as 0.5% RFLP-containing sequence can be detected quantitatively using this assay.

**Table 18: Oligonucleotides for analysis of the human IL-2R$\gamma$ gene**

| Oligonucleotide | Sequence |
|---|---|
| Ex5_1.5detF1 | GATTCAACCAGACAGATAGAAGG (SEQ ID NO:166) |
| Ex5_1.5detR1 | TTACTGTCTCATCCTTTACTCC (SEQ ID NO:167) |

**Example 15: Targeted recombination at the IL-2R$\gamma$ locus in K562 cells**

K562 is a cell line derived from a human chronic myelogenous leukemia. The proteins used for targeted cleavage were *Fok*I fusions to the 5-8G and 5-9D zinc finger DNA-binding domains (Example 14, Table 17). The donor sequence was the 1.5 kbp fragment of the human IL-2R$\gamma$ gene containing a *BsrBI* site introduced by mutation, described in Example 14.

K562 cells were cultured in RPMI Medium 1640 (Invitrogen), supplemented with 10% fetal bovine serum (FBS) (Hyclone) and 2 mM L-glutamine. All cells were maintained at 37°C in an atmosphere of 5% $CO_2$. These cells were transfected by Nucleofection™ (Solution V, Program T16) (Amaxa Biosystems), according to the manufacturers' protocol, transfecting 2 million cells per sample. DNAs for transfection, used in various combinations as described below, were a plasmid encoding the 5-8G ZFP-*Fok*I fusion endonuclease, a plasmid encoding the 5-9D ZFP-*Fok*I fusion endonuclease, a plasmid containing the donor sequence (described above and in Example 14) and the peGFP-N1 vector (BD Biosciences) used as a control.

In the first experiment, cells were transfected with various plasmids or combinations of plasmids as shown in Table 19.

**Table 19**

| Sample # | p-eGFP-N1 | p5-8G | p5-9D | donor | vinblastine |
|---|---|---|---|---|---|
| 1 | 5 $\mu$g | - | - | - | - |
| 2 | - | - | - | 50 $\mu$g | - |
| 3 | - | - | - | 50 $\mu$g | yes |
| 4 | - | 10 $\mu$g | 10 $\mu$g | - | - |

(continued)

| Sample # | p-eGFP-N1 | p5-8G | p5-9D | donor | vinblastine |
|---|---|---|---|---|---|
| 5 | - | 5 μg | 5 μg | 25 μg | - |
| 6 | - | 5 μg | 5 μg | 25 μg | yes |
| 7 | - | 7.5 μg | 7.5 μg | 25 μg | - |
| 8 | - | 7.5 μg | 7.5 μg | 25 μg | yes |
| 9 | - | 7.5 μg | 7.5 μg | 50 μg | - |
| 10 | - | 7.5 μg | 7.5 μg | 50 μg | yes |

Vinblastine-treated cells were exposed to 0.2 uM vinblastine at 24 hours after transfection for 30 hours. The cells were collected, washed twice with PBS, and re-plated in growth medium. Cells were harvested 4 days after transfection for analysis of genomic DNA.

Genomic DNA was extracted from the cells using the DNEasy kit (Qiagen). One hundred nanograms of genomic DNA from each sample were used in a PCR reaction with the following primers:

Exon 5 forward: GCTAAGGCCAAGAAAGTAGGGCTAAAG (SEQ ID NO:168)
Exon 5 reverse: TTCCTTCCATCACCAAACCCTCTTG (SEQ ID NO:169)

These primers amplify a 1,669 bp fragment of the X chromosome corresponding to positions 69195100-69196768 on the "-" strand (UCSC human genome release July 2003) that contain exon 5 of the IL2Rγ gene. Amplification of genomic DNA which has undergone homologous recombination with the donor DNA yields a product containing a *BsrBI* site; whereas the amplification product of genomic DNA which has not undergone homologous recombination with donor DNA will not contain this restriction site.

Ten microcuries each of $\alpha$-$^{32}$PdCTP and $\alpha$-$^{32}$PdATP were included in each amplification reaction to allow visualization of reaction products. Following 20 cycles of PCR, the reaction was desalted on a Sephadex G-50 column (Pharmacia), and digested with 10 Units of *Bsr*BI (New England Biolabs) for 1 hour at 37°C. The reaction was then resolved on a 10% non-denaturing PAGE, dried, and exposed to a PhosphorImager screen.

The results of this experiment are shown in Figure 35. When cells were transfected with the control GFP plasmid, donor plasmid alone or the two ZFP-encoding plasmids in the absence of donor, no BsrBI site was present in the amplification product, as indicated by the absence of the band marked "rflp" in the lanes corresponding to these samples in Figure 35. However, genomic DNA of cells that were transfected with the donor plasmid and both ZFP-encoding plasmids contained the *Bsr*BI site introduced by homologous recombination with the donor DNA (band labeled "rflp"). Quantitation of the percentage of signal represented by the RFLP-containing DNA, shown in Figure 35, indicated that, under optimal conditions, up to 18% of all IL-2Rγ genes in the transfected cell population were altered by homologous recombination.

A second experiment was conducted according to the protocol just described, except that the cells were expanded for 10 days after transfection. DNAs used for transfection are shown in Table 20.

**Table 20**

| Sample # | p-eGFP-N1 | p5-8G | p5-9D | donor | vinblastine |
|---|---|---|---|---|---|
| 1 | 50 μg | - | - | - | - |
| 2 | - | - | - | 50 μg | - |
| 3 | - | - | - | 50 μg | yes |
| 4 | - | 7.5 μg | 7.5 μg | - | - |
| 5 | - | 5 μg | 5 μg | 25 μg | - |
| 6 | - | 5 μg | 5 μg | 25 μg | yes |
| 7 | - | 7.5 μg | 7.5 μg | 50 μg | - |
| 8 | - | 7.5 μg | 7.5 μg | 50 μg | yes |

Analysis of *Bsr*BI digestion of amplified DNA, shown in Figure 36, again demonstrated that up to 18% of IL-2Rγ genes had undergone sequence alteration through homologous recombination, after multiple rounds of cell division. Thus, the targeted recombination events are stable.

In addition, DNA from transfected cells in this second experiment was analyzed by Southern blotting. For this analysis, twelve micrograms of genomic DNA from each sample were digested with 100 units *Eco*RI, 50 units *Bsr*BI, and 40 units of *Dpn*I (all from New England Biolabs) for 12 hours at 37°C. This digestion generates a 7.7 kbp *Eco* RI fragment from the native IL-2Rγ gene (lacking a *Bsr*BI site) and fragments of 6.7 and 1.0 kbp from a chromosomal IL-2Rγ gene whose sequence has been altered, by homologous recombination, to include the *Bsr*BI site. *Dpn*I, a methylation-dependent restriction enzyme, was included to destroy the *dam*-methylated donor DNA. Unmethylated K562 cell genomic DNA is resistant to *Dpn*I digestion.

Following digestion, genomic DNA was purified by phenol-chloroform extraction and ethanol precipitation, resuspended in TE buffer, and resolved on a 0.8% agarose gel along with a sample of genomic DNA digested with EcoRI and SphI to generate a size marker. The gel was processed for alkaline transfer following standard procedure and DNA was transferred to a nylon membrane (Schleicher and Schuell). Hybridization to the blot was then performed by using a radiolabelled fragment of the IL-2Rγ locus corresponding to positions 69198428-69198769 of the "-" strand of the X chromosome (UCSC human genome July 2003 release). This region of the gene is outside of the region homologous to donor DNA. After hybridization, the membrane was exposed to a PhosphorImager plate and the data quantitated using Molecular Dynamics software. Alteration of the chromosomal IL-2Rγ sequence was measured by analyzing the intensity of the band corresponding to the *Eco*RI-*Bsr*BI fragment (arrow next to autoradiograph; *Bsr*BI site indicated by filled triangle in the map above the autoradiograph).

The results, shown in Figure 37, indicate up to 15% of chromosomal IL-2Rγ sequences were altered by homologous recombination, thereby confirming the results obtained by PCR analysis that the targeted recombination event was stable through multiple rounds of cell division. The Southern blot results also indicate that the results shown in Figure 36 do not result from an amplification artifact.

**Example 16: Targeted recombination at the IL-2Rγ locus in CD34-positive hematopoietic stem cells**

Genetic diseases (*e.g.*, severe combined immune deficiency (SCID) and sickle cell anemia) can be treated by homologous recombination-mediated correction of the specific DNA sequence alteration responsible for the disease. In certain cases, maximal efficiency and stability of treatment would result from correction of the genetic defect in a pluripotent cell. To this end, this example demonstrates alteration of the sequence of the IL-2Rγ gene in human CD34-positive bone marrow cells. CD34⁺ cells are pluripotential hematopoietic stem cells which give rise to the erythroid, myeloid and lymphoid lineages.

Bone marrow-derived human CD34 cells were purchased from AllCells, LLC and shipped as frozen stocks. These cells were thawed and allowed to stand for 2 hours at 37°C in an atmosphere of 5% $CO_2$ in RPMI Medium 1640 (Invitrogen), supplemented with 10% fetal bovine serum (FBS) (Hyclone) and 2 mM L-glutamine. Cell samples ($1 \times 10^6$ or $2 \times 10^6$ cells) were transfected by Nucleofection™ (amaxa biosystems) using the Human CD34 Cell Nucleofector™ Kit, according to the manufacturers' protocol. After transfection, cells were cultured in RPMI Medium 1640 (Invitrogen), supplemented with 10% FBS, 2 mM L-glutamine, 100ng/ml granulocyte-colony stimulating factor (G-CSF), 100ng/ml stem cell factor (SCF), 100ng/ml thrombopoietin (TPO), 50ng/ml Flt3 Ligand, and 20ng/ml Interleukin-6 (IL-6). The caspase inhibitor zVAD-FMK (Sigma-Aldrich) was added to a final concentration of 40 uM in the growth medium immediately after transfection to block apoptosis. Additional caspase inhibitor was added 48 hours later to a final concentration of 20 uM to further prevent apoptosis. These cells were maintained at 37°C in an atmosphere of 5% $CO_2$ and were harvested 3 days post-transfection.

Cell numbers and DNAs used for transfection are shown in Table 21.

**Table 21**

| Sample | # cells | p-eGFP-N1[1] | Donor[2] | p5-8G[3] | p5-9D[3] |
|---|---|---|---|---|---|
| 1 | $1 \times 10^6$ | 5 ug | - | - | - |
| 2 | $2 \times 10^6$ | - | 50 ug | - | - |
| 3 | $2 \times 10^6$ | - | 50 ug | 7.5 ug | 7.5 ug |

1. This is a control plasmid encoding an enhanced green fluorescent protein.
2. The donor DNA is a 1.5 kbp fragment containing sequences from exon 5 of the IL-2Rγ gene with an introduced *Bsr*BI site (see Example 14).
3. These are plasmids encoding *Fok*I fusions with the 5-8 G and 5-9D zinc finger DNA binding domains (see Table 17).

Genomic DNA was extracted from the cells using the MasterPure DNA Purification Kit (Epicentre). Due to the presence of glycogen in the precipitate, accurate quantitation of this DNA used as input in the PCR reaction is impossible; estimates using analysis of ethidium bromide-stained agarose gels indicate that ca. 50 ng genomic DNA was used in each sample. Thirty cycles of PCR were then performed using the following primers, each of which hybridizes to the chromosomal IL-2R$\gamma$ locus immediately outside of the region homologous to the 1.5 kb donor:

> ex5_1.5detF3 GCTAAGGCCAAGAAAGTAGGGCTAAAG (SEQ ID NO:170)
> ex5_1.5detR3 TTCCTTCCATCACCAAACCCTCTTG (SEQ ID NO:171)

Twenty microcuries each of $\alpha$-$^{32}$PdCTP and $\alpha$-$^{32}$PdATP were included in each PCR reaction to allow detection of PCR products. To provide an in-gel quantitation reference, the existence of a spontaneously occurring SNP in exon 5 of the IL-2Rgamma gene in Jurkat cells was exploited: this SNP creates a RFLP by destroying a MaeII site that is present in normal human DNA. A reference standard was therefore created by adding 1 or 10 nanograms of normal human genomic DNA (obtained from Clontech, Palo Alto, CA) to 100 or 90 ng of Jurkat genomic DNA, respectively, and performing the PCR as described above. The PCR reactions were desalted on a G-50 column (Amersham), and digested for 1 hour with restriction enzyme: experimental samples were digested with 10 units of *Bsr*BI (New England Biolabs); the "reference standard" reactions were digested with *Mae*II. The digestion products were resolved on a 10% non-denaturing PAGE (BioRad), the gel dried and analyzed by exposure to a PhosphorImager plate (Molecular Dynamics).

The results are shown in Figure 38. In addition to the major PCR product, corresponding to the 1.6 kb fragment of the IL2R$\gamma$ locus ("wt" in the right-hand panel of Figure 38), an additional band (labeled "rflp") was observed in lanes corresponding to samples from cells that were transfected with plasmids encoding both ZFP-nucleases and the donor DNA construct. This additional band did not appear in the control lanes, consistent with the idea that ZFP-nuclease assisted gene targeting of exon 5 of the common gamma chain gene occurred in this experiment.

Although accurate quantitation of the targeting rate is complicated by the proximity of the RFLP band to the wild-type band; the targeting frequency was estimated, by comparison to the reference standard (left panel), to be between 1-5%.

### Example 17: Donor-target homology effects

The effect, on frequency of homologous recombination, of the degree of homology between donor DNA and the chromosomal sequence with which it recombines was examined in T18 cell line, described in Example 9. This line contains a chromosomally integrated defective eGFP gene, and the donor DNA contains sequence changes, with respect to the chromosomal gene, that correct the defect.

Accordingly, the donor sequence described in Example 10 was modified, by PCR mutagenesis, to generate a series of ~700 bp donor constructs with different degrees of non-homology to the target. All of the modified donors contained sequence changes that corrected the defect in the chromosomal eGFP gene and contained additional silent mutations (DNA mutations that do not change the sequence of the encoded protein) inserted into the coding region surrounding the cleavage site. These silent mutations were intended to prevent the binding to, and cleavage of, the donor sequence by the zinc finger-cleavage domain fusions, thereby reducing competition between the intended chromosomal target and the donor plasmid for binding by the chimeric nucleases. In addition, following homologous recombination, the ability of the chimeric nucleases to bind and re-cleave the newly-inserted chromosomal sequences (and possibly stimulating another round of recombination, or causing non-homologous end joining or other double-strand break-driven alterations of the genome) would be minimized.

Four different donor sequences were tested. Donor 1 contains 8 mismatches with respect to the chromosomal defective eGFP target sequence, Donor 2 has 10 mismatches, Donor 3 has 6 mismatches, and Donor 5 has 4 mismatches. Note that the sequence of donor 5 is identical to wild-type eGFP sequence, but contains 4 mismatches with respect to the defective chromosomal eGFP sequence in the T18 cell line. Table 22 provides the sequence of each donor between nucleotides 201-242. Nucleotides that are divergent from the sequence of the defective eGFP gene integrated into the genome of the T18 cell line are shown in bold and underlined. The corresponding sequences of the defective chromosomal eGFP gene (GFP mut) and the normal eGFP gene (GFP wt) are also shown.

**Table 22**

| Donor | Sequence | SEQ ID NO. |
|-------|----------|------------|
| Donor 1 | CTTCAGCCGCTA**T**CC**AG**A**C**CAC**AT**GAAA**C**AA**A**CACGACTTCTT | 172 |
| Donor2 | CTTCAGCCG**G**TA**T**CC**AG**A**C**CAC**AT**GAAA**C**AA**C**A**T**GACTTCTT | 173 |
| Donor3 | CTTCAGCCGCTACCC**AG**A**C**CAC**AT**GAAA**A**CAGCACGACTTCTT | 174 |

(continued)

| Donor | Sequence | SEQ ID NO. |
|---|---|---|
| Donor 5 | CTTCAGCCGCTACCCC**GA<u>C</u>**CAC**AT**GAAGCAGCACGACTTCTT | 175 |
| GFP mut | CTTCAGCCGCTACCCCTAACAC--GAAGCAGCACGACTTCTT | 176 |
| GFP wt | CTTCAGCCGCTACCCC**GA<u>C</u>**CAC**AT**GAAGCAGCACGACTTCTT | 177 |

The T18 cell line was transfected, as described in Example 11, with 50 ng of the 287-*Fok*I and 296-*Fok*I expression constructs (Example 7 and Table 12) and 500 ng of each donor construct. FACS analysis was conducted as described in Example 11.

The results, shown in Table 23, indicate that a decreasing degree of mismatch between donor and chromosomal target sequence (*i.e.,* increased homology) results in an increased frequency of homologous recombination as assessed by restoration of GFP function.

Table 23[1]

| Donor | # mismatches | Percent cells with corrected eGFP gene[2] |
|---|---|---|
| Donor 2 | 10 | 0.45% |
| Donor 1 | 8 | 0.53% |
| Donor 3 | 6 | 0.89% |
| Donor 5 | 4 | 1.56% |

1: T18 cells, containing a defective chromosomal eGFP gene, were transfected with plasmids encoding two ZFP nucleases and with donor plasmids encoding a nondefective eGFP sequence having different numbers of sequence mismatches with the chromosomal target sequence. Expression of the chromosomal eGFP gene was induced with doxycycline and FACS analysis was conducted 5 days after transfection.
2: The number is the percent of total fluorescence exhibiting high emission at 525 nm and low emission at 570 nm (region E of the FACS trace).

The foregoing results show that levels of homologous recombination are increased by decreasing the degree of target-donor sequence divergence. Without wishing to be bound by any particular theory or to propose a particular mechanism, it is noted that greater homology between donor and target could facilitate homologous recombination by increasing the efficiency by which the cellular homologous recombination machinery recognizes the donor molecule as a suitable template. Alternatively, an increase in donor homology to the target could also lead to cleavage of the donor by the chimeric ZFP nucleases. A cleaved donor could help facilitate homologous recombination by increasing the rate of strand invasion or could aid in the recognition of the cleaved donor end as a homologous stretch of DNA during homology search by the homologous recombination machinery. Moreover, these possibilities are not mutually exclusive.

**Example 18: Preparation of siRNA**

To test whether decreasing the cellular levels of proteins involved in non-homologous end joining (NHEJ) facilitates targeted homologous recombination, an experiment in which levels of the Ku70 protein were decreased through siRNA inhibition was conducted. siRNA molecules targeted to the Ku70 gene were generated by transcription of Ku70 cDNA followed by cleavage of double-stranded transcript with Dicer enzyme.

Briefly, a cDNA pool generated from 293 and U2OS cells was used in five separate amplification reactions, each using a different set of amplification primers specific to the Ku70 gene, to generate five pools of cDNA fragments (pools A-E), ranging in size from 500-750 bp. Fragments in each of these five pools were then reamplified using primers containing the bacteriophage T7 RNA polymerase promoter element, again using a different set of primers for each cDNA pool. cDNA generation and PCR reactions were performed using the Superscript Choice cDNA system and Platinum Taq High Fidelity Polymerase (both from Invitrogen, Carlsbad, CA), according to manufacturers protocols and recommendations.

Each of the amplified DNA pools was then transcribed *in vitro* with bacteriophage T7 RNA polymerase to generate five pools (A-E) of double stranded RNA (dsRNA), using the RNAMAXX *in vitro* transcription kit (Stratagene, San Diego, CA) according to the manufacturer's instructions. After precipitation with ethanol, the RNA in each of the pools was resuspended and cleaved *in vitro* using recombinant Dicer enzyme (Stratagene, San Diego, CA) according to the manufacturer's instructions. 21-23 bp siRNA products in each of the five pools were purified by a two-step method, first using a Microspin G-25 column (Amershan), followed by a Microcon YM-100 column (Amicon). Each pool of siRNA products was transiently transfected into the T7 cell line using Lipofectamone 2000®.

Western blots to assay the relative effectiveness of the siRNA pools in suppressing Ku70 expression were performed approximately 3 days post-transfection. Briefly, cells were lysed and disrupted using RIPA buffer (Santa Cruz Biotechnology), and homogenized by passing the lysates through a QIAshredder (Qiagen, Valencia, CA). The clarified lysates were then treated with SDS PAGE sample buffer (with $\beta$ mercaptoethanol used as the reducing agent) and boiled for 5 minutes. Samples were then resolved on a 4-12% gradient NUPAGE gel and transferred onto a PVDF membrane. The upper portion of the blot was exposed to an anti-Ku70 antibody (Santa Cruz sc-5309) and the lower portion exposed to an anti-TF IIB antibody (Santa Cruz sc-225, used as an input control). The blot was then exposed to horseradish peroxidase-conjugated goat anti-mouse secondary antibody and processed for electrochemiluminescent (ECL) detection using a kit from Pierce Chemical Co. according to the manufacturer's instructions.

Figure 39 shows representative results following transfection of two of the siRNA pools (pools D and E) into T7 cells. Transfection with 70 ng of siRNA E results in a significant decrease in Ku70 protein levels (Figure 39, lane 3).

## Example 19: Increasing the Frequency of Homologous Recombination by Inhibition of Expression of a Protein Involved in Non-Homologous End Joining

Repair of a double-stranded break in genomic DNA can proceed along two different cellular pathways; homologous recombination (HR) or non-homologous end joining (NHEJ). Ku70 is a protein involved in NHEJ, which binds to the free DNA ends resulting from a double-stranded break in genomic DNA. To test whether lowering the intracellular concentration of a protein involved in NHEJ increases the frequency of HR, small interfering RNAs (siRNAs), prepared as described in Example 18, were used to inhibit expression of Ku70 mRNA, thereby lowering levels of Ku70 protein, in cells co-transfected with donor DNA and with plasmids encoding chimeric nucleases.

For these experiments, the T7 cell line (see Example 9 and Figure 27) was used. These cells contain a chromosomally-integrated defective eGFP gene, but have been observed to exhibit lower levels of targeted homologous recombination than the T18 cell line used in Examples 11-13.

T7 cells were transfected, as described in Example 11, with either 70 or 140 ng of one of two pools of dicer product targeting Ku70 (*see* Example 18). Protein blot analysis was performed on extracts derived from the transfected cells to determine whether the treatment of cells with siRNA resulted in a decrease in the levels of the Ku70 protein (*see* previous Example). Figure 39 shows that levels of the Ku70 protein were reduced in cells that had been treated with 70 ng of siRNA from pool E.

Separate cell samples in the same experiment were co-transfected with 70 or 140 ng of siRNA (pool D or pool E) along with 50 ng each of the 287-FokI and 296-FokI expression constructs (Example 7 and Table 12) and 500 ng of the 1.5 kbp GFP donor (Example 13), to determine whether lowering Ku70 levels increased the frequency of homologous recombination. The experimental protocol is described in Table 24. Restoration of eGFP activity, due to homologous recombination, was assayed by FACS analysis as described in Example 11.

### Table 24

| Expt. # | Donor[1] | ZFNs[2] | SiRNA[3] | % correction[4] |
|---------|----------|---------|----------|-----------------|
| 1 | 500 ng | - | - | 0.05 |
| 2 | - | 50 ng each | - | 0.01 |
| 3 | 500 ng | 50 ng each | - | 0.79 |
| 4 | 500 ng | 50 ng each | 70 ng pool D | 0.68 |
| 5 | 500 ng | 50 ng each | 140 ng pool D | 0.59 |
| 6 | 500 ng | 50 ng each | 70 ng pool E | 1.25 |
| 7 | 500 ng | 50 ng each | 140 ng pool E | 0.92 |

**1.** A plasmid containing a 1.5 kbp sequence encoding a functional eGFP protein which is homologous to the chromosomally integrated defective eGFP gene
**2.** Plasmids encoding the eGFP-targeted 287 and 296 zinc finger protein/*Fok*I fusion endonucleases
**3.** See Example 18
**4.** Percent of total fluorescence exhibiting high emission at 525 nm and low emission at 570 nm (region E of the FACS trace, see Example 11).

The percent correction of the defective eGFP gene in the transfected T7 cells (indicative of the frequency of targeted homologous recombination) is shown in the rightmost column of Table 24. The highest frequency of targeted recombination is observed in Experiment 6, in which cells were transfected with donor DNA, plasmids encoding the two eGFP-targeted fusion nucleases and 70 ng of siRNA Pool E. Reference to Example 18 and Figure 39 indicates that 70 ng of Pool E siRNA significantly depressed Ku70 protein levels. Thus, methods that reduce cellular levels of proteins involved in NHEJ can be used as a means of facilitating homologous recombination.

**Example 20: Zinc finger-*Fok*I fusion nucleases targeted to the human β-globin gene**

A number of four-finger zinc finger DNA binding domains, targeted to the human β-globin gene, were designed and plasmids encoding each zinc finger domain, fused to a *Fok*I cleavage half-domain, were constructed. Each zinc finger domain contained four zinc fingers and recognized a 12 bp target site in the region of the human β-globin gene encoding the mutation responsible for Sickle Cell Anemia. The binding affinity of each of these proteins to its target sequence was assessed, and four proteins exhibiting strong binding (sca-r29b, sca-36a, sca-36b, and sca-36c) were used for construction of *Fok*I fusion endonucleases.

The target sites of the ZFP DNA binding domains, aligned with the sequence of the human β-globin gene, are shown below. The translational start codon (ATG) is in bold and underlined, as is the A-T substitution causing Sickle Cell Anemia.

```
sca-36a GAAGTCTGCCGT (SEQ ID NO:178)
sca-36b GAAGTCtGCCGTT (SEQ ID NO:179)
sca-36c GAAGTCtGCCGTT (SEQ ID NO:180)


CAAACAGACACC**ATG**GTGCATCTGACTCCTG**T**GGAGAAGTCTGCCGTTACTG
GTTTGTCTGTGGTACCACGTAGACTGAGGAC**A**CCTCTTCAGACGGCAATGAC   (SEQ ID NO:181)


sca-r29b ACGTAGaCTGAGG (SEQ ID NO:182)
```

Amino acid sequences of the recognition regions of the zinc fingers in these four proteins are shown in Table 25. The complete amino acid sequences of these zinc finger domains are shown in Figure 40. The sca-36a domain recognizes a target site having 12 contiguous nucleotides (shown in upper case above), while the other three domain recognize a thirteen nucleotide sequence consisting of two six-nucleotide target sites (shown in upper case) separated by a single nucleotide (shown in lower case). Accordingly, the sca-r29b, sca-36b and sca-36c domains contain a non-canonical inter-finger linker having the amino acid sequence TGGGGSQKP (SEQ ID NO:183) between the second and the third of their four fingers.

**Table 25**

| ZFP | F1 | F2 | F3 | F4 |
|---|---|---|---|---|
| sca-r29b | QSGDLTR (SEQ ID NO: 184) | TSANLSR (SEQ ID NO: 185) | DRSALSR (SEQ ID NO: 186) | QSGHLSR (SEQ ID NO: 187) |
| sca-36a | RSQTRKT (SEQ ID NO: 188) | QKRNRTK (SEQ ID NO: 189) | DRSALSR (SEQ ID NO: 190) | QSGNLAR (SEQ ID NO: 191) |
| sca-36b | TSGSLSR (SEQ ID NO: 192) | DRSDLSR (SEQ ID NO: 193) | DRSALSR (SEQ ID NO: 194) | QSGNLAR (SEQ ID NO: 195) |
| sca-36c | TSSSLSR (SEQ ID NO: 196) | DRSDLSR (SEQ ID NO: 197) | DRSALSR (SEQ ID NO: 198) | QSGNLAR (SEQ ID NO: 199) |

**Example 21: *In vitro* cleavage of a DNA target sequence by β-globin-targeted *ZFP*/*Fok*I fusion endonucleases**

Fusion proteins containing a *Fok*I cleavage half-domain and one the four ZFP DNA binding domains described in the previous example were tested for their ability to cleave DNA *in vitro* with the predicted sequence specificity. These ZFP domains were cloned into the pcDNA3.1 expression vector via KpnI and BamHI sites and fused in-frame to the *Fok*I cleavage domain *via* a 4 amino acid ZC linker, as described above. A DNA fragment containing 700 bp of the human β-globin gene was cloned from genomic DNA obtained from K562 cells. The isolation and sequence of this fragment was described in Example 3, *supra.*

To produce fusion endonucleases (ZFNs) for the *in vitro* assay, circular plasmids encoding *Fok*I fusions to sca-r29b, sca-36a, sca-36b, and sca-36c protein were incubated in an *in vitro* transcription/translation system. *See* Example 4. A total of 2 ul of the TNT reaction (2 ul of a single reaction when a single protein was being assayed or 1 ul of each reaction when a pair of proteins was being assayed) was added to 13 ul of the cleavage buffer mix and 3 ul of labeled probe (~1 ng/ul). The probe was end-labeled with $^{32}$P using polynucleotide kinase. This reaction was incubated for 1 hour at room temperature to allow binding of the ZFNs. Cleavage was stimulated by the addition of 8 ul of 8 mM MgCl$_2$, diluted in cleavage buffer, to a final concentration of approximately 2.5 mM. The cleavage reaction was incubated for 1 hour at 37°C and stopped by the addition of 11 ul of phenol/chloroform. The DNA was isolated by phenol/chloroform extraction and analyzed by gel electrophoresis, as described in Example 4. As a control, 3 ul of probe was analyzed on the gel to mark the migration of uncut DNA (labeled "U" in figure 41).

The results are shown in Figure 41. Incubation of the target DNA with any single zinc finger/*Fok*I fusion resulted in no change in size of the template DNA. However, the combination of the sca-r29b nuclease with either of the sca-36b or sca-36c nucleases resulted in cleavage of the target DNA, as evidenced by the presence of two shorter DNA fragments (rightmost two lanes of Figure 41).

**Example 22: ZFP/*Fok*I fusion endonucleases, targeted to the β-globin gene, tested in a chromosomal GFP reporter system**

A DNA fragment containing the human β-globin gene sequence targeted by the ZFNs described in Example 20 was synthesized and cloned into a *Spe*I site in an eGFP reporter gene thereby, disrupting eGFP expression. The fragment contained the following sequence, in which the nucleotide responsible for the sickle cell mutation is in bold and underlined):

CTAGACACCATGGTGCATCTGACTCCTG**T**GGAGAAGTCTGCCGTTACT GCCCTAG (SEQ ID NO:200)

This disrupted eGFP gene containing inserted β-globin sequences was cloned into pcDNA4/TO (Invitrogen, Carlsbad, CA) using the *Hind*III and *Not*I sites, and the resulting vector was transfected into HEK293 TRex cells (Invitrogen). Individual stable clones were isolated and grown up, and the clones were tested for targeted homologous recombination by transfecting each of the sca-36 proteins (sca-36a, sca-36b, sca-36c) paired with sca-29b (*See* Example 20 and Table 25 for sequences and binding sites of these chimeric nucleases). Cells were transfected with 50 ng of plasmid encoding each of the ZFNs and with 500 ng of the 1.5-kb GFP Donor (Example 13). Five days after transfection, cells were tested for homologous recombination at the inserted defective eGFP locus. Initially, cells were examined by fluorescence microscopy for eGFP function. Cells exhibiting fluorescence were then analyzed quantitatively using a FACS assay for eGFP fluorescence, as described in Example 11.

The results showed that all cell lines transfected with sca-29b and sca-36a were negative for eGFP function, when assayed by fluorescence microscopy. Some of the lines transfected with sca-29b paired with either sca-36b or sca-36c were positive for eGFP expression, when assayed by fluorescence microscopy, and were therefore further analyzed by FACS analysis. The results of FACS analysis of two of these lines are shown in Table 26, and indicate that zinc finger nucleases targeted to β-globin sequences are capable of catalyzing sequence-specific double-stranded DNA cleavage to facilitate homologous recombination in living cells.

**Table 26**

| Cell line | DNA transfected: | | | | % corr.[1] |
|-----------|---------|---------|---------|---------|------------|
|           | sca-29b | sca-36a | sca-36b | sca-36c |            |
| #20       | +       | +       |         |         | 0          |
|           | +       |         | +       |         | 0.08       |
|           | +       |         |         | +       | 0.07       |
| #40       | +       | +       |         |         | 0          |
|           | +       |         | +       |         | 0.18       |
|           | +       |         |         | +       | 0.12       |

**1.** Percent of total fluorescence exhibiting high emission at 525 nm and low emission at 570 nm (region E of the FACS trace, see Example 11).

**Example 23: Effect of transcription level on targeted homologous recombination**

Since transcription of a chromosomal DNA sequence involves alterations in its chromatin structure (generally to make the transcribed sequences more accessible), it is possible that an actively transcribed gene might be a more favorable substrate for targeted homologous recombination. This idea was tested using the T18 cell line (Example 9) which contains chromosomal sequences encoding a defective eGFP gene whose transcription is under the control of a doxycycline-inducible promoter.

Separate samples of T18 cells were transfected with plasmids encoding the eGFP-targeted 287 and 296 zinc finger/*Fok*I fusion proteins (Example 7) and a 1.5 kbp donor DNA molecule containing sequences that correct the defect in the chromosomal eGFP gene (Example 9). Five hours after transfection, transfected cells were treated with different concentrations of doxycycline, then eGFP mRNA levels were measured 48 hours after addition of doxycycline. eGFP fluorescence at 520 nm (indicative of targeted recombination of the donor sequence into the chromosome to replace the inserted β-globin sequences) was measured by FACS at 4 days after transfection.

The results are shown in Figure 42. Increasing steady-state levels of eGFP mRNA normalized to GAPDH mRNA (equivalent, to a first approximation, to the rate of transcription of the defective chromosomal eGFP gene) are indicated by the bars. The number above each bar indicate the percent of cells exhibiting eGFP fluorescence. The results show that increasing transcription rate of the target gene is accompanied by higher frequencies of targeted recombination. This suggests that targeted activation of transcription (as disclosed, *e.g.* in co-owned U.S. Patents 6,534,261 and 6,607,882) can be used, in conjunction with targeted DNA cleavage, to stimulate targeted homologous recombination in cells.

**Example 24: Generation of a cell line containing a mutation in the IL-2Rγ gene**

K562 cells were transfected with plasmids encoding the 5-8GL0 and the 5-9DL0 zinc finger nucleases (ZFNs) (see Example 14; Table 17) and with a 1.5 kbp *Dra*I donor construct. The *Dra*I donor is comprised of a sequence with homology to the region encoding the 5th exon of the IL2Rγ gene, but inserts an extra base between the ZFN-binding sites to create a frameshift and generate a *Dra*I site.

24 hours post-transfection, cells were treated with 0.2 uM vinblastine (final concentration) for 30 hours. Cells were washed three times with PBS and re-plated in medium. Cells were allowed to recover for 3 days and an aliquot of cells were removed to perform a PCR-based RFLP assay, similar to that described in Example 14, testing for the presence of a *Dra*I site. It was determined the gene correction frequency within the population was approximately 4%.

Cells were allowed to recover for an additional 2 days and 1600 individual cells were plated into 40x 96-well plates in 100 ul of medium.

The cells are grown for about 3 weeks, and cells homozygous for the *Dra*I mutant phenotype are isolated. The cells are tested for genome modification (by testing for the presence of a *Dra*I site in exon 5 of the IL-2Rγ gene) and for levels of IL-2Rγ mRNA (by real-time PCR) and protein (by Western blotting) to determine the effect of the mutation on gene expression. Cells are tested for function by FACS analysis.

Cells containing the *Dra*I frameshift mutation in the IL-2Rγ gene are transfected with plasmids encoding the 5-8GL0 and 5-9DL0 fusion proteins and a 1.5 kb *Bsr*BI donor construct (Example 14) to replace the *Dra*I frameshift mutation with a sequence encoding a functional protein. Levels of homologous recombination greater than 1% are obtained in these cells, as measured by assaying for the presence of a *Bsr*BI site as described in Example 14. Recovery of gene function is demonstrated by measuring mRNA and protein levels and by FACS analysis.

**Claims**

1. A method for altering a first nucleotide sequence in a region of interest in cellular chromatin, the method comprising:

   (a) engineering a first zinc finger binding domain to bind to a second nucleotide sequence in the region of interest, wherein the second sequence comprises at least 9 nucleotides;
   (b) providing a second zinc finger binding domain to bind to a third nucleotide sequence, wherein the third sequence comprises at least 9 nucleotides ;
   (c) expressing a first fusion protein in the cell, the first fusion protein comprising the first zinc finger binding domain and a first *Fok*I cleavage half-domain;
   (d) expressing a second fusion protein in the cell, the second fusion protein comprising the second zinc finger binding domain and a second *Fok*I cleavage half-domain, wherein at least one of the fusion proteins comprises an alteration in the amino acid residue at one or more of positions 446, 447, 479, 483, 484, 486, 487, 490, 491, 496, 498, 499, 500, 531, 534, 537 and 538 of the dimerization interface of the cleavage half-domain that inhibits homodimerization of the cleavage half-domain; and

(e) contacting the cell with a polynucleotide comprising a fourth nucleotide sequence, wherein the fourth nucleotide sequence is homologous but non-identical with the first nucleotide sequence;

wherein binding of the first fusion protein to the second sequence and binding of the second fusion protein to the third sequence positions the cleavage half-domains such that the cellular chromatin is cleaved in the region of interest, thereby facilitating homologous recombination between the first nucleotide sequence and the fourth nucleotide sequence, resulting in alteration of the first nucleotide sequence.

2. The method of claim 1, wherein cleavage occurs between the second and third nucleotide sequences.

3. The method of claim 1, wherein the second zinc finger binding domain is engineered to bind to the third nucleotide sequence.

4. The method of claim 1, wherein the cellular chromatin is in a chromosome.

5. The method of claim 1, wherein the fourth nucleotide sequence contains sequences not present in the region of interest that are flanked by sequences homologous to the region of interest.

6. The method of claim 1, wherein the first nucleotide sequence comprises a mutation in a gene.

7. The method of claim 6, wherein the mutation is selected from the group consisting of at least one of a point mutation, a substitution, a deletion and an insertion.

8. The method of claim 6, wherein the fourth nucleotide sequence comprises the wild-type sequence of the gene.

9. The method of claim 6, wherein the cellular chromatin is cleaved at a site located within 100 nucleotides on either side of the mutation.

10. The method of claim 1, wherein, in step (e), the polynucleotide is linear.

11. The method of claim 1, wherein, in step (e), the polynucleotide is circular.

12. The method of claim 1, wherein, in step (e), the polynucleotide is double-stranded.

13. The method of claim 1, wherein, in step (e), the polynucleotide is single-stranded.

14. The method of claim 1, wherein, in step (e), the cell is contacted with a virus comprising the polynucleotide.

15. The method of claim 14, wherein the virus is an adenovirus or an adeno-associated virus.

16. The method of claim 1, wherein, in steps (c) and (d), the cell is contacted with two polynucleotides, the first polynucleotide encoding the first fusion protein and the second polynucleotide encoding the second fusion protein.

17. The method of claim 1, wherein, in steps (c) and (d), the cell is contacted with a polynucleotide encoding two fusion proteins, the first fusion protein comprising the first zinc finger binding domain and a first cleavage half-domain and the second fusion protein comprising the second zinc finger binding domain and a second cleavage half-domain.

18. The method of claim 1, wherein the cell is arrested in the G2 phase of the cell cycle.

19. The method of claim 1, wherein the near edges of the second and third nucleotide sequences are separated by 6 base pairs.

20. The method of claim 19, wherein, in at least one of the fusion proteins, the amino acid sequence between the zinc finger binding domain and the cleavage half-domain (Z-C linker) consists of 4 amino acid residues.

21. The method of claim 1, wherein the second and third nucleotide sequences are present in the polynucleotide comprising the fourth nucleotide sequence.

**22.** The method of claim 21, wherein the fourth polynucleotide sequence is cleaved.

**23.** The method of claim 1, wherein the first nucleotide sequence is converted to the fourth nucleotide sequence.

**24.** A cell comprising:

(a) a first nucleotide sequence in a region of interest in the cellular chromatin of the cell;
(b) a first fusion protein or a polynucleotide encoding the first fusion protein, wherein the first fusion protein comprises a first zinc finger binding domain and a first *FokI* cleavage half-domain, wherein the first zinc finger domain is engineered to bind to a second nucleotide sequence in the region of interest, wherein the second sequence comprises at least 9 nucleotides;
(c) a second fusion protein or a polynucleotide encoding the second fusion protein, wherein the second fusion protein comprises a second zinc finger binding domain and a second *FokI* cleavage half-domain, wherein the second zinc finger domain is capable of binding to a third nucleotide sequence, wherein the third sequence comprises at least 9 nucleotides,
wherein at least one of the fusion proteins comprises an alteration in the amino acid residue at one or more of positions 446, 447, 479, 483, 484, 486, 487, 490, 491, 496, 498, 499, 500, 531, 534, 537 and 538 of the dimerization interface of the cleavage half-domain that inhibits homodimerization of the cleavage half-domain; and
(d) a polynucleotide comprising a fourth nucleotide sequence, wherein the fourth nucleotide sequence is homologous but non-identical with the first nucleotide sequence; wherein binding of the first fusion protein to the second sequence and binding of the second fusion protein to the third sequence positions the cleavage half-domains such that the cellular chromatin is cleaved in the region of interest, thereby facilitating homologous recombination between the first nucleotide sequence and the fourth nucleotide sequence, resulting in alteration of the first nucleotide sequence.

**25.** A pharmaceutical composition for altering a first nucleotide sequence in a region of interest in cellular chromatin comprising:

(a) a first fusion protein or a polynucleotide encoding the first fusion protein, wherein the first fusion protein comprises a first zinc finger binding domain and a first *FokI* cleavage half-domain,
wherein the first zinc finger domain is engineered to bind to a second nucleotide sequence in the region of interest, wherein the second sequence comprises at least 9 nucleotides;
(b) a second fusion protein or a polynucleotide encoding the second fusion protein, wherein the second fusion protein comprises a second zinc finger binding domain and a second *FokI* cleavage half-domain;
wherein the second zinc finger domain is capable of binding to a third nucleotide sequence, wherein the third sequence comprises at least 9 nucleotides,
wherein at least one of the fusion proteins comprises an alteration in the amino acid residue at one or more of positions 446, 447, 479, 483, 484, 486, 487, 490, 491, 496, 498, 499, 500, 531, 534, 537 and 538 of the dimerization interface of the cleavage half-domain that inhibits homodimerization of the cleavage half-domain; and
(c) a polynucleotide comprising a fourth nucleotide sequence, wherein the fourth nucleotide sequence is homologous but non-identical with the first nucleotide sequence, wherein, in use, binding of the first fusion protein to the second sequence and binding of the second fusion protein to the third sequence positions the cleavage half-domains such that the cellular chromatin is cleaved in the region of interest, thereby facilitating homologous recombination between the first nucleotide sequence and the fourth nucleotide sequence, resulting in alteration of the first nucleotide sequence.

**26.** An in vitro method for inactivating a target gene by targeted deletion of a genomic sequence of the target gene, the method comprising cleaving the target gene at two cleavage sites such that the two sites are joined so as to delete the sequence between the two cleavage sites, thereby inactivating the target gene, wherein the method comprises expressing two fusion proteins in a cell, each fusion protein comprising a zinc finger binding domain and a *FokI* cleavage half-domain, wherein at least one of the fusion proteins comprises an alteration in the amino acid residue at one or more of positions 446, 447, 479, 483, 484, 486, 487, 490, 491, 496, 498, 499, 500, 531, 534, 537 and 538 of the dimerization interface of the cleavage half-domain that inhibits homodimerization of the cleavage half-domain, wherein binding of the fusion proteins to the target gene positions the cleavage half-domains such that a functional cleavage domain is reconstituted leading to cleavage of the target gene.

**Patentansprüche**

1. Verfahren zur Änderung einer ersten Nukleotidsequenz in einer Region von Interesse in zellulärem Chromatin, wobei das Verfahren Folgendes umfasst:

   (a) Gestalten einer ersten Zinkfingerbindungsdomäne zum Binden an eine zweite Nukleotidsequenz in der Region von Interesse, wobei die zweite Sequenz mindestens 9 Nukleotide umfasst;
   (b) Bereitstellen einer zweiten Zinkfingerbindungsdomäne zum Binden an eine dritte Nukleotidsequenz, wobei die dritte Sequenz mindestens 9 Nukleotide umfasst;
   (c) Exprimieren eines ersten Fusionsproteins in der Zelle, wobei das erste Fusionsprotein die erste Zinkfingerbindungsdomäne und eine erste *FokI*-Spaltungshalbdomäne umfasst;
   (d) Exprimieren eines zweiten Fusionsproteins in der Zelle, wobei das zweite Fusionsprotein die zweite Zinkfingerbindungsdomäne und eine zweite *FokI*-Spaltungshalbdomäne umfasst,wobei mindestens eines der Fusionsproteine eine Änderung in dem Aminosäurerest an einer oder mehreren der Positionen 446, 447, 479, 483, 484, 486, 487, 490, 491, 496, 498, 499, 500, 531, 534, 537 und 538 der Dimerisierungsschnittstelle der Spaltungshalbdomäne umfasst, welche die Homodimerisierung der Spaltungshalbdomäne hemmt; und
   (e) Kontaktieren der Zelle mit einem Polynukleotid, das eine vierte Nukleotidsequenz umfasst, wobei die vierte Nukleotidsequenz mit der ersten Nukleotidsequenz homolog, aber nicht identisch ist;

   wobei das Binden des ersten Fusionsproteins an die zweite Sequenz und das Binden des zweiten Fusionsproteins an die dritte Sequenz die Spaltungshalbdomänen derart positioniert, dass das zelluläre Chromatin in der Region von Interesse gespalten wird, wodurch die homologe Rekombination zwischen der ersten Nukleotidsequenz und der vierten Nukleotidsequenz vereinfacht wird, was zu einer Änderung der ersten Nukleotidsequenz führt.

2. Verfahren nach Anspruch 1, wobei die Spaltung zwischen der zweiten und dritten Nukleotidsequenz stattfindet.

3. Verfahren nach Anspruch 1, wobei die zweite Zinkfingerbindungsdomäne gestaltet ist, um an die dritte Nukleotidsequenz zu binden.

4. Verfahren nach Anspruch 1, wobei das zelluläre Chromatin in einem Chromosom ist.

5. Verfahren nach Anspruch 1, wobei die vierte Nukleotidsequenz in der Region von Interesse nicht enthaltene Sequenzen enthält, die von Sequenzen flankiert werden, die zu der Region von Interesse homolog sind.

6. Verfahren nach Anspruch 1, wobei die erste Nukleotidsequenz eine Mutation in einem Gen umfasst.

7. Verfahren nach Anspruch 6, wobei die Mutation aus der Gruppe ausgewählt ist, die aus mindestens einem von einer Punktmutation, einer Substitution, einer Löschung und einer Einfügung besteht.

8. Verfahren nach Anspruch 6, wobei die vierte Nukleotidsequenz die Wildtypsequenz des Gens umfasst.

9. Verfahren nach Anspruch 6, wobei das zelluläre Chromatin an einer Stelle gespalten ist, die sich innerhalb von 100 Nukleotiden an beiden Seiten der Mutation befindet.

10. Verfahren nach Anspruch 1, wobei in Schritt (e) das Polynukleotid linear ist.

11. Verfahren nach Anspruch 1, wobei in Schritt (e) das Polynukleotid kreisförmig ist.

12. Verfahren nach Anspruch 1, wobei in Schritt (e) das Polynukleotid doppelsträngig ist.

13. Verfahren nach Anspruch 1, wobei in Schritt (e) das Polynukleotid einzelsträngig ist.

14. Verfahren nach Anspruch 1, wobei in Schritt (e) die Zelle mit einem Virus kontaktiert wird, welches das Polynukleotid umfasst.

15. Verfahren nach Anspruch 14, wobei das Virus ein Adenovirus oder ein adenoassoziiertes Virus ist.

16. Verfahren nach Anspruch 1, wobei die Zelle in Schritt (c) und (d) mit zwei Polynukleotiden kontaktiert wird, wobei das erste Polynukleotid das erste Fusionsprotein codiert und das zweite Polynukleotid das zweite Fusionsprotein codiert.

17. Verfahren nach Anspruch 1, wobei die Zelle in Schritt (c) und (d) mit einem Polynukleotid kontaktiert wird, das zwei Fusionsproteine codiert, wobei das erste Fusionsprotein die erste Zinkfingerbindungsdomäne und eine erste Spaltungshalbdomäne umfasst und das zweite Fusionsprotein die zweite Zinkfingerbindungsdomäne und eine zweite Spaltungshalbdomäne umfasst.

18. Verfahren nach Anspruch 1, wobei die Zelle in der G2-Phase des Zellzyklus aufgehalten wird.

19. Verfahren nach Anspruch 1, wobei die nahen Ränder der zweiten und dritten Nukleotidsequenz durch 6 Basenpaare getrennt sind.

20. Verfahren nach Anspruch 19, wobei in mindestens einem der Fusionsproteine die Aminosäuresequenz zwischen der Zinkfingerbindungsdomäne und der Spaltungshalbdomäne (Z-/C-Linker) aus 4 Aminosäureresten besteht.

21. Verfahren nach Anspruch 1, wobei die zweite und dritte Nukleotidsequenz in dem Polynukleotid vorhanden sind, welches die vierte Nukleotidsequenz umfasst.

22. Verfahren nach Anspruch 21, wobei die vierte Polynukleotidsequenz gespalten ist.

23. Verfahren nach Anspruch 1, wobei die erste Nukleotidsequenz in die vierte Nukleotidsequenz umgewandelt wird.

24. Zelle, umfassend:

(a) eine erste Nukleotidsequenz in einer Region von Interesse in dem zellulären Chromatin der Zelle;
(b) ein erstes Fusionsprotein oder ein Polynukleotid, welches das erste Fusionsprotein codiert, wobei das erste Fusionsprotein eine erste Zinkfingerbindungsdomäne und eine erste *FokI*-Spaltungshalbdomäne umfasst, wobei die erste Zinkfingerdomäne gestaltet ist, um an eine zweite Nukleotidsequenz in der Region von Interesse zu binden, wobei die zweite Sequenz mindestens 9 Nukleotide umfasst;
(c) ein zweites Fusionsprotein oder ein Polynukleotid, welches das zweite Fusionsprotein codiert, wobei das zweite Fusionsprotein eine zweite Zinkfingerbindungsdomäne und eine zweite *FokI*-Spaltungshalbdomäne umfasst, wobei die zweite Zinkfingerdomäne dazu in der Lage ist, an eine dritte Nukleotidsequenz zu binden, wobei die dritte Sequenz mindestens 9 Nukleotide umfasst,
wobei mindestens eines der Fusionsproteine eine Änderung in dem Aminosäurerest an einer oder mehreren der Positionen 446, 447, 479, 483, 484, 486, 487, 490, 491, 496, 498, 499, 500, 531, 534, 537 und 538 der Dimerisierungsschnittstelle der Spaltungshalbdomäne umfasst, welche die Homodimerisierung der Spaltungshalbdomäne hemmt; und
(d) ein Polynukleotid, das eine vierte Nukleotidsequenz umfasst, wobei die vierte Nukleotidsequenz mit der ersten Nukleotidsequenz homolog, aber nicht identisch ist;

wobei das Binden des ersten Fusionsproteins an die zweite Sequenz und das Binden des zweiten Fusionsproteins an die dritte Sequenz die Spaltungshalbdomänen derart positioniert, dass das zelluläre Chromatin in der Region von Interesse gespalten wird, wodurch die homologe Rekombination zwischen der ersten Nukleotidsequenz und der vierten Nukleotidsequenz vereinfacht wird, was zu einer Änderung der ersten Nukleotidsequenz führt.

25. Pharmazeutische Zusammensetzung zur Änderung einer ersten Nukleotidsequenz in einer Region von Interesse in zellulärem Chromatin, umfassend:

(a) ein erstes Fusionsprotein oder ein Polynukleotid, welches das erste Fusionsprotein codiert, wobei das erste Fusionsprotein eine erste Zinkfingerbindungsdomäne und eine erste *FokI*-Spaltungshalbdomäne umfasst, wobei die erste Zinkfingerdomäne gestaltet ist, um an eine zweite Nukleotidsequenz in der Region von Interesse zu binden, wobei die zweite Sequenz mindestens 9 Nukleotide umfasst;
(b) ein zweites Fusionsprotein oder ein Polynukleotid, welches das zweite Fusionsprotein codiert, wobei das zweite Fusionsprotein eine zweite Zinkfingerbindungsdomäne und eine zweite *FokI*-Spaltungshalbdomäne umfasst;
wobei die zweite Zinkfingerdomäne dazu in der Lage ist, an eine dritte Nukleotidsequenz zu binden, wobei die dritte Sequenz mindestens 9 Nukleotide umfasst,

wobei mindestens eines der Fusionsproteine eine Änderung in dem Aminosäurerest an einer oder mehreren der Positionen 446, 447, 479, 483, 484, 486, 487, 490, 491, 496, 498, 499, 500, 531, 534, 537 und 538 der Dimerisierungsschnittstelle der Spaltungshalbdomäne umfasst, welche die Homodimerisierung der Spaltungshalbdomäne hemmt; und

(c) ein Polynukleotid, das eine vierte Nukleotidsequenz umfasst, wobei die vierte Nukleotidsequenz mit der ersten Nukleotidsequenz homolog, aber nicht identisch ist;

wobei in Verwendung das Binden des ersten Fusionsproteins an die zweite Sequenz und das Binden des zweiten Fusionsproteins an die dritte Sequenz die Spaltungshalbdomänen derart positioniert, dass das zelluläre Chromatin in der Region von Interesse gespalten wird, wodurch die homologe Rekombination zwischen der ersten Nukleotidsequenz und der vierten Nukleotidsequenz vereinfacht wird, was zu einer Änderung der ersten Nukleotidsequenz führt.

26. In-Vitro-Verfahren zur Inaktivierung eines Zielgens durch gezielte Löschung einer genomischen Sequenz des Zielgens, wobei das Verfahren die Spaltung des Zielgens an zwei Spaltungsstellen umfasst, derart, dass die zwei Stellen verbunden werden, um die Sequenz zwischen den zwei Spaltungsstellen zu löschen, wodurch das Zielgen inaktiviert wird,
wobei das Verfahren das Exprimieren von zwei Fusionsproteinen in einer Zelle umfasst, wobei jedes Fusionsprotein eine Zinkfingerbindungsdomäne und eine *FokI*-Spaltungshalbdomäne umfasst, wobei mindestens eines der Fusionsproteine eine Änderung in dem Aminosäurerest an einer oder mehreren der Positionen 446, 447, 479, 483, 484, 486, 487, 490, 491, 496, 498, 499, 500, 531, 534, 537 und 538 der Dimerisierungsschnittstelle der Spaltungshalbdomäne umfasst, welche die Homodimerisierung der Spaltungshalbdomäne hemmt, wobei das Binden der Fusionsproteine an das Zielgen die Spaltungshalbdomänen derart positioniert, dass eine funktionelle Spaltungsdomäne wiederhergestellt wird, was zur Spaltung des Zielgens führt.

## Revendications

1. Méthode pour modifier une première séquence nucléotidique dans une région d'intérêt dans la chromatine cellulaire, la méthode comprenant :

   (a) la modification d'un premier domaine de liaison à doigt de zinc pour se lier à une deuxième séquence nucléotidique dans la région d'intérêt, dans laquelle la deuxième séquence comprend au moins 9 nucléotides ;
   (b) la fourniture d'un second domaine de liaison à doigt de zinc pour se lier à une troisième séquence nucléotidique, dans laquelle la troisième séquence comprend au moins 9 nucléotides ;
   (c) l'expression d'une première protéine de fusion dans la cellule, la première protéine de fusion comprenant le premier domaine de liaison à doigt de zinc et un premier demi-domaine de clivage de *FokI* ;
   (d) l'expression d'une seconde protéine de fusion dans la cellule, la seconde protéine de fusion comprenant le second domaine de liaison à doigt de zinc et un second demi-domaine de clivage de *FokI,* dans laquelle au moins l'une des protéines de fusion comprend une modification du résidu d'acide aminé au niveau d'une ou de plusieurs des positions 446, 447, 479, 483, 484, 486, 487, 490, 491, 496, 498, 499, 500, 531, 534, 537 et 538 de l'interface de dimérisation du demi-domaine de clivage qui inhibe l'homodimérisation du demi-domaine de clivage ; et
   (e) la mise en contact de la cellule avec un polynucléotide comprenant une quatrième séquence nucléotidique, dans laquelle la quatrième séquence nucléotidique est homologue mais non identique à la première séquence nucléotidique ;

   dans laquelle la liaison de la première protéine de fusion à la deuxième séquence et la liaison de la seconde protéine de fusion à la troisième séquence positionnent les demi-domaines de clivage de sorte que la chromatine cellulaire est clivée dans la région d'intérêt, facilitant ainsi la recombinaison homologue entre la première séquence nucléotidique et la quatrième séquence nucléotidique, entraînant une modification de la première séquence nucléotidique.

2. Méthode selon la revendication 1, dans laquelle le clivage se produit entre les deuxième et troisième séquences nucléotidiques.

3. Méthode selon la revendication 1, dans laquelle le second domaine de liaison à doigt de zinc est modifié pour se lier à la troisième séquence nucléotidique.

**4.** Méthode selon la revendication 1, dans laquelle la chromatine cellulaire est dans un chromosome.

**5.** Méthode selon la revendication 1, dans laquelle la quatrième séquence nucléotidique contient des séquences non présentes dans la région d'intérêt qui sont flanquées de séquences homologues à la région d'intérêt.

**6.** Méthode selon la revendication 1, dans laquelle la première séquence nucléotidique comprend une mutation dans un gène.

**7.** Méthode selon la revendication 6, dans laquelle la mutation est choisie dans le groupe constitué d'au moins l'une parmi une mutation ponctuelle, une substitution, une délétion et une insertion.

**8.** Méthode selon la revendication 6, dans laquelle la quatrième séquence nucléotidique comprend la séquence de type sauvage du gène.

**9.** Méthode selon la revendication 6, dans laquelle la chromatine cellulaire est clivée au niveau d'un site situé à moins de 100 nucléotides de chaque côté de la mutation.

**10.** Méthode selon la revendication 1, dans laquelle, dans l'étape (e), le polynucléotide est linéaire.

**11.** Méthode selon la revendication 1, dans laquelle, dans l'étape (e), le polynucléotide est circulaire.

**12.** Méthode selon la revendication 1, dans laquelle, dans l'étape (e), le polynucléotide est double brin.

**13.** Méthode selon la revendication 1, dans laquelle, dans l'étape (e), le polynucléotide est monocaténaire.

**14.** Méthode selon la revendication 1, dans laquelle, dans l'étape (e), la cellule est mise en contact avec un virus comprenant le polynucléotide.

**15.** Méthode selon la revendication 14, dans laquelle le virus est un adénovirus ou un virus adéno-associé.

**16.** Méthode selon la revendication 1, dans laquelle, dans les étapes (c) et (d), la cellule est mise en contact avec deux polynucléotides, le premier polynucléotide codant pour la première protéine de fusion et le second polynucléotide codant pour la seconde protéine de fusion.

**17.** Méthode selon la revendication 1, dans laquelle, dans les étapes (c) et (d), la cellule est mise en contact avec un polynucléotide codant pour deux protéines de fusion, la première protéine de fusion comprenant le premier domaine de liaison à doigt de zinc et un premier demi-domaine de clivage et la seconde protéine de fusion comprenant le second domaine de liaison à doigt de zinc et un second demi-domaine de clivage.

**18.** Méthode selon la revendication 1, dans laquelle la cellule est arrêtée dans la phase G2 du cycle cellulaire.

**19.** Méthode selon la revendication 1, dans laquelle les bords proches des deuxième et troisième séquences nucléotidiques sont séparés par 6 paires de bases.

**20.** Méthode selon la revendication 19, dans laquelle, dans au moins l'une des protéines de fusion, la séquence d'acides aminés entre le domaine de liaison à doigt de zinc et le demi-domaine de clivage (lieur Z-C) est constituée de 4 résidus d'acides aminés.

**21.** Méthode selon la revendication 1, dans laquelle les deuxième et troisième séquences nucléotidiques sont présentes dans le polynucléotide comprenant la quatrième séquence nucléotidique.

**22.** Méthode selon la revendication 21, dans laquelle la quatrième séquence polynucléotidique est clivée.

**23.** Méthode selon la revendication 1, dans laquelle la première séquence nucléotidique est convertie en la quatrième séquence nucléotidique.

**24.** Cellule comprenant :

(a) une première séquence nucléotidique dans une région d'intérêt dans la chromatine cellulaire de la cellule ;

(b) une première protéine de fusion ou un polynucléotide codant pour la première protéine de fusion, dans laquelle la première protéine de fusion comprend un premier domaine de liaison à doigt de zinc et un premier demi-domaine de clivage de *FokI,* dans laquelle le premier domaine à doigt de zinc est modifié pour se lier à une deuxième séquence nucléotidique dans la région d'intérêt, dans laquelle la deuxième séquence comprend au moins 9 nucléotides ;

(c) une seconde protéine de fusion ou un polynucléotide codant pour la seconde protéine de fusion, dans laquelle la seconde protéine de fusion comprend un second domaine de liaison à doigt de zinc et un second demi-domaine de clivage de *FokI,* dans laquelle le second domaine à doigt de zinc peut se lier à une troisième séquence nucléotidique, dans laquelle la troisième séquence comprend au moins 9 nucléotides,

dans laquelle au moins l'une des protéines de fusion comprend une modification du résidu d'acide aminé au niveau d'une ou de plusieurs des positions 446, 447, 479, 483, 484, 486, 487, 490, 491, 496, 498, 499, 500, 531, 534, 537 et 538 de l'interface de dimérisation du demi-domaine de clivage qui inhibe l'homodimérisation du demi-domaine de clivage ; et

(d) un polynucléotide comprenant une quatrième séquence nucléotidique, dans laquelle la quatrième séquence nucléotidique est homologue mais non identique à la première séquence nucléotidique ;

dans laquelle la liaison de la première protéine de fusion à la deuxième séquence et la liaison de la seconde protéine de fusion à la troisième séquence positionnent les demi-domaines de clivage de sorte que la chromatine cellulaire est clivée dans la région d'intérêt, facilitant ainsi la recombinaison homologue entre la première séquence nucléotidique et la quatrième séquence nucléotidique, entraînant une modification de la première séquence nucléotidique.

**25.** Composition pharmaceutique pour modifier une première séquence nucléotidique dans une région d'intérêt dans la chromatine cellulaire, comprenant :

(a) une première protéine de fusion ou un polynucléotide codant pour la première protéine de fusion, dans laquelle la première protéine de fusion comprend un premier domaine de liaison à doigt de zinc et un premier demi-domaine de clivage de *FokI,*

dans laquelle le premier domaine à doigt de zinc est modifié pour se lier à une deuxième séquence nucléotidique dans la région d'intérêt, dans laquelle la deuxième séquence comprend au moins 9 nucléotides ;

(b) une seconde protéine de fusion ou un polynucléotide codant pour la seconde protéine de fusion, dans laquelle la seconde protéine de fusion comprend un second domaine de liaison à doigt de zinc et un second demi-domaine de clivage de *FokI ;*

dans laquelle le second domaine à doigt de zinc peut se lier à une troisième séquence nucléotidique, dans laquelle la troisième séquence comprend au moins 9 nucléotides,

dans laquelle au moins l'une des protéines de fusion comprend une modification du résidu d'acide aminé au niveau d'une ou de plusieurs des positions 446, 447, 479, 483, 484, 486, 487, 490, 491, 496, 498, 499, 500, 531, 534, 537 et 538 de l'interface de dimérisation du demi-domaine de clivage qui inhibe l'homodimérisation du demi-domaine de clivage ; et

(c) un polynucléotide comprenant une quatrième séquence nucléotidique, dans laquelle la quatrième séquence nucléotidique est homologue mais non identique à la première séquence nucléotidique, dans laquelle, en utilisation, la liaison de la première protéine de fusion à la deuxième séquence et la liaison de la seconde protéine de fusion à la troisième séquence positionnent les demi-domaines de clivage de sorte que la chromatine cellulaire est clivée dans la région d'intérêt, facilitant ainsi la recombinaison homologue entre la première séquence nucléotidique et la quatrième séquence nucléotidique, entraînant une modification de la première séquence nucléotidique.

**26.** Méthode in vitro pour inactiver un gène cible par délétion ciblée d'une séquence génomique du gène cible, la méthode comprenant le clivage du gène cible au niveau de deux sites de clivage de sorte que les deux sites sont joints de façon à supprimer la séquence entre les deux sites de clivage, inactivant ainsi le gène cible, dans laquelle la méthode comprend l'expression de deux protéines de fusion dans une cellule, chaque protéine de fusion comprenant un domaine de liaison à doigt de zinc et un demi-domaine de clivage de *FokI,* dans laquelle au moins l'une des protéines de fusion comprend une modification du résidu d'acide aminé au niveau d'une ou de plusieurs des positions 446, 447, 479, 483, 484, 486, 487, 490, 491, 496, 498, 499, 500, 531, 534, 537 et 538 de l'interface de dimérisation du demi-domaine de clivage qui inhibe l'homodimérisation du demi-domaine de clivage,

dans laquelle la liaison des protéines de fusion au gène cible positionne les demi-domaines de clivage de sorte qu'un domaine de clivage fonctionnel est reconstitué, conduisant au clivage du gène cible.

# FIGURE 1

```
                         MetGlyPheLeuLysLeuIle   (SEQ ID NO:2)
CTGCCGCCGGCGCCGCGGCCGTCATGGGGGTTCCTGAAACTGATT
GACGGCGGCCGCGGCGCCGGCAGTACCCCAAGGACTTTGACTAA   (SEQ ID NO:1)
      |      |      |      |      |      |      |      |
      5     10     15     20     25     30     35     40
```

# FIGURE 2

# FIGURE 3

A

```
Base Position:   5237000|   5238000|   5239000|   5240000|   5241000|   5242000|
                        Chromosome Bands Localized by FISH Mapping Clones
Chromosome Band
                   Known Genes Based on SWISS-PROT, TrEMBL, mRNA, and RefSeq
SMC1L1
BC046147
FLJ32783
BC036767
```

B

Initial amp 1

Chrom. specific                    [+1]        SMC1 coding sequence          Initial amp 2

C

```
                        SMC1 ZFP #1
                        ~~~~~~~~~~~~~
                        MetGlyPheLeuLysLeuIleGlu
CTGCCGCCGGCGCCGCGGCCGTCATGGGGTTCCTGAAACTGATTGAG          Chromosomal
GACGGCGGCCGCGGCGCCGGCAGTACCCCAAGGACTTTGACTAACTC          target DNA
                        ~~~~~~~~~~~~~
                        SMC1 ZFP #2
```

D

```
CTGCCGCCGGCGCCGCGGCCGTCATAAGAAGCTTCCTGAAACTGATTGAG      donor DNA
GACGGCGGCCGCGGCGCCGGCAGTATTCTTCGAAGGACTTTGACTAACTC      (fragment)
```

# FIGURE 4

M13 REV

SMC1 donor

M13 FOR

pCR4 BLUNT TOPO

4654 bp

# FIGURE 5

# FIGURE 6

```
TAGTCCTGCAGGTTTAAACGAATTCGCCCTTCTCAGCAAGCGTGAGCTCA    50
GGTCTCCCCCGCCTCCTTGAACCTCAAGAACTGCTCTGACTCCGCCCAGC    100
AACAACTCCTCCGGGGATCTGGTCCGCAGGAGCAAGTGTTTGTTGTTGCC    150
ATGCAACAAGAAAAGGGGGCGGAGGCACCACGCCAGTCGTCAGCTCGCTC    200
CTCGTATACGCAACATCAGTCCCCGCCCCTGGTCCCACTCCTGCCGGAAG    250
GCGAAGATCCCGTTAGGCCTGGACGTATTCTCGCGACATTTGCCGGTCGC    300
CCGGCTTGCACTGCGGCGTTTCCCGCGCGGGCTACCTCAGTTCTCGGGCG    350
TACGGCGCGGCCTGTCCTACTGCTGCCGGCGCCGCGGCCGTCATaagaag    400
cTTCCTGAAACTGATTGAAGGGCGAATTCGCGGCCGCTAAATTCAATTCG    450
CCCTATAGTGAGT
```

(SEQ ID NO: 6)

# FIGURE 7

```
                         TyrLysAsnSerAspAsnAspLysVal      (SEQ ID
NO:8)
CTTCCAACCTTTCTCCTCTAGGTACAAGAACTCGGATAATGATAAAGTCC
GAAGGTTGGAAAGAGGAGATCCATGTTCTTGAGCCTATTACTATTTCAGG      (SEQ ID
NO:7)
     |    |    |    |    |    |    |    |    |    |
     5   10   15   20   25   30   35   40   45   50
```

# FIGURE 8

pcDNA-ZFP-FokI
6346 bp

AmpR

pCMV

NLS

Zinc Finger Domain

FokI cleavage domain

BGH pas

ff1 ori

SV40 promoter

NeoR

SV40 pas

# FIGURE 9

A

Base Position:| 68561000| 68561500| 68562000| 68562500| 68563000| 68563500| 68564000| 68564500|

Chromosome Bands Localized by FISH Mapping Clones
Chromosome Band: Xq13.1
Known Genes based on SWISS-PROT, TrEMBL, mRNA, and RefSeq
IL2RC
RefSeq Genes
IL2RC

B

Initial amp 1        IL2Rg exon 3        Initial amp 2

Chrom. specific

C

IL2Ry ZFP #1
~~~~~~~~~~~~
TyrLysAsnSerAspAsnAspLysVal
GTTCCTCTTCCTTCCAACCTTTCTCCTCTAGGTACAAGAACTCGGATAATGATAAAGTC
CAAGGAGAAGGAAGGTTGGAAAGAGGAGATCCATGTTCTTGAGCCTATTACTATTTCAG
~~~~~~~~~~~~~
IL2Ry ZFP #2

Chromosomal
target DNA

D

GTTCCTCTTCCTTCCAACCTTTCTCCTCTAGGTAAAAGAATTCCGACAACGATAAAGTC
CAAGGAGAAGGAAGGTTGGAAAGAGGAGATCCATTTTCTTAAGGCTGTTGCTATTTCAG

Donor DNA
(fragment)

# FIGURE 10

M13 REV

Il2Rg donor

M13 FOR

pCR4 BLUNT TOPO

4654 bp

# FIGURE 11

# FIGURE 12

```
TAGTCCTGCAGGTTTAAACGAATTCGCCCTTTCCTCTAGGTAaAAGAAtT          50
CcGAcAAcGATAAAGTCCAGAAGTGCAGCCACTATCTATTCCCTGAAGAA         100
ATCACTTCTGGCTGTCAGTTGCAAAAAAAGGAGATCCACCTCTACCAAAC         150
ATTTGTTGTTCAGCTCCAGGACCCACGGGAACCCAGGAGACAGGCCACAC         200
AGATGCTAAAACTGCAGAATCTGGGTAATTTGGAAAGAAAGGGTCAAGAG         250
ACCAGGGATACTGTGGGACATTGGAGTCTACAGAGTAGTGTTCTTTTATC         300
ATAAGGGTACATGGGCAGAAAAGAGGAGGTAGGGGATCATGATGGGAAGG         350
GAGGAGGTATTAGGGGCACTACCTTCAGGATCCTGACTTGTCTAGGCCAG         400
GGGAATGACCACATATGCACACATATCTCCAGTGATCCCCTGGGCTCCAG         450
AGAACCTAACACTTCACAAACTGAGTGAATCCCAGCTAGAACTGAACTGG         500
AACAACAGATTCTTGAACCACTGTTTGGAGCACTTGGTGCAGTACCGGAC         550
TAAGGGCGAATTCGCGGCCGCTAAATTCAATTCGCCCTATAGTGAGTCGT         600
ATTACAATTCACTGGCCGTCGTTT
```

(SEQ ID NO:12)

# FIGURE 13

```
TACTGATGGTATGGGGCCAAGAGATATATCTTAGAGGGAGGGCTGAGGGT        50
TTGAAGTCCAACTCCTAAGCCAGTGCCAGAAGAGCCAAGGACAGGTACGG       100
CTGTCATCACTTAGACCTCACCCTGTGGAGCCACACCCTAGGGTTGGCCA       150
ATCTACTCCCAGGAGCAGGGAGGGCAGGAGCCAGGGCTGGGCATAAAAGT       200
CAGGGCAGAGCCATCTATTGCTTACATTTGCTTCTGACACAACTGTGTTC       250
ACTAGCAACCTCAAACAGACACCATGGTGCATCTGACTCCTGAGGAGAAG       300
TCTGCCGTTACTGCCCTGTGGGGCAAGGTGAACGTGGATGAAGTTGGTGG       350
TGAGGCCCTGGGCAGGTTGGTATCAAGGTTACAAGACAGGTTTAAGGAGA       400
CCAATAGAAACTGGGCATGTGGAGACAGAGAAGACTCTTGGGTTTCTGAT       450
AGGCACTGACTCTCTCTGCCTATTGGTCTATTTTCCCACCCTTAGGCTGC       500
TGGTGGTCTACCCTTGGACCCAGAGGTTCTTTGAGTCCTTTGGGGATCTG       550
TCCACTCCTGATGCTGTTATGGGCAACCCTAAGGTGAAGGCTCATGGCAA       600
GAAAGTGCTCGGTGCCTTTAGTGATGGCCTGGCTCACCTGGACAACCTCA       650
AGGGCACCTTTGCCACACTGAGTGAGCTGCACTGTGACAAGCTGCACGTG       700
```

(SEQ ID NO:13)

# FIGURE 14

Amp

pCMV

NLS

Zinc Finger
Domain

pcDNA-ZFP-FokI
6346

FokI cleavage
domain

SV40

BGH

ff1

Neo

SV40

# FIGURE 15

Initial amp 1   Exon 1   Exon2 Initial amp 2

Beta globin human genomic TARGET
700 bp

# FIGURE 16

ApaLI (4219)

PstI (274)

EcoRI (284)

NcoI (567)

EcoRI (612)

**beta globin donor**

EcoRI (1002)

pCR4-TOPO-HBBdonor

4657 bp

ApaLI (1318)

ApaLI (2973)

NcoI (2420)

# FIGURE 17

Loading Control                    GC-specific Product

# FIGURE 18

```
TGCTTACCAAGCTGTGATTCCAAATATTACGTAAATACACTTGCAAAGGA        50
GGATGTTTTTAGTAGCAATTTGTACTGATGGTATGGGGCCAAGAGATATA       100
TCTTAGAGGGAGGGCTGAGGGTTTGAAGTCCAACTCCTAAGCCAGTGCCA       150
GAAGAGCCAAGGACAGGTACGGCTGTCATCACTTAGACCTCACCCTGTGG       200
AGCCACACCCTAGGGTTGGCCAATCTACTCCCAGGAGCAGGGAGGGCAGG       250
AGCCAGGGCTGGGCATAAAAGTCAGGGCAGAGCCATCTATTGCTTACATT       300
TGCTTCTGACACAACTGTGTTCACTAGCAACCTCAAACAGACACCATGGT       350
GCATCTGACTCCTGAGGAGAAGTCTGgCGTTAgTGCCCgaattccgAtcG       400
TcAACcac
```

(SEQ ID NO:14)

# FIGURE 19

```
                                        5-8
CACGTTTCGTGTTCGGAGCCGCTTTAACCCACTCTGTGGAAG
GTGCAAAGCACAAGCCTCGGCGAAATTGGGTGAGACACCTTC
   5-10
```

(SEQ ID NO:15)

# FIGURE 20

```
  1 MAPKKKRKVG IHGVPAAMAE RPFQCRICMR NFSRSDNLSE HIRTHTGEKP FACDICGRKF
 61 ARNAHRINHT KIHTGSQKPF QCRICMRNFS RSDTLSEHIR THTGEKPFAC DICGRKFAAR
121 STRTTHTKIH LRQKDAARGS QLVKSELEEK KSELRHKLKY VPHEYIELIE IARNSTQDRI
181 LEMKVMEFFM KVYGYRGKHL GGSRKPDGAI YTVGSPIDYG VIVDTKAYSG GYNLPIGQAD
241 EMQRYVEENQ TRNKHINPNE WWKVYPSSVT EFKFLFVSGH FKGNYKAQLT RLNHITNCNG
301 AVLSVEELLI GGEMIKAGTL TLEEVRRKFN NGEINF
```

(SEQ ID NO:16)

# FIGURE 21

```
  1 MAPKKKRKVG IHGVPAAMAE RPFQCRICMR NFSRSDSLSR HIRTHTGEKP FACDICGRKF
 61 ADSSNRKTHT KIHTGGGGSQ KPFQCRICMR NFSRSDSLSV HIRTHTGEKP FACDICGRKF
121 ADRSNRITHT KIHLRQKDAA RGSQLVKSEL EEKKSELRHK LKYVPHEYIE LIEIARNSTQ
181 DRILEMKVME FFMKVYGYRG KHLGGSRKPD GAIYTVGSPI DYGVIVDTKA YSGGYNLPIG
241 QADEMQRYVE ENQTRNKHIN PNEWWKVYPS SVTEFKFLFV SGHFKGNYKA QLTRLNHITN
301 CNGAVLSVEE LLIGGEMIKA GTLTLEEVRR KFNNGEINF
```

(SEQ ID NO:17)

# FIGURE 22

CGAATTCTGCAGTCGACGGTACCGCGGGCCCGGGATCCACCGGTCGCCACCATGGTGAGC
AAGGGCGAGGAGCTGTTCACCGGGGTGGTGCCCATCCTGGTCGAGCTGGACGGCGACGTA
AACGGCCACAAGTTCAGCGTGTCCGGCGAGGGCGAGGGCGATGCCACCTACGGCAAGCTG
ACCCTGAAGTTCATCTGCACCACCGGCAAGCTGCCCGTGCCCTGGCCCACCCTCGTGACC
ACCCTGACCTACGGCGTGCAGTGCTTCAGCCGCTACCCCGACCACATGAAGCAGCACGAC
TTCTTCAAGTCCGCCATGCCCGAAGGCTACGTCCAGGAGCGCACCATCTTCTTCAAGGAC
GACGGCAACTACAAGACCCGCGCCGAGGTGAAGTTCGAGGGCGACACCCTGGTGAACCGC
ATCGAGCTGAAGGGCATCGACTTCAAGGAGGACGGCAACATCCTGGGGCACAAGCTGGAG
TACAACTACAACAGCCACAACGTCTATATCATGGCCGACAAGCAGAAGAACGGCATCAAG
GTGAACTTCAAGATCCGCCACAACATCGAGGACGGCAGCGTGCAGCTCGCCGACCACTAC
CAGCAGAACACCCCCATCGGCGACGGCCCCGTGCTGCTGCCCGACAACCACTACCTGAGC
ACCCAGTCCGCCCTGAGCAAAGACCCCAACGAGAAGCGCGATCACATGGTCCTGCTGGAG
TTCGTGACCGCCGCCGGGATCACTCTCGGCATGGACGAGCTGTACAAGTAAAGCGGCCGC
GACTCTAGATCATAATC

(SEQ ID NO:18)

# FIGURE 23

CGAATTCTGCAGTCGACGGTACCGCGGGCCCGGGATCCACCGGTCGCCACCATGGTGAGC
AAGGGCGAGGAGCTGTTCACCGGGGTGGTGCCCATCCTGGTCGAGCTGGACGGCGACGTA
AACGGCCACAAGTTCAGCGTGTCCGGCGAGGGCGAGGGCGATGCCACCTACGGCAAGCTG
ACCCTGAAGTTCATCTGCACCACCGGCAAGCTGCCCGTGCCCTGGCCCACCCTCGTGACC
ACCCTGACCTACGGCGTGCAGTGCTTCAGC<u>CGCTACCCC</u>TAACAC<u>GAAGCAGC</u>ACGACTT
CTTCAAGTCCGCCATGCCCGAAGGCTACGTCCAGGAGCGCACCATCTTCTTCAAGGACGA
CGGCAACTACAAGACCCGCGCCGAGGTGAAGTTCGAGGGCGACACCCTGGTGAACCGCAT
CGAGCTGAAGGGCATCGACTTCAAGGAGGACGGCAACATCCTGGGGCACAAGCTGGAGTA
CAACTACAACAGCCACAACGTCTATATCATGGCCGACAAGCAGAAGAACGGCATCAAGGT
GAACTTCAAGATCCGCCACAACATCGAGGACGGCAGCGTGCAGCTCGCCGACCACTACCA
GCAGAACACCCCCATCGGCGACGGCCCCGTGCTGCTGCCCGACAACCACTACCTGAGCAC
CCAGTCCGCCCTGAGCAAAGACCCCAACGAGAAGCGCGATCACATGGTCCTGCTGGAGTT
CGTGACCGCCGCCGGGATCACTCTCGGCATGGACGAGCTGTACAAGTAAAGCGGCCGCGA
CTCTAGATCATAATC

(SEQ ID NO:19)

# FIGURE 24

# FIGURE 25

# FIGURE 26

# FIGURE 27

# FIGURE 28

# FIGURE 29

```
GGCGAGGAGCTGTTCACCGGGGTGGTGCCCATCCTGGTCGAGCTGGACGGCGACGTAAAC
GGCCACAAGTTCAGCGTGTCCGGCGAGGGCGAGGGCGATGCCACCTACGGCAAGCTGACC
CTGAAGTTCATCTGCACCACCGGCAAGCTGCCCGTGCCCTGGCCCACCCTCGTGACCACC
CTGACCTACGGCGTGCAGTGCTTCAGCCGCTACCCCGACCACATGAAGCAGCACGACTTC
TTCAAGTCCGCCATGCCCGAAGGCTACGTCCAGGAGCGCACCATCTTCTTCAAGGACGAC
GGCAACTACAAGACCCGCGCCGAGGTGAAGTTCGAGGGCGACACCCTGGTGAACCGCATC
GAGCTGAAGGGCATCGACTTCAAGGAGGACGGCAACATCCTGGGGCACAAGCTGGAGTAC
AACTACAACAGCCACAACGTCTATATCATGGCCGACAAGCAGAAGAACGGCATCAAGGTG
AACTTCAAGATCCGCCACAACATCGAGGACGGCAGCGTGCAGCTCGCCGACCACTACCAG
CAGAACACCCCCATCGGCGACGGCCCCGTGCTGCTGCCCGACAACCACTACCTGAGCACC
CAGTCCGCCCTGAGCAAAGACCCCAACGAGAAGCGCGATCACATGGTCCTGCTGGAGTTC
GTGACCGCCGCCGGGATCACTCTCGGCATGGACGAGCTGTACAAGTAAAGCGGCCGCGAC
TCTAGATCATAATC
```

(SEQ ID NO:20)

# FIGURE 30

# FIGURE 31

# FIGURE 32

GGCGAGGAGCTGTTCACCGGGGTGGTGCCCATCCTGGTCGAGCTGGACGGCGACGTAAAC
GGCCACAAGTTCAGCGTGTCCGGCGAGGGCGAGGGCGATGCCACCTACGGCAAGCTGACC
CTGAAGTTCATCTGCACCACCGGCAAGCTGCCCGTGCCCTGGCCCACCCTCGTGACCACC
CTGACCTACGGCGTGCAGTGCTTCAGCCGCTACCCCGACCACATGAAGCAGCACGACTTC
TTCAAGTCCGCCATGCCCGAAGGCTACGTCCAGGAGCGCACCATCTTCTTCAAGGACGAC
GGCAACTACAAGACCCGCGCCGAGGTGAAGTTCGAGGGCGACACCCTGGTGAACCGCATC
GAGCTGAAGGGCATCGACTTCAAGGAGGACGGCAACATCCTGGGGCACAAGCTGGAGTAC
AACTACAACAGCCACAACGTCTATATCATGGCCGACAAGCAGAAGAACGGCATCAAGGTG
AACTTCAAGATCCGCCACAACATCGAGGACGGCAGCGTGCAGCTCGCCGACCACTACCAG
CAGAACACCCCCATCGGCGACGGCCCCGTGCTGCTGCCCGACAACCACTACCTGAGCACC
CAGTCCGCCCTGAGCAAAGACCCCAACGAGAAGCGCGATCACATGGTCCTGCTGGAGTTC
GTGACCGCCGCCGGGATCACTCTCGGCATGGACGAGCTGTACAAGTAAAGCGGCCGCTCG
AGTCTAGAGGGCCCGTTTAAACCCGCTGATCAGCCTCGACTGTGCCTTCTAGTTGCCAGC
CATCTGTTGTTTGCCCCTCCCCCGTGCCTTCCTTGACCCTGGAAGGTGCCACTCCCACTG
TCCTTTCCTAATAAAATGAGGAAATTGCATCGCATTGTCTGAGTAGGTGTCATTCTATTC
TGGGGGGTGGGGTGGGGCAGGACAGCAAGGGGGAGGATTGGGAAGACAATAGCAGGCATG
CTGGGGATGCGGTGGGCTCTATGGCTTCTGAGGCGGAAAGAACCAGCTGGGGCTCTAGGG
GGTATCCCCACGCGCCCTGTAGCGGCGCATTAAGCGCGGCGGGTGTGGTGGTTACGCGCA
GCGTGACCGCTACACTTGCCAGCGCCCTAGCGCCCGCTCCTTTCGCTTTCTTCCCTTCCT
TTCTCGCCACGTTCGCCGGCTTTCCCCGTCAAGCTCTAAATCGGGGGCTCCCTTTAGGGT
TCCGATTTAGTGCTTTACGGCACCTCGACCCCAAAAAACTTGATTAGGGTGATGGTTCAC
GTAGTGGGCCATCGCCCTGATAGACGGTTTTTCGCCCTTTGACGTTGGAGTCCACGTTCT
TTAATAGTGGACTCTTGTTCCAAACTGGAACAACACTCAACCCTATCTCGGTCTATTCTT
TTGATTTATAAGGGATTTTGCCGATTTCGGCCTATTGGTTAAAAAATGAGCTGATTTAAC
AAAAATTTAACGCGAATTAATTCTGTGGAATGTGTGTCAGTTAGGGTGTGGAAAGTCCCC
AGGCTCCCCAGCAGGCAGAAGTATGCA

(SEQ ID NO:21)

# FIGURE 33

# FIGURE 34

# FIGURE 35

# FIGURE 36

# FIGURE 37

# FIGURE 38

# FIGURE 39

# FIGURE 40

**sca-29b:**

MAERPFQCRICMRNFSQSGDLTRHIRTHTGEKPFACDICGRKFATSANLSRHTK
IHTGGGGSQKPFQCRICMRNFSDRSALSRHIRTHTGEKPFACDICGRKFAQSG
HLSRHTKIH  (SEQ ID NO:22)

**sca-36a:**

MAERPFQCRICMRNFSRSQTRKTHIRTHTGEKPFACDICGRKFAQKRNRTKHT
KIHTGSQKPFQCRICMRNFSDRSALSRHIRTHTGEKPFACDICGRKFAQSGNLA
RHTKIH  (SEQ ID NO:23)

**sca-36b:**

MAERPFQCRICMRNFSTSGSLSRHIRTHTGEKPFACDICGRKFADRSDLSRHTK
IHTGGGGSQKPFQCRICMRNFSDRSALSRHIRTHTGEKPFACDICGRKFAQSG
NLARHTKIH  (SEQ ID NO:24)

**sca-36c:**

MAERPFQCRICMRNFSTSSSLSRHIRTHTGEKPFACDICGRKFADRSDLSRHTK
IHTGGGGSQKPFQCRICMRNFSDRSALSRHIRTHTGEKPFACDICGRKFAQSG
NLARHTKIH  (SEQ ID NO:25)

# FIGURE 41

# FIGURE 42

## REFERENCES CITED IN THE DESCRIPTION

### Patent documents cited in the description

- US 6528313 B
- US 6528314 B
- US 6140081 A
- US 6453242 B
- US 6534261 B
- WO 9853058 A
- WO 9853059 A
- WO 9853060 A
- WO 02016536 A
- WO 03016496 A
- US 5789538 A
- US 5925523 A
- US 6007988 A
- US 6013453 A
- US 6200759 B
- WO 9519431 A
- WO 9606166 A
- WO 9853057 A
- WO 9854311 A
- WO 0027878 A
- WO 0160970 A
- WO 0188197 A
- WO 02099084 A
- US 5176996 A
- US 5422251 A
- US 5585245 A
- WO 9844350 A
- WO 02077227 A
- US 6479626 B
- WO 0153480 A
- WO 02057293 A
- US 6410248 B
- US 6140466 A
- US 6242568 B
- WO 9837186 A
- GB 2338237 A
- US 5356802 A
- US 5436150 A
- US 5487994 A
- US 6506559 B
- US 5049386 A
- US 4946787 A
- US 4897355 A
- WO 9117424 A
- WO 9116024 A
- US 4186183 A
- US 4217344 A
- US 4235871 A
- US 4261975 A
- US 4485054 A
- US 4501728 A
- US 4774085 A
- US 4837028 A
- US 9405700 W
- US 4797368 A
- WO 9324641 A
- US 5173414 A
- US 5928638 A
- US 4957773 A
- US 4603044 A
- US 5061620 A
- US 5681559 A
- US 6335195 B
- US 6645489 B
- US 6667064 B
- WO 0183793 A
- US 6607882 B

### Non-patent literature cited in the description

- **CAPECCHI.** *Science,* 1989, vol. 244, 1288-1292
- **ROUET et al.** *Mol. Cell. Biol.,* 1994, vol. 14, 8096-8106
- **CHOULIKA et al.** *Mol. Cell. Biol.,* 1995, vol. 15, 1968-1973
- **DONOHO et al.** *Mol. Cell. Biol.,* 1998, vol. 18, 4070-4078
- **JOHNSON et al.** *Biochem. Soc. Trans.,* 2001, vol. 29, 196-201
- **YANEZ et al.** *Gene Therapy,* 1998, vol. 5, 149-159
- **BIBIKOVA et al.** *Science,* 2003, vol. 300 (5620), 764
- **BIBIKOVA et al.** *Genetics,* 2002, vol. 161 (3), 1169-1175
- **TSIEN.** *Ann. Rev. Biochem.,* 1998, vol. 67, 509-544

- **SAMBROOK et al.** MOLECULAR CLONING: A LABORATORY MANUAL. Cold Spring Harbor Laboratory Press, 1989
- **AUSUBEL et al.** CURRENT PROTOCOLS IN MOLECULAR BIOLOGY. John Wiley & Sons, 1987
- METHODS IN ENZYMOLOGY. Academic Press
- **WOLFFE.** CHROMATIN STRUCTURE AND FUNCTION. Academic Press, 1998
- Chromatin. METHODS IN ENZYMOLOGY. Academic Press, 1999, vol. 304
- Chromatin Protocols. METHODS IN MOLECULAR BIOLOGY. Humana Press, 1999, vol. 119
- **SMITH ; WATERMAN.** *Advances in Applied Mathematics,* 1981, vol. 2, 482-489
- National Biomedical Research Foundation. **DAYHOFF.** Atlas of Protein Sequences and Structure. vol. 5, 353-358
- **GRIBSKOV.** *Nucl. Acids Res.,* 1986, vol. 14 (6), 6745-6763
- Wisconsin Sequence Analysis Package Program Manual. 1995
- **SAMBROOK et al.** Nucleic Acid Hybridization: A Practical Approach. IRL Press, 1985
- **SAMBROOK et al.** Molecular Cloning: A Laboratory Manual. Cold Spring Harbor, 1989
- Nucleic Acid Hybridization: A Practical Approach. IRL Press, 1985
- **FIELDS et al.** *Nature,* 1989, vol. 340, 245-246
- **MILLER et al.** *EMBO J.,* 1985, vol. 4, 1609-1614
- **RHODES.** *Scientific American,* February 1993, 56-65
- **BEERLI et al.** *Nature Biotechnol.,* 2002, vol. 20, 135-141
- **PABO et al.** *Ann. Rev. Biochem.,* 2001, vol. 70, 313-340
- **ISALAN et al.** *Nature Biotechnol.,* 2001, vol. 19, 656-660
- **SEGAL et al.** *Curr. Opin. Biotechnol.,* 2001, vol. 12, 632-637
- **CHOO et al.** *Curr. Opin. Struct. Biol.,* 2000, vol. 10, 411-416
- **MOORE et al.** *Proc. Natl. Acad. Sci. USA,* 2001, vol. 98, 1432-1436
- **MOORE et al.** *Proc. Natl. Acad. Sci. USA,* 2001, vol. 98, 1437-1441
- **BELFORT et al.** *Nucleic Acids Res.,* 1997, vol. 25, 3379-3388
- **LINN ET AL.** Nucleases. Cold Spring Harbor Laboratory Press, 1993
- **LI et al.** *Proc. Natl. Acad. Sci. USA,* 1992, vol. 89, 4275-4279
- **LI et al.** *Proc. Natl. Acad. Sci. USA,* 1993, vol. 90, 2764-2768
- **KIM et al.** *Proc. Natl. Acad. Sci. USA,* 1994, vol. 91, 883-887
- **KIM et al.** *J. Biol. Chem.,* 1994, vol. 269, 31, , 978-31, 982
- **BITINAITE et al.** *Proc. Natl. Acad. Sci. USA,* 1998, vol. 95, 10, , 570-10, 575
- **ROBERTS et al.** *Nucleic Acids Res.,* 2003, vol. 31, 418-420
- **SMITH et al.** *Nucleic Acids Res.,* 2000, vol. 28, 3361-3369
- **BIBIKOVA et al.** *Mol. Cell. Biol.,* 2001, vol. 21, 289-297
- **WAH et al.** *Proc. Natl. Acad. Sci. USA,* 1998, vol. 95, 10564-10569
- **CHANG et al.** *Proc. Natl. Acad. Sci. USA,* 1987, vol. 84, 4959-4963
- **NEHLS et al.** *Science,* 1996, vol. 272, 886-889
- **HOEIJMAKERS.** *Nature,* 2001, vol. 411, 366-374
- **TAUCHI et al.** *Oncogene,* 2002, vol. 21, 8967-8980
- **VALERIE et al.** *Oncogene,* 2003, vol. 22, 5792-5812
- **JANZ et al.** *Oncogene,* 2002, vol. 21, 5929-5933
- **MEKEEL et al.** *Oncogene,* 1997, vol. 14, 1847-1857
- **BERTRAND et al.** *Oncogene,* 1997, vol. 14, 1117-1122
- **SAINTIGNY et al.** *Oncogene,* 1999, vol. 18, 3553-3563
- **BOEHDEN et al.** *Oncogene,* 2003, vol. 22, 4111-4117
- **SAMBROOK et al.** Molecular Cloning, A Laboratory Manual
- **KRIEGLER.** *Gene Transfer and Expression: A Laboratory Manual,* 1990
- *Current Protocols in Molecular Biology*
- **PALVA et al.** *Gene,* 1983, vol. 22, 229-235
- **GOSSEN ; BUJARD.** *PNAS,* 1992, vol. 89, 5547
- **OLIGINO et al.** *Gene Ther.,* 1998, vol. 5, 491-496
- **WANG et al.** *Gene Ther.,* 1997, vol. 4, 432-441
- **NEERING et al.** *Blood,* 1996, vol. 88, 1147-1155
- **RENDAHL et al.** *Nat. Biotechnol.,* 1998, vol. 16, 757-761
- **COLLEY et al.** *J. Biol. Chem.,* 1989, vol. 264, 17619-17622
- *Guide to Protein Purification, in Methods in Enzymology,* 1990, vol. 182
- **MORRISON.** *J. Bact.,* 1977, vol. 132, 349-351
- **CLARK-CURTISS ; CURTISS et al.** Methods in Enzymology. 1983, vol. 101, 347-362
- **ANDERSON.** *Science,* 1992, vol. 256, 808-813

- **NABEL ; FELGNER.** *TIBTECH,* 1993, vol. 11, 211-217
- **MITANI ; CASKEY.** *TIBTECH,* 1993, vol. 11, 162-166
- **DILLON.** *TIBTECH,* 1993, vol. 11, 167-175
- **MILLER.** *Nature,* 1992, vol. 357, 455-460
- **VAN BRUNT.** *Biotechnology,* 1988, vol. 6 (10), 1149-1154
- **VIGNE.** *Restorative Neurology and Neuroscience,* 1995, vol. 8, 35-36
- **KREMER ; PERRICAUDET.** *British Medical Bulletin,* 1995, vol. 51 (1), 31-44
- **HADDADA et al.** *Current Topics in Microbiology and Immunology,* 1995
- **YU et al.** *Gene Therapy,* 1994, vol. 1, 13-26
- **CRYSTAL.** *Science,* 1995, vol. 270, 404-410
- **BLAESE et al.** *Cancer Gene Ther.,* 1995, vol. 2, 291-297
- **BEHR et al.** *Bioconjugate Chem.,* 1994, vol. 5, 382-389
- **REMY et al.** *Bioconjugate Chem.,* 1994, vol. 5, 647-654
- **GAO et al.** *Gene Therapy,* 1995, vol. 2, 710-722
- **AHMAD et al.** *Cancer Res.,* 1992, vol. 52, 4817-4820
- **BUCHSCHER et al.** *J. Virol.,* 1992, vol. 66, 2731-2739
- **JOHANN et al.** *J. Virol.,* 1992, vol. 66, 1635-1640
- **SOMMERFELT et al.** *Virol.,* 1990, vol. 176, 58-59
- **WILSON et al.** *J. Virol.,* 1989, vol. 63, 2374-2378
- **MILLER et al.** *J. Virol.,* 1991, vol. 65, 2220-2224
- **WEST et al.** *Virology,* 1987, vol. 160, 38-47
- **KOTIN.** *Human Gene Therapy,* 1994, vol. 5, 793-801
- **MUZYCZKA.** *J. Clin. Invest.,* 1994, vol. 94, 1351
- **TRATSCHIN et al.** *Mol. Cell. Biol.,* 1985, vol. 5, 3251-3260
- **TRATSCHIN et al.** *Mol. Cell. Biol.,* 1984, vol. 4, 2072-2081
- **HERMONAT ; MUZYCZKA.** *PNAS,* 1984, vol. 81, 6466-6470
- **SAMULSKI et al.** *J. Virol.,* 1989, vol. 63, 03822-3828
- **DUNBAR et al.** *Blood,* 1995, vol. 85, 3048-305
- **KOHN et al.** *Nat. Med.,* 1995, vol. 1, 1017-102
- **MALECH et al.** 94. *PNAS,* 1997, (22), 12133-12138
- **BLAESE et al.** *Science,* 1995, vol. 270, 475-480
- **ELLEM et al.** *Immunol Immunother.,* 1997, vol. 44 (1), 10-20
- **DRANOFF et al.** *Hum. Gene Ther.,* 1997, vol. 1, 111-2
- **WAGNER et al.** *Lancet,* 1998, vol. 351 (9117), 1702-3
- **KEARNS et al.** *Gene Ther.,* 1996, vol. 9, 748-55
- **STERMAN et al.** *Hum. Gene Ther.,* 1998, vol. 7, 1083-9
- **ROSENECKER et al.** *Infection,* 1996, vol. 24 (1), 5-10
- **STERMAN et al.** *Hum. Gene Ther.,* 1998, vol. 9 (7), 1083-1089
- **WELSH et al.** *Hum. Gene Ther.,* 1995, vol. 2, 205-18
- **ALVAREZ et al.** *Hum. Gene Ther.,* 1997, vol. 5, 597-613
- **TOPF et al.** *Gene Ther.,* 1998, vol. 5, 507-513
- **STERMAN et al.** *Hum. Gene Ther.,* 1998, vol. 7, 1083-1089
- **HAN et al.** *Proc. Natl. Acad. Sci. USA,* 1995, vol. 92, 9747-9751
- **FRESHNEY et al.** Culture of Animal Cells, A Manual of Basic Technique. 1994
- **INABA et al.** *J. Exp. Med.,* 1992, vol. 176, 1693-1702
- Remington's Pharmaceutical Sciences. 1989
- **WEISSBACH ; WEISSBACH.** Methods for Plant Molecular Biology. Academic Press, 1988, 421-463
- **GRIERSON ; COREY.** *Plant Molecular Biology,* 1988
- **KLEIN et al.** *Nature,* 1987, vol. 327, 70-73
- **HORSCH et al.** *Science,* 1984, vol. 233, 496-498
- **FRALEY et al.** *Proc. Nat'l. Acad. Sci. USA,* 1983, vol. 80, 4803
- **BEVAN.** *Nuc. Acid Res.,* 1984, vol. 12, 8711-8721
- **HORSCH et al.** *Science,* 1985, vol. 227, 1229-1231
- **BEVAN et al.** *Ann. Rev. Genet,* 1982, vol. 16, 357-384
- **ROGERS et al.** *Methods Enzymol.,* 1986, vol. 118, 627-641
- **HERNALSTEEN et al.** *EMBO J,* 1984, vol. 3, 3039-3041
- **HOOYKASS-VAN SLOGTEREN et al.** *Nature,* 1984, vol. 311, 763-764
- **GRIMSLEY et al.** *Nature,* 1987, vol. 325, 1677-179
- **BOULTON et al.** *Plant Mol. Biol.,* 1989, vol. 12, 31-40
- **GOULD et al.** *Plant Physiol.,* 1991, vol. 95, 426-434
- **PASZKOWSKI et al.** *EMBO J,* 1984, vol. 3, 2717-2722
- **POTRYKUS et al.** *Molec. Gen. Genet.,* 1985, vol. 199, 169-177

- **FROMM et al.** *Proc. Nat. Acad. Sci. USA,* 1985, vol. 82, 5824-5828
- **SHIMAMOTO.** *Nature,* 1989, vol. 338, 274-276
- **D'HALLUIN et al.** *Plant Cell,* 1992, vol. 4, 1495-1505
- **KAEPPLER et al.** *Plant Cell Reporter,* 1990, vol. 9, 415-418
- **KLEIN et al.** *Proc. Nat. Acad. Sci. USA,* 1988, vol. 85, 4305-4309
- **GORDON-KAMM et al.** *Plant Cell,* 1990, vol. 2, 603-618
- Protoplasts Isolation and Culture. **EVANS et al.** Handbook of Plant Cell Culture. Macmillian Publishing Company, 1983, 124-176
- **BINDING.** Regeneration of Plants, Plant Protoplasts. CRC Press, 1985, 21-73
- **KLEE et al.** *Ann. Rev. of Plant Phys.,* 1987, vol. 38, 467-486
- **PROCHIANTZ.** *Current Opinion in Neurobiology,* 1996, vol. 6, 629-634
- **LIN et al.** *J. Biol. Chem.,* 1995, vol. 270 (1), 4255-14258
- **FAHRAEUS et al.** *Current Biology,* 1996, vol. 6, 84
- **DEROSSI et al.** *J. Biol. Chem.,* 1994, vol. 269, 10444
- **ELLIOT ; O'HARE.** *Cell,* 1997, vol. 88, 223-233
- **YEH et al.** *Molecular Therapy,* 2003, vol. 7 (5), S461
- **ARORA et al.** *J. Biol. Chem.,* 1993, vol. 268, 3334-3341
- **PERELLE et al.** *Infect. Immun.,* 1993, vol. 61, 5147-5156
- **STENMARK et al.** *J. Cell Biol.,* 1991, vol. 113, 1025-1032
- **DONNELLY et al.** *PNAS,* 1993, vol. 90, 3530-3534
- **CARBONETTI et al.** *Abstr. Annu. Meet. Am. Soc. Microbiol.,* 1995, vol. 95, 295
- **SEBO et al.** *Infect. Immun.,* 1995, vol. 63, 3851-3857
- **KLIMPEL et al.** *PNAS U.S.A.,* 1992, vol. 89, 10277-10281
- **NOVAK et al.** *J. Biol. Chem.,* 1992, vol. 267, 17186-17193
- *PNAS,* 1987, vol. 84, 7851
- *Biochemistry,* 1989, vol. 28, 908
- **SZOKA et al.** *Ann. Rev. Biophys. Bioeng.,* 1980, vol. 9, 467
- **DEAMER ; BANGHAM.** *Biochim. Biophys. Acta,* 1976, vol. 443, 629-634
- **FRALEY et al.** *PNAS,* 1979, vol. 76, 3348-3352
- **HOPE et al.** *Biochim. Biophys. Acta,* 1985, vol. 812, 55-65
- **MAYER et al.** *Biochim. Biophys. Acta,* 1986, vol. 858, 161-168
- **WILLIAMS et al.** *PNAS,* 1988, vol. 85, 242-246
- Liposomes. 1983
- **HOPE et al.** *Chem. Phys. Lip.,* 1986, vol. 40, 89
- **GREGORIADIS.** *Liposome Technology,* 1984
- **LASIC.** *Liposomes: from Physics to Applications,* 1993
- **RENNEISEN et al.** *J. Biol. Chem.,* 1990, vol. 265, 16337-16342
- **LEONETTI et al.** *PNAS,* 1990, vol. 87, 2448-2451
- Cell Biology. 1976
- Remington's Pharmaceutical Sciences. 1985
- Virology. 1988
- Fundamental Virology. 1991
- **COFFMAN et al.** *J Immunol,* 1993, vol. 151, 425-35
- **MARKMANN et al.** *Transplantation,* 1992, vol. 54, 1085-9
- **KOLLER et al.** *Science,* 1990, vol. 248, 1227-30
- **AKOPIAN et al.** *Proc. Natl. Acad. Sci. USA,* 2003, vol. 100, 8688-8691
- **KITA et al.** *J. Biol. Chem.,* 1989, vol. 264, 5751-5756
- **HO et al.** *Gene,* 1989, vol. 77, 51-59
- **PUCK et al.** *Blood,* 1997, vol. 89, 1968-1977
- **HEIM et al.** *Nature,* 1995, vol. 373, 663-664
- **CORMACK et al.** *Gene,* 1996, vol. 173, 33-38
- **LOONEY et al.** *Gene,* 1989, vol. 80, 193-208